# EUROPEAN PATENT SPECIFICATION

(11) **EP 2 992 015 B1**
(45) Date of publication and mention of the grant of the patent: **29.08.2018**
(21) Application number: 14721825.9
(22) Date of filing: 30.04.2014
(51) Int. Cl.: C07K 16/28, A61K 47/00, A61P 35/00

(54) **COMBINATION THERAPY OF AN AFUCOSYLATED CD20 ANTIBODY WITH A CD22 ANTIBODY-DRUG CONJUGATE**
KOMBINATIONSTHERAPIE AUS EINEM AFUCOSYLIERTEN CD20-ANTIKÖRPER MIT EINEM CD22 ANTIKÖRPER-WIRKSTOFF-KONJUGAT
POLYTHÉRAPIE AVEC UN ANTICORPS AFUCOSYLÉ ANTI-CD20 ET AVEC UN CONJUGUÉ ANTICORPS ANTI-CD22-MÉDICAMENT

(30) Priority: 02.05.2013 US 201361818810 P
(43) Date of publication of application: 09.03.2016
(73) Proprietor: F. Hoffmann-La Roche AG, 4070 Basel (CH)
(72) Inventor: KLEIN, Christian, CH-8906 Bonstetten (CH); LANG, Sabine, CH-8953 Dietikon (CH); POLSON, Andrew, South San Francisco, California 94080 (US); UMANA, Pablo, CH-8832 Wollerau (CH)
(74) Representative: Klein, Thomas
(86) International application number: PCT/EP2014/058831
(87) International publication number: WO 2014/177617

(56) References cited:
- WO-A2-01/97858
- US-A1- 2011 305 631
- OGURA M ET AL: "Phase I study of anti-CD22 immunoconjugate inotuzumab ozogamicin plus rituximab in relapsed/refractory B-cell non-Hodgkin lymphoma", CANCER SCIENCE, JAPANESE CANCER ASSOCIATION, TOKYO, JP, vol. 103, no. 5, 1 May 2012 (2012-05-01), pages 933-938, XP002714417, ISSN: 1347-9032, DOI: 10.1111/J.1349-7006.2012.02241.X [retrieved on 2012-03-20]
- L. FAYAD ET AL: "Safety and Clinical Activity of a Combination Therapy Comprising Two Antibody-Based Targeting Agents for the Treatment of Non-Hodgkin Lymphoma: Results of a Phase I/II Study Evaluating the Immunoconjugate Inotuzumab Ozogamicin With Rituximab", JOURNAL OF CLINICAL ONCOLOGY, vol. 31, no. 5, 7 January 2013 (2013-01-07), pages 573-583, XP055128142, ISSN: 0732-183X, DOI: 10.1200/JCO.2012.42.7211
- Anonymous: "An Open-Label, Multicenter, Phase I Trial of the Safety and Pharmacokinetics of Escalating Doses of DCDT2980S in Patients With Relapsed or Refractory B-Cell Non-Hodgkin's Lymphoma and Chronic Lymphocytic Leukemia And DCDT2980S in Combination With Rituximab in Patients With Relapsed or Refractory B-C", , 11 March 2013 (2013-03-11), XP055128133, Retrieved from the Internet: URL:http://clinicaltrials.gov/archive/NCT0 1209130/2013_03_11 [retrieved on 2014-07-10]
- Anonymous: "A Randomized, Open-Label, Multicenter, Phase II Trial Evaluating the Safety and Activity of DCDT2980S in Combination With Rituximab or DCDS4501A in Combination With Rituximab in Patients With Relapsed or Refractory B-cell Non Hodgkin's Lymphoma", , 19 February 2013 (2013-02-19), pages 1-9, XP055128138, Retrieved from the Internet: URL:http://clinicaltrials.gov/archive/NCT0 1691898/2013_02_19 [retrieved on 2014-07-10]
- M. J. MATTES ET AL: "Therapy of Advanced B-Lymphoma Xenografts with a Combination of 90Y-anti-CD22 IgG (Epratuzumab) and Unlabeled Anti-CD20 IgG (Veltuzumab)", CLINICAL CANCER RESEARCH, vol. 14, no. 19, 1 October 2008 (2008-10-01), pages 6154-6160, XP055128443, ISSN: 1078-0432, DOI: 10.1158/1078-0432.CCR-08-0404
- UMANA PABLO ET AL: "GA101, a novel humanized type IICD20 antibody with glycoengineered Fc and enhanced cell death induction, exhibits superior anti-tumour efficacy and superior tissue B-cell depletion in vivo", BLOOD, AMERICAN SOCIETY OF HEMATOLOGY, US, vol. 110, no. 11, 1 January 2007 (2007-01-01), XP002559271, ISSN: 0006-4971

### SEQUENCE LISTING

<110> F. Hoffmann-La Roche AG
<120> Combination therapy of an afucosylated CD20 antibody with a CD22 antibody-drug conjugate
<130> 31512 and PR5606
<140>
   <141>
<150> US 61/818810
   <151> 2013-05-02
<160> 51
<170> PatentIn version 3.2
<210> 1
   <211> 25
   <212> PRT
   <213> Artificial sequence
<220>
   <223> sequence is synthesized
<400> 1
<210> 2
   <211> 10
   <212> PRT
   <213> Artificial sequence
<220>
   <223> sequence is synthesized
<400> 2
   Gly Tyr Glu Phe Ser Arg Ser Trp Met Asn
   1               5                   10
<210> 3
   <211> 13
   <212> PRT
   <213> Artificial sequence
<220>
   <223> sequence is synthesized
<400> 3
   Trp Val Arg Gln Ala Pro Gly Lys Gly Leu Glu Trp Val
                   5                   10
<210> 4
   <211> 18
   <212> PRT
   <213> Artificial sequence
<220>
   <223> sequence is synthesized
<400> 4
   Gly Arg Ile Tyr Pro Gly Asp Gly Asp Thr Asn Tyr Ser Gly Lys
   1 5 10 15
   Phe Lys Gly
<210> 5
   <211> 32
   <212> PRT
   <213> Artificial sequence
<220>
   <223> sequence is synthesized
<400> 5
   Arg Phe Thr Ile Ser Ala Asp Thr Ser Lys Asn Thr Ala Tyr Leu
   1 5 10 15
   Gln Met Asn Ser Leu Arg Ala Glu Asp Thr Ala Val Tyr Tyr Cys
   20 25 30
   Ala Arg
<210> 6
   <211> 11
   <212> PRT
   <213> Artificial sequence
<220>
   <223> sequence is synthesized
<400> 6
   Asp Gly Ser Ser Trp Asp Trp Tyr Phe Asp Val
   5 10
<210> 7
   <211> 11
   <212> PRT
   <213> Artificial sequence
<220>
   <223> sequence is synthesized
<400> 7
   Trp Gly Gln Gly Thr Leu Val Thr Val Ser Ser
   5 10
<210> 8
   <211> 23
   <212> PRT
   <213> Artificial sequence
<220>
   <223> sequence is synthesized
<400> 8
   Asp Ile Gln Met Thr Gln Ser Pro Ser Ser Leu Ser Ala Ser Val
   1 5 10 15
   Gly Asp Arg Val Thr Ile Thr Cys
   20
<210> 9
   <211> 16
   <212> PRT
   <213> Artificial sequence
<220>
   <223> sequence is synthesized
<400> 9
   Arg Ser Ser Gln Ser Ile Val His Ser Asn Gly Asn Thr Phe Leu
   1 5 10 15
   Glu
<210> 10
   <211> 16
   <212> PRT
   <213> Artificial sequence
<220>
   <223> sequence is synthesized
<400> 10
   Arg Ser Ser Gln Ser Ile Val His Ser Val Gly Asn Thr Phe Leu
   1 5 10 15
   Glu
<210> 11
   <211> 15
   <212> PRT
   <213> Artificial sequence
<220>
   <223> sequence is synthesized
<400> 11
   Trp Tyr Gln Gln Lys Pro Gly Lys Ala Pro Lys Leu Leu Ile Tyr
   1 5 10 15
<210> 12
   <211> 7
   <212> PRT
   <213> Artificial sequence
<220>
   <223> sequence is synthesized
<400> 12
   Lys Val Ser Asn Arg Phe Ser
   1               5
<210> 13
   <211> 32
   <212> PRT
   <213> Artificial sequence
<220>
   <223> sequence is synthesized
<400> 13
   Gly Val Pro Ser Arg Phe Ser Gly Ser Gly Ser Gly Thr Asp Phe
   1               5                   10                  15
   Thr Leu Thr Ile Ser Ser Leu Gln Pro Glu Asp Phe Ala Thr Tyr
                   20                  25                  30
   Tyr Cys
<210> 14
   <211> 9
   <212> PRT
   <213> Artificial sequence
<220>
   <223> sequence is synthesized
<400> 14
   Phe Gln Gly Ser Gln Phe Pro Tyr Thr
   1               5
<210> 15
   <211> 10
   <212> PRT
   <213> Artificial sequence
<220>
   <223> sequence is synthesized
<400> 15
   Phe Gly Gln Gly Thr Lys Val Glu Ile Lys
   1               5                   10
<210> 16
   <211> 120
   <212> PRT
   <213> Artificial sequence
<220>
   <223> sequence is synthesized
<400> 16
   Glu Val Gln Leu Val Glu Ser Gly Gly Gly Leu Val Gln Pro Gly
   1               5                   10                  15
   Gly Ser Leu Arg Leu Ser Cys Ala Ala Ser Gly Tyr Glu Phe Ser
                   20                  25                  30
   Arg Ser Trp Met Asn Trp Val Arg Gln Ala Pro Gly Lys Gly Leu
                   35                  40                  45
   Glu Trp Val Gly Arg Ile Tyr Pro Gly Asp Gly Asp Thr Asn Tyr
                   50                  55                  60
   Ser Gly Lys Phe Lys Gly Arg Phe Thr Ile Ser Ala Asp Thr Ser
                   65                  70                  75
   Lys Asn Thr Ala Tyr Leu Gln Met Asn Ser Leu Arg Ala Glu Asp
                   80                  85                  90
   Thr Ala Val Tyr Tyr Cys Ala Arg Asp Gly Ser Ser Trp Asp Trp
                   95                  100                 105
   Tyr Phe Asp Val Trp Gly Gln Gly Thr Leu Val Thr Val Ser Ser
                   110                 115                 120
<210> 17
   <211> 112
   <212> PRT
   <213> Artificial sequence
<220>
   <223> sequence is synthesized
<400> 17
   Asp Ile Gln Met Thr Gln Ser Pro Ser Ser Leu Ser Ala Ser Val
   1               5                   10                  15
   Gly Asp Arg Val Thr Ile Thr Cys Arg Ser Ser Gln Ser Ile Val
                   20                  25                  30
   His Ser Asn Gly Asn Thr Phe Leu Glu Trp Tyr Gln Gln Lys Pro
                   35                  40                  45
   Gly Lys Ala Pro Lys Leu Leu Ile Tyr Lys Val Ser Asn Arg Phe
                   50                  55                  60
   Ser Gly Val Pro Ser Arg Phe Ser Gly Ser Gly Ser Gly Thr Asp
                   65                  70                  75
   Phe Thr Leu Thr Ile Ser Ser Leu Gln Pro Glu Asp Phe Ala Thr
                   80                  85                  90
   Tyr Tyr Cys Phe Gln Gly Ser Gln Phe Pro Tyr Thr Phe Gly Gln
                   95                  100                 105
   Gly Thr Lys Val Glu Ile Lys
                   110
<210> 18
   <211> 112
   <212> PRT
   <213> Artificial sequence
<220>
   <223> sequence is synthesized
<400> 18
   Asp Ile Gln Met Thr Gln Ser Pro Ser Ser Leu Ser Ala Ser Val
   1               5                   10                  15
   Gly Asp Arg Val Thr Ile Thr Cys Arg Ser Ser Gln Ser Ile Val
                   20                  25                  30
   His Ser Val Gly Asn Thr Phe Leu Glu Trp Tyr Gln Gln Lys Pro
                   35                  40                  45
   Gly Lys Ala Pro Lys Leu Leu Ile Tyr Lys Val Ser Asn Arg Phe
                   50                  55                  60
   Ser Gly Val Pro Ser Arg Phe Ser Gly Ser Gly Ser Gly Thr Asp
                   65                  70                  75
   Phe Thr Leu Thr Ile Ser Ser Leu Gln Pro Glu Asp Phe Ala Thr
                   80                  85                  90
   Tyr Tyr Cys Phe Gln Gly Ser Gln Phe Pro Tyr Thr Phe Gly Gln
                   95                  100                 105
   Gly Thr Lys Val Glu Ile Lys
                   110
<210> 19
   <211> 16
   <212> PRT
   <213> Artificial sequence
<220>
   <223> sequence is synthesized
<400> 19
   Arg Ser Ser Gln Ser Ile Val His Ser Ala Gly Asn Thr Phe Leu
   1               5                   10                  15
   Glu
<210> 20
   <211> 16
   <212> PRT
   <213> Artificial sequence
<220>
   <223> sequence is synthesized
<400> 20
   Arg Ser Ser Gln Ser Ile Val His Ser Gln Gly Asn Thr Phe Leu
   1               5                   10                  15
   Glu
<210> 21
   <211> 16
   <212> PRT
   <213> Artificial sequence
<220>
   <223> sequence is synthesized
<400> 21
   Arg Ser Ser Gln Ser Ile Val His Ser Ser Gly Asn Thr Phe Leu
   1               5                   10                  15
   Glu
<210> 22
   <211> 16
   <212> PRT
   <213> Artificial sequence
<220>
   <223> sequence is synthesized
<400> 22
   Arg Ser Ser Gln Ser Ile Val His Ser Asp Gly Asn Thr Phe Leu
   1 5 10 15
   Glu
<210> 23
   <211> 16
   <212> PRT
   <213> Artificial sequence
<220>
   <223> sequence is synthesized
<400> 23
   Arg Ser Ser Gln Ser Ile Val His Ser Ile Gly Asn Thr Phe Leu
   1 5 10 15
   Glu
<210> 24
   <211> 16
   <212> PRT
   <213> Artificial sequence
<220>
   <223> sequence is synthesized
<400> 24
   Arg Ser Ser Gln Ser Ile Val His Ser Ile Gly Ala Thr Phe Leu
   1 5 10 15
   Glu
<210> 25
   <211> 16
   <212> PRT
   <213> Artificial sequence
<220>
   <223> sequence is synthesized
<400> 25
   Arg Ser Ser Gln Ser Ile Val His Ser Ile Gly Gln Thr Phe Leu
   1 5 10 15
   Glu
<210> 26
   <211> 120
   <212> PRT
   <213> Artificial sequence
<220>
   <223> sequence is synthesized
<400> 26
   Gln Val Gln Leu Gln Gln Ser Gly Pro Glu Leu Val Lys Pro Gly
   1 5 10 15
   Ala Ser Val Lys Ile Ser Cys Lys Ala Ser Gly Tyr Glu Phe Ser
   20 25 30
   Arg Ser Trp Met Asn Trp Val Lys Gln Arg Pro Gly Gln Gly Arg
   35 40 45
   Glu Trp Ile Gly Arg Ile Tyr Pro Gly Asp Gly Asp Thr Asn Tyr
   50 55 60
   Ser Gly Lys Phe Lys Gly Lys Ala Thr Leu Thr Ala Asp Lys Ser
   65 70 75
   Ser Ser Thr Ala Tyr Met Gln Leu Ser Ser Leu Thr Ser Val Asp
   80 85 90
   Ser Ala Val Tyr Phe Cys Ala Arg Asp Gly Ser Ser Trp Asp Trp
   95 100 105
   Tyr Phe Asp Val Trp Gly Ala Gly Thr Thr Val Thr Val Ser Ser
   110 115 120
<210> 27
   <211> 112
   <212> PRT
   <213> Artificial sequence
<220>
   <223> sequence is synthesized
<400> 27
   Asp Ile Leu Met Thr Gln Thr Pro Leu Ser Leu Pro Val Ser Leu
   1 5 10 15
   Gly Asp Gln Ala Ser Ile Ser Cys Arg Ser Ser Gln Ser Ile Val
   20 25 30
   His Ser Asn Gly Asn Thr Phe Leu Glu Trp Tyr Leu Gln Lys Pro
   35 40 45
   Gly Gln Ser Pro Lys Leu Leu Ile Tyr Lys Val Ser Asn Arg Phe
   50 55 60
   Ser Gly Val Pro Asp Arg Phe Ser Gly Ser Gly Ser Gly Thr Asp
   65 70 75
   Phe Thr Leu Lys Ile Ser Arg Val Glu Ala Glu Asp Leu Gly Val
   80 85 90
   Tyr Tyr Cys Phe Gln Gly Ser Gln Phe Pro Tyr Thr Phe Gly Gly
   95 100 105
   Gly Thr Lys Val Glu Ile Lys
   110
<210> 28
   <211> 112
   <212> PRT
   <213> Mus sp.
<220>
   <221> MISC_FEATURE
   <223> amino acid sequence of variable region of the heavy chain (VH) of murine monoclonal anti-CD20 antibody B-Ly1
<400> 28
   Gly Pro Glu Leu Val Lys Pro Gly Ala Ser Val Lys Ile Ser Cys Lys
   1               5                   10                  15
   Ala Ser Gly Tyr Ala Phe Ser Tyr Ser Trp Met Asn Trp Val Lys Leu
               20                  25                  30
   Arg Pro Gly Gln Gly Leu Glu Trp Ile Gly Arg Ile Phe Pro Gly Asp
           35                  40                  45
   Gly Asp Thr Asp Tyr Asn Gly Lys Phe Lys Gly Lys Ala Thr Leu Thr
       50                  55                  60
   Ala Asp Lys Ser Ser Asn Thr Ala Tyr Met Gln Leu Thr Ser Leu Thr
   65                  70                  75                  80
   Ser Val Asp Ser Ala Val Tyr Leu Cys Ala Arg Asn Val Phe Asp Gly
                   85                  90                  95
   Tyr Trp Leu Val Tyr Trp Gly Gln Gly Thr Leu Val Thr Val Ser Ala
               100                 105                 110
<210> 29
   <211> 103
   <212> PRT
   <213> Mus sp.
<220>
   <221> MISC_FEATURE
   <223> amino acid sequence of variable region of the light chain (VL) of murine monoclonal anti-CD20 antibody B-Ly1
<400> 29
   Asn Pro Val Thr Leu Gly Thr Ser Ala Ser Ile Ser Cys Arg Ser Ser
   1               5                   10                  15
   Lys Ser Leu Leu His Ser Asn Gly Ile Thr Tyr Leu Tyr Trp Tyr Leu
               20                  25                  30
   Gln Lys Pro Gly Gln Ser Pro Gln Leu Leu Ile Tyr Gln Met Ser Asn
           35                  40                  45
   Leu Val Ser Gly Val Pro Asp Arg Phe Ser Ser Ser Gly Ser Gly Thr
       50                  55                  60
   Asp Phe Thr Leu Arg Ile Ser Arg Val Glu Ala Glu Asp Val Gly Val
   65                  70                  75                  80
   Tyr Tyr Cys Ala Gln Asn Leu Glu Leu Pro Tyr Thr Phe Gly Gly Gly
                   85                  90                  95
   Thr Lys Leu Glu Ile Lys Arg
               100
<210> 30
   <211> 119
   <212> PRT
   <213> Artificial
<220>
   <223> amino acid sequences of variable region of the heavy chain (VH) of humanized B-Ly1 antibody (B-HH2)
<400> 30
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 31
   <211> 119
   <212> PRT
   <213> Artificial
<220>
   <223> amino acid sequences of variable region of the heavy chain (VH) of humanized B-Ly1 antibody (B-HH3)
<400> 31
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Leu Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 32
   <211> 119
   <212> PRT
   <213> Artificial
<220>
   <223> amino acid sequences of variable region of the heavy chain (VH) of humanized B-Ly1 antibody (B-HH4)
<400> 32
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 33
   <211> 119
   <212> PRT
   <213> Artificial
<220>
   <223> amino acid sequences of variable region of the heavy chain (VH) of humanized B-Ly1 antibody (B-HH5)
<400> 33
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Ser Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 34
   <211> 119
   <212> PRT
   <213> Artificial
<220>
   <223> amino acid sequences of variable region of the heavy chain (VH) of humanized B-Ly1 antibody (B-HH6)
<400> 34
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Ile Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 35
   <211> 119
   <212> PRT
   <213> Artificial
<220>
   <223> amino acid sequences of variable region of the heavy chain (VH) of humanized B-Ly1 antibody (B-HH7)
<400> 35
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Ile Ser Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 36
   <211> 119
   <212> PRT
   <213> Artificial
<220>
   <223> amino acid sequences of variable region of the heavy chain (VH) of humanized B-Ly1 antibody (B-HH8)
<400> 36
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Tyr Ser
   20 25 30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 37
   <211> 119
   <212> **PRT**
   <213> Artificial
<220>
   <223> amino acid sequences of variable region of the heavy chain (VH) of humanized B-Ly1 antibody (B-HH9)
<400> 37
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 38
   <211> 119
   <212> PRT
   <213> Artificial
<220>
   <223> amino acid sequences of variable region of the heavy chain (VH) of humanized B-Ly1 antibody (B-HL8)
<400> 38
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 39
   <211> 119
   <212> PRT
   <213> Artificial
<220>
   <223> amino acid sequences of variable region of the heavy chain (VH) of humanized B-Ly1 antibody (B-HL10)
<400> 39
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Ala Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 40
   <211> 119
   <212> PRT
   <213> Artificial
<220>
   <223> amino acid sequences of variable region of the heavy chain (VH) of humanized B-Ly1 antibody (B-HL11)
<400> 40
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 41
   <211> 119
   <212> PRT
   <213> Artificial
<220>
   <223> amino acid sequences of variable region of the heavy chain (VH) of humanized B-Ly1 antibody (B-HL12)
<400> 41
   Glu Val Gln Leu Val Glu Ser Gly Ala Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 42
   <211> 119
   <212> PRT
   <213> Artificial
<220>
   <223> amino acid sequences of variable region of the heavy chain (VH) of humanized B-Ly1 antibody (B-HL13)
<400> 42
   Glu Val Gln Leu Val Glu Ser Gly Gly Gly Val Val Lys Pro Gly Gly
   1 5 10 15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 43
   <211> 119
   <212> PRT
   <213> Artificial
<220>
   <223> amino acid sequences of variable region of the heavy chain (VH) of humanized B-Ly1 antibody (B-HL14)
<400> 43
   Glu Val Gln Leu Val Glu Ser Gly Gly Gly Leu Lys Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 44
   <211> 119
   <212> PRT
   <213> Artificial
<220>
   <223> amino acid sequences of variable region of the heavy chain (VH) of humanized B-Ly1 antibody (B-HL15)
<400> 44
   Glu Val Gln Leu Val Glu Ser Gly Gly Gly Leu Val Lys Pro Gly Ser
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 45
   <211> 119
   <212> PRT
   <213> Artificial
<220>
   <223> amino acid sequences of variable region of the heavy chain (VH) of humanized B-Ly1 antibody (B-HL16)
<400> 45
   Glu Val Gln Leu Val Glu Ser Gly Gly Gly Leu Val Lys Pro Gly Gly
   1 5 10 15
   Ser Leu Arg Val Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 46
   <211> 119
   <212> PRT
   <213> Artificial
<220>
   <223> amino acid sequences of variable region of the heavy chain (VH) of humanized B-Ly1 antibody (B-HL17)
<400> 46
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 47
   <211> 115
   <212> PRT
   <213> Artificial
<220>
   <223> amino acid sequences of variable region of the light chain (VL) of humanized B-Ly1 antibody B-KV1
<400> 47
   Asp Ile Val Met Thr Gln Thr Pro Leu Ser Leu Pro Val Thr Pro Gly
   1               5                   10                  15
   Glu Pro Ala Ser Ile Ser Cys Arg Ser Ser Lys Ser Leu Leu His Ser
   20 25 30
   Asn Gly Ile Thr Tyr Leu Tyr Trp Tyr Leu Gln Lys Pro Gly Gln Ser
   35 40 45
   Pro Gln Leu Leu Ile Tyr Gln Met Ser Asn Leu Val Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ala Gln Asn
   85 90 95
   Leu Glu Leu Pro Tyr Thr Phe Gly Gly Gly Thr Lys Val Glu Ile Lys
   100 105 110
   Arg Thr Val
   115
<210> 48
   <211> 113
   <212> PRT
   <213> Artificial sequence
<220>
   <223> sequence is synthesized
<400> 48
   Glu Val Gln Leu Val Glu Ser Gly Gly Gly Leu Val Gln Pro Gly
   1 5 10 15
   Gly Ser Leu Arg Leu Ser Cys Ala Ala Ser Gly Phe Thr Phe Ser
   20 25 30
   Ser Tyr Ala Met Ser Trp Val Arg Gln Ala Pro Gly Lys Gly Leu
   35 40 45
   Glu Trp Val Ser Val Ile Ser Gly Asp Gly Gly Ser Thr Tyr Tyr
   50 55 60
   Ala Asp Ser Val Lys Gly Arg Phe Thr Ile Ser Arg Asp Asn Ser
   65 70 75
   Lys Asn Thr Leu Tyr Leu Gln Met Asn Ser Leu Arg Ala Glu Asp
   80 85 90
   Thr Ala Val Tyr Tyr Cys Ala Arg Gly Phe Asp Tyr Trp Gly Gln
   95 100 105
   Gly Thr Leu Val Thr Val Ser Ser
   110
<210> 49
   <211> 107
   <212> PRT
   <213> Artificial sequence
<220>
   <223> sequence is synthesized
<400> 49
   Asp Ile Gln Met Thr Gln Ser Pro Ser Ser Leu Ser Ala Ser Val
   1               5                   10                  15
   Gly Asp Arg Val Thr Ile Thr Cys Arg Ala Ser Gln Ser Ile Ser
                   20                  25                  30
   Asn Tyr Leu Ala Trp Tyr Gln Gln Lys Pro Gly Lys Ala Pro Lys
                   35                  40                  45
   Leu Leu Ile Tyr Ala Ala Ser Ser Leu Glu Ser Gly Val Pro Ser
                   50                  55                  60
   Arg Phe Ser Gly Ser Gly Ser Gly Thr Asp Phe Thr Leu Thr Ile
                   65                  70                  75
   Ser Ser Leu Gln Pro Glu Asp Phe Ala Thr Tyr Tyr Cys Gln Gln
                   80                  85                  90
   Tyr Asn Ser Leu Pro Trp Thr Phe Gly Gln Gly Thr Lys Val Glu
                   95                  100                 105
   Ile Lys
<210> 50
   <211> 219
   <212> PRT
   <213> Artificial sequence
<220>
   <223> sequence is synthesized
<400> 50
   Asp Ile Gln Met Thr Gln Ser Pro Ser Ser Leu Ser Ala Ser Val
   1               5                   10                  15
   Gly Asp Arg Val Thr Ile Thr Cys Arg Ser Ser Gln Ser Ile Val
               20                  25                  30
   His Ser Val Gly Asn Thr Phe Leu Glu Trp Tyr Gln Gln Lys Pro
           35                  40                  45
   Gly Lys Ala Pro Lys Leu Leu Ile Tyr Lys Val Ser Asn Arg Phe
       50                  55                  60
   Ser Gly Val Pro Ser Arg Phe Ser Gly Ser Gly Ser Gly Thr Asp
   65                  70                  75
   Phe Thr Leu Thr Ile Ser Ser Leu Gln Pro Glu Asp Phe Ala Thr
                   80                  85                  90
   Tyr Tyr Cys Phe Gln Gly Ser Gln Phe Pro Tyr Thr Phe Gly Gln
               95                  100                 105
   Gly Thr Lys Val Glu Ile Lys Arg Thr Val Ala Ala Pro Ser Val
           110                 115                 120
   Phe Ile Phe Pro Pro Ser Asp Glu Gln Leu Lys Ser Gly Thr Ala
       125                 130                 135
   Ser Val Val Cys Leu Leu Asn Asn Phe Tyr Pro Arg Glu Ala Lys
   140                 145                 150
   Val Gln Trp Lys Val Asp Asn Ala Leu Gln Ser Gly Asn Ser Gln
                   155                 160                 165
   Glu Ser Val Thr Glu Gln Asp Ser Lys Asp Ser Thr Tyr Ser Leu
               170                 175                 180
   Ser Ser Thr Leu Thr Leu Ser Lys Ala Asp Tyr Glu Lys His Lys
           185                 190                 195
   Val Tyr Ala Cys Glu Val Thr His Gln Gly Leu Ser Ser Pro Val
       200                 205                 210
   Thr Lys Ser Phe Asn Arg Gly Glu Cys
   215
<210> 51
   <211> 450
   <212> PRT
   <213> Artificial sequence
<220>
   <223> sequence is synthesized
<400> 51
   Glu Val Gln Leu Val Glu Ser Gly Gly Gly Leu Val Gln Pro Gly
    1               5                   10                  15
   Gly Ser Leu Arg Leu Ser Cys Ala Ala Ser Gly Tyr Glu Phe Ser
                   20                  25                  30
   Arg Ser Trp Met Asn Trp Val Arg Gln Ala Pro Gly Lys Gly Leu
                   35                  40                  45
   Glu Trp Val Gly Arg Ile Tyr Pro Gly Asp Gly Asp Thr Asn Tyr
                   50                  55                  60
   Ser Gly Lys Phe Lys Gly Arg Phe Thr Ile Ser Ala Asp Thr Ser
                   65                  70                  75
   Lys Asn Thr Ala Tyr Leu Gln Met Asn Ser Leu Arg Ala Glu Asp
                   80                  85                  90
   Thr Ala Val Tyr Tyr Cys Ala Arg Asp Gly Ser Ser Trp Asp Trp
                   95                  100                 105
   Tyr Phe Asp Val Trp Gly Gln Gly Thr Leu Val Thr Val Ser Ser
                   110                 115                 120
   Ala Ser Thr Lys Gly Pro Ser Val Phe Pro Leu Ala Pro Ser Ser
                   125                 130                 135
   Lys Ser Thr Ser Gly Gly Thr Ala Ala Leu Gly Cys Leu Val Lys
                   140                 145                 150
   Asp Tyr Phe Pro Glu Pro Val Thr Val Ser Trp Asn Ser Gly Ala
                   155                 160                 165
   Leu Thr Ser Gly Val His Thr Phe Pro Ala Val Leu Gln Ser Ser
                   170                 175                 180
   Gly Leu Tyr Ser Leu Ser Ser Val Val Thr Val Pro Ser Ser Ser
                   185                 190                 195
   Leu Gly Thr Gln Thr Tyr Ile Cys Asn Val Asn His Lys Pro Ser
                   200                 205                 210
   Asn Thr Lys Val Asp Lys Lys Val Glu Pro Lys Ser Cys Asp Lys
                   215                 220                 225
   Thr His Thr Cys Pro Pro Cys Pro Ala Pro Glu Leu Leu Gly Gly
                   230                 235                 240
   Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser
                   260                 265                 270
   His Glu Asp Pro Glu Val Lys Phe Asn Trp Tyr Val Asp Gly Val
                   275                 280                 285
   Glu Val His Asn Ala Lys Thr Lys Pro Arg Glu Glu Gln Tyr Asn
                   290                 295                 300
   Ser Thr Tyr Arg Val Val Ser Val Leu Thr Val Leu His Gln Asp
                   305                 310                 315
   Trp Leu Asn Gly Lys Glu Tyr Lys Cys Lys Val Ser Asn Lys Ala
                   320                 325                 330
   Leu Pro Ala Pro Ile Glu Lys Thr Ile Ser Lys Ala Lys Gly Gln
                   335                 340                 345
   Pro Arg Glu Pro Gln Val Tyr Thr Leu Pro Pro Ser Arg Glu Glu
                   350                 355                 360
   Met Thr Lys Asn Gln Val Ser Leu Thr Cys Leu Val Lys Gly Phe
                   365                 370                 375
   Tyr Pro Ser Asp Ile Ala Val Glu Trp Glu Ser Asn Gly Gln Pro
                   380                 385                 390
   Glu Asn Asn Tyr Lys Thr Thr Pro Pro Val Leu Asp Ser Asp Gly
                   395                 400                 405
   Ser Phe Phe Leu Tyr Ser Lys Leu Thr Val Asp Lys Ser Arg Trp
                   410                 415                 420
   Gln Gln Gly Asn Val Phe Ser Cys Ser Val Met His Glu Ala Leu
                   425                 430                 435
   His Asn His Tyr Thr Gln Lys Ser Leu Ser Leu Ser Pro Gly Lys
                   440                 445                 450

## Description

The present invention is directed to the combination therapy of an afucosylated CD20 antibody with a CD22 antibody-drug conjugate for the treatment of cancer.

### Background of the Invention

### Afucosylated antibodies

Cell-mediated effector functions of monoclonal antibodies can be enhanced by engineering their oligosaccharide component as described in Umaña, P., et al., Nature Biotechnol. 17 (1999) 176-180; and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A., and Morrison, S.L., Trends Biotechnol. 15 (1997) 26-32). Umaña, P., et al.. Nature Biotechnol. 17 (1999) 176-180 and WO 99/154342 showed that overexpression in Chinese hamster ovary (CHO) cells of β(1,4)-N-acetylglucosaminyltransferase III ("GnTIII"), a glycosyltransferase catalyzing the formation of bisected oligosaccharides, significantly increases the in vitro ADCC activity of antibodies. Alterations in the composition of the N297 carbohydrate or its elimination affect also binding to Fc binding to FcyR and C1 q (Umaña, P., et al., Nature Biotechnol. 17 (1999) 176-180; Davies, J., et al., Biotechnol. Bioeng. 74 (2001) 288-294; Mimura, Y., et al., J. Biol. Chem. 276 (2001) 45539-45547; Radaev, S., et al., J. Biol. Chem. 276 (2001) 16478-16483; Shields, R.L., et al., J. Biol. Chem. 276 (2001) 6591-6604; Shields, R.L., et al., J. Biol. Chem. 277 (2002) 26733-26740; Simmons, L.C., et al., J. Immunol. Methods 263 (2002) 133-147).

Studies discussing the activities of afucosylated and fucosylated antibodies, including anti-CD20 antibodies, have been reported (e.g., Iida, S., et al., Clin. Cancer Res. 12 (2006) 2879-2887; Natsume, A., et al., J. Immunol. Methods 306 (2005) 93-103; Satoh, M., et al., Expert Opin. Biol. Ther. 6 (2006) 1161-1173; Kanda, Y., et al., Biotechnol. Bioeng. 94 (2006) 680-688; Davies, J., et al., Biotechnol. Bioeng. 74 (2001) 288-294.

### CD20 and anti CD20 antibodies

The CD20 molecule (also called human B-lymphocyte-restricted differentiation antigen or Bp35) is a hydrophobic transmembrane protein located on pre-B and mature B lymphocytes that has been described extensively (Valentine, M.A., et al., J. Biol. Chem. 264 (1989) 11282-11287; and Einfeld, D.A., et al., EMBO J. 7 (1988) 711-717; Tedder, T.F., et al., Proc. Natl. Acad. Sci. U.S.A. 85 (1988) 208-212; Stamenkovic, I., et al., J. Exp. Med. 167 (1988) 1975-1980; Tedder, T.F., et al., J. Immunol. 142 (1989) 2560-2568). CD20 is expressed on greater than 90 % of B cell non-Hodgkin's lymphomas (NHL) (Anderson, K.C., et al., Blood 63 (1984) 1424-1433) but is not found on hematopoietic stem cells, pro-B cells, normal plasma cells, or other normal tissues (Tedder, T.F., et al., J, Immunol. 135 (1985) 973- 979).

There exist two different types of anti-CD20 antibodies differing significantly in their mode of CD20 binding and biological activities (Cragg, M.S., et al., Blood 103 (2004) 2738-2743; and Cragg, M.S., et al., Blood 101 (2003) 1045-1052). Type I antibodies, as, e.g., rituximab (a non-afucosylated antibody with an amount of fucose of 85 % or higher), are potent in complement mediated cytotoxicity.

Type II antibodies, as e.g. Tositumomab (B1), 11B8, AT80 or humanized B-Ly1 antibodies, effectively initiate target cell death via caspase-independent apoptosis with concomitant phosphatidylserine exposure.

### CD22 antibody-drug conjugates

Other B-cell antigens, such as CD19, CD22, and CD52, represent targets of therapeutic potential for treatment of lymphoma (Grillo-Lopez A.J. et al., Curr Pharm Biotechnol, 2:301-11, (2001)). CD22 is a 135-kDa B-cell-restricted sialoglycoprotein expressed on the B-cell surface only at the mature stages of differentiation (Dorken, B. et al., J. Immunol. 136:4470-4479 (1986)). The predominant form of CD22 in humans is CD22beta which contains seven immunoglobulin superfamily domains in the extracellular domain (Wilson, G.L. et al., J. Exp. Med. 173:137-146 (1991)). A variant form, CD22 alpha, lacks immunoglobulin superfamily domains 3 and 4 (Stamenkovic, I. and Seed, B., Nature 345:74-77 (1990)). Ligand-binding to human CD22 has been shown to be associated with immunoglobulin superfamily domains 1 and 2 (also referred to as epitopes 1 and 2) (Engel, P. et al., J. Exp. Med. 181:1581-1586, 1995).

In B-cell NHL, CD22 expression ranges from 91% to 99% in the aggressive and indolent populations, respectively (Cesano, A. et al., Blood 100:350a (2002)). CD22 may function both as a component of the B-cell activation complex (Sato, S. et al., Semin. Immunol. 10:287-296 (1998)) and as an adhesion molecule (Engel, Pl t al., J. Immunol. 150:4719-4732 (1993)). The B cells of CD22-deficient mice have a shorter life span and enhanced apoptosis, which suggests a key role of this antigen in B-cell survival (Otipoby, K.L. et al., Nature (Lond) 384:634-637 (1996)). After binding with its natural ligand(s) or antibodies, CD22 is rapidly internalized, providing a potent co-stimulatory signal in primary B cells and proapoptotic signals in neoplastic B cells (Sato, S. et al., Immunity 5:551-562 (1996)).

Anti-CD22 antibodies have been studied as potential therapies for B cell cancers and other B cell proliferative diseases. Such anti-CD22 antibodies include RFB4 Mansfield, E. et al., Blood 90:2020-2026 (1997)), CMC-544 (DiJoseph, J.F., Blood 103:1807-1814 (2004)) and LL2 (Pawlak-Byczkowska, E.J. et al., Cancer Res. 49:4568-4577 (1989)). The LL2 antibody (formerly called HPB-2) is an IgG2a mouse monoclonal antibody directed against the CD22 antigen (Pawlak-Byczkowska, E.J. et al. (1989), supra). In vitro immunohistological evaluations demonstrated reactivity of the LL2 antibody with 50 of 51 B-cell NHL specimens tested, but not with other malignancies or normal nonlymphoid tissues (Pawlak-Byczkowska (1989), supra; Stein, R. et al., Cancer Immunol. Immunother. 37:293-298 (1993)).

The use of antibody-drug conjugates for the local delivery of cytotoxic or cytostatic agents, i.e. drugs to kill or inhibit tumor cells in the treatment of cancer (Syrigos and Epenetos (1999) Anticancer Research 19:605-614; Niculescu-Duvaz and Springer (1997) Adv. Drg Del. Rev. 26:151-172; U.S. patent 4975278) allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al. (ed.s), pp. 475-506). Maximal efficacy with minimal toxicity is sought thereby. Both polyclonal antibodies and monoclonal antibodies have been reported as useful in these strategies (Rowland et al., (1986) Cancer Immunol. Immunother., 21:183-87). Drugs used in these methods include daunomycin, doxorubicin, methotrexate, and vindesine (Rowland et al., Cancer Immunol. Immunother. 21:183-87 (1986)). Toxins used in antibody-toxin conjugates include bacterial toxins such as diphtheria toxin, plant toxins such as ricin, small molecule toxins such as geldanamycin (Kerr et al (1997) Bioconjugate Chem. 8(6):781-784; Mandler et al (2000) Journal of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may affect their cytotoxic and cytostatic effects by mechanisms including tubulin binding, DNA binding, or topoisomerase inhibition (Meyer, D.L. and Senter, P.D. "Recent Advances in Antibody Drug Conjugates for Cancer Therapy" in Annual Reports in Medicinal Chemistry, Vol 38 (2003) Chapter 23, 229-237). Some cytotoxic drugs tend to be inactive or less active when conjugated to large antibodies or protein receptor ligands.

ZEVALIN® (ibritumomab tiuxetan, Biogen/Idec) is an antibody-radioisotope conjugate composed of a murine IgG1 kappa monoclonal antibody directed against the CD20 antigen found on the surface of normal and malignant B lymphocytes and ¹¹¹In or ⁹⁰Y radioisotope bound by a thiourea linker-chelator (Wiseman et al (2000) Eur. Jour. Nucl. Med. 27(7):766-77; Wiseman et al (2002) Blood 99(12):4336-42; Witzig et al (2002) J. Clin. Oncol. 20(10):2453-63; Witzig et al (2002) J. Clin. Oncol. 20(15):3262-69). Although ZEVALIN has activity against B-cell non-Hodgkin's Lymphoma (NHL), administration results in severe and prolonged cytopenias in most patients. MYLOTARG™ (gemtuzumab ozogamicin, Wyeth Pharmaceuticals), an antibody drug conjugate composed of a hu CD33 antibody linked to calicheamicin, was approved in 2000 for the treatment of acute myeloid leukemia by injection (Drugs of the Future (2000) 25(7):686; US Patent Nos. 4970198; 5079233; 5585089; 5606040; 5693762; 5739116; 5767285; 5773001). Cantuzumab mertansine (Immunogen, Inc.), an antibody drug conjugate composed of the huC242 antibody linked via the disulfide linker SPP to the maytansinoid drug moiety, DM1, is being developed for the treatment of cancers that express CanAg antigen, such as colon, pancreatic, gastric, and others. MLN-2704 (Millennium Pharm., BZL Biologics, Immunogen Inc.), an antibody drug conjugate composed of the anti-prostate specific membrane antigen (PSMA) monoclonal antibody linked to the maytansinoid drug moiety, DM1, is under development for the potential treatment of prostate tumors. The same maytansinoid drug moiety, DM1, was linked through a non-disulfide linker, SMCC, to a mouse murine monoclonal antibody, TA.1 (Chari et al. (1992) Cancer Research 52:127-131). This conjugate was reported to be 200-fold less potent than the corresponding disulfide linker conjugate. The SMCC linker was considered therein to be "noncleavable. "

Several short peptidic compounds have been isolated from the marine mollusk, Dolabella auricularia, and found to have biological activity (Pettit et al (1993) Tetrahedron 49:9151; Nakamura et al (1995) Tetrahedron Letters 36:5059-5062; Sone et al (1995) Journal Org Chem. 60:4474). Analogs of these compounds have also been prepared, and some were found to have biological activity (for a review, see Pettit et al (1998) Anti-Cancer Drug Design 13:243-277). For example, auristatin E (US 5635483) is a synthetic analogue of the marine natural product Dolastatin 10, an agent that inhibits tubulin polymerization by binding to the same site on tubulin as the anticancer drug vincristine (G. R. Pettit, (1997) Prog. Chem. Org. Nat. Prod. 70:1-79). Dolastatin 10, auristatin PE, and auristatin E are linear peptides having four amino acids, three of which are unique to the dolastatin class of compounds, and a C-terminal amide.

The auristatin peptides, auristain E (AE) and monomethylauristatin (MMAE), synthetic analogs of dolastatin, were conjugated to: (i) chimeric monoclonal antibodies cBR96 (specific to Lewis Y on carcinomas); (ii) cAC10 which is specific to CD30 on hematological malignancies (Klussman, et al (2004), Bioconjugate Chemistry 15(4):765-773; Doronina et al (2003) Nature Biotechnology 21(7):778-784; "Monomethylvaline Compounds Capable of Conjugation to Ligands"; Francisco et al (2003) Blood 102(4):1458-1465; US 2004/0018194; (iii) anti-CD20 antibodies such as Rituxan® (rituximab) (WO 04/032828) for the treatment of CD20-expressing cancers and immune disorders; (iv) anti-EphB2 antibodies 2H9 and anti-IL-8 for treatment of colorectal cancer (Mao, et al (2004) Cancer Research 64(3):781-788); (v) E-selectin antibody (Bhaskar et al (2003) Cancer Res. 63:6387-6394); and (vi) other anti-CD30 antibodies (WO 03/043583). Monomethylauristatin (MMAE) has also been conjugated to 2H9, an antibody against EphB2R which is a type 1 TM tyrosine kinase receptor with close homology between mouse and human, and is over-expressed in colorectal cancer cells (Mao et al (2004) Cancer Res. 64:781-788).

Monomethylauristatin MMAF, a variant of auristatin E (MMAE) with a phenylalanine at the C-terminus (US 5767237; US 6124431), has been reported to be less potent than MMAE, but more potent when conjugated to monoclonal antibodies (Senter et al, Proceedings of the American Association for Cancer Research, Volume 45, Abstract Number 623, presented March 28, 2004). Auristatin F phenylene diamine (AFP); a phenylalanine variant of MMAE was linked to an anti-CD70 mAb, 1F6, through the C-terminus of 1F6 via a phenylene diamine spacer (Law et al, Proceedings of the American Association for Cancer Research, Volume 45, Abstract Number 625, presented March 28, 2004).

Anti-CD22 antibody-toxin conjugates have also been studied as potential therapeutic compounds. For example, early reports described ricin A chain-containing immunotoxins directed against anti-CD22 as potential anti-cancer agents (May, R.D. et al., Chemical Abstracts 106(21):168656x pages 35-36 (1987); Ghetie, M.A. et al., Cancer Research 48:2610-2617 (1988); and Amlot, P.L. et al., Blood 82(9):2624-2633 (1993)). Where the toxin was a radioisotope, Epratuzumab, the humanized (CDR-grafted) IgG1 version of LL2, has shown evidence of therapeutic activity for the radioimmunoconjugate (Juweid, M.E. et al., Clin. Cancer Res. 5 (Suppl 10):3292s-3303s (1999); Griffiths, G.L. et al., J. Nucl. Med. 44:77-84 (2003); Linden, O. et al., Clin. Cancer Res. 5(suppl 10):3287s-3291s (1999))..

The Antibody drug conjugate (ADC) Anti-CD22-vc-MMAE was developed by Genentech and already proved in different xenograft models to be very efficacious (Poison AG, Williams M et al., Anti-CD22-MCC-DM1: an antibody-drug conjugate with a stable linker for the treatment of non-Hodgkin's lymphoma. Leukemia. 2010 Sep;24(9):1566-73. Epub 2010 Jul 1). Monomethyl Auristatin E (MMAE), a synthetic analog of the natural product dolastin 10, is a microtubule inhibitor. This cytotoxic drug is conjugated to the anti-CD22-Antibody. After binding of the ADC to the tumor cell, it is internalized and degraded releasing MMAE and therefore inducing cell death.

There exists a need in the art for additional drugs to treat various B cell-related cancers such as lymphomas such as non-Hodgkin's lymphoma and other B cell proliferative disorders. Particularly useful drugs for this purpose include B cell targeted anti-CD22 antibody-drug conjugates having a significantly lower toxicity, yet useful therapeutic efficiency. These and other limitations and problems of the past are addressed by the present invention.

Ogura et al. (2012) Cancer Science, Vol 103, No 5, pages 933-938 describes the treatment of relapsed/refractory B-cell non Hodgkin's Lymphoma with rituximab and an anti CD22 antibody-drug conjugate, namely, inotuzumab ozgamicin. Fayad et al. (2013) Journal of Clinical Oncology, Vol 31, No 5, pages 573-583 describe the treatment of replapsed/refractory B-cell non Hodgkin's Lymphoma with rituximab and an anti CD22 antibody-drug conjugate, namely inotuzumab ozogamicin having a high response rate. An Open-Label, Multicenter, Phase I study ((2013), XP055128133) describes the treatment of replapsed/refractory B-cell non Hodgkin's Lymphoma and Chronic Lymphocytic Leukemia with rituximab and an anti CD22 antibody-drug conjugate, namely inotuzumab ozogamicin. A Radomized, Open-Label, Multicenter, Phase II Trial ((2013), pages 1-9, XP055128138) describes the treatment of replapsed/refractory B-cell non Hodgkin's Lymphoma with rituximab and an anti CD22 antibody-drug conjugate, namely inotuzumab ozogamicin. WO01/97858 describes the treatment of replapsed/refractory B-cell non Hodgkin's Lymphoma with rituximab and an anti CD22 radio-labelled (⁹⁰Y) antibody. Mattes et al. (2008), Clinical Cancer Research, Vol 14, No 19 pages 6154-6160, describe the treatment of advanced lymphoma with veltuzumab and anti ⁹⁰Y CD22 epratuzumab. The combination shows a synergistiv effect compared to either antibody alone. US2011305631 describes the treatment of hematologic tumors with epratuzumab conjugated to SN-38 and veltuzumab. Umana et al (2007) Blood, Vol 110, No 11 describe GA101, a glycoengineered anti CD20 antibody having superior anti tumor efficacy compared to rituximab.

The recitation of any reference in this application is not an admission that the reference is prior art to this application.

### Summary of the Invention

We have now found out that the combination of an afucosylated anti-CD20 antibody with a CD22 antibody-drug conjugate showed significantly enhanced antiproliferative effects.

One aspect of the invention is an afucosylated anti-CD20 antibody with an amount of fucose of 60% or less of the total amount of oligosaccharides (sugars) at Asn297, for the treatment of cancer in combination with a CD22 antibody-drug conjugate.

Another aspect of the invention is the use of an afucosylated anti-CD20 antibody with an amount of fucose of 60% or less of the total amount of oligosaccharides (sugars) at Asn297, for the manufacture of a medicament for the treatment of cancer in combination with a CD22 antibody-drug conjugate.

Another aspect of the disclosure is a method of treatment of patient suffering from cancer by administering an afucosylated anti-CD20 antibody with an amount of fucose of 60% or less of the total amount of oligosaccharides (sugars) at Asn297, in combination with a CD22 antibody-drug conjugate, to a patient in the need of such treatment.

In one aspect, the amount of fucose is between 40% and 60% of the total amount of oligosaccharides (sugars) at Asn297. In another aspect, the amount of fucose is 0% of the total amount of oligosaccharides (sugars) at Asn297.

In one aspect, the afucosylated anti-CD20 antibody is an IgG1 antibody. In another embodiment, said cancer is a CD20 expressing cancer, preferably a lymphoma or lymphocytic leukemia. In one aspect said afucosylated anti-CD20 antibody is a humanized B-Ly1 antibody. In a specific embodiment, the anti-CD20 antibody is obinutuzumab (recommended INN, WHO Drug Information, Vol. 26, No. 4, 2012, p. 453). As used herein, obinutuzumab is synonymous for GA101 or RO5072759. This replaces all previous versions (e.g. Vol. 25, No. 1, 2011, p.75-76), and is formerly known as afutuzumab (recommended INN, WHO Drug Information, Vol. 23, No. 2, 2009, p. 176;Vol. 22, No. 2, 2008, p. 124).

In one aspect, the CD22 antibody in the CD22 antibody-drug conjugate comprises (a) an HVR-L1 comprising an amino acid sequence selected from SEQ ID NOs:9, 10, 19-23, 32 and 33, and (b) at least one, two, three, four, or five HVRs selected from:
(1) an HVR-H1 comprising the amino acid sequence of SEQ ID NO:2;
(2) an HVR-H2 comprising the amino acid sequence of SEQ ID NO:4;
(3) an HVR-H3 comprising the amino acid sequence of SEQ ID NO:6;
(4) an HVR-L2 comprising the amino acid sequence of SEQ ID NO:12; and
(5) an HVR-L3 comprising an amino acid sequence of SEQ ID NO:14.

In one aspect, the CD22 antibody-drug conjugate having the formula Ab-(L-D)p, wherein
(a) Ab is the CD22 antibody as defined herein;
(b) L is a linker;
(c) D is a drug moiety.

In one aspect of the CD22 antibody-drug conjugate having the formula Ab-(L-D)p, L is selected from 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio) pentanoate (SPP), N-succinimidyl 4-(N-maleimidomethyl) cyclohexane-1 carboxylate (SMCC), and N-Succinimidyl (4-iodo-acetyl) aminobenzoate (SIAB). In one aspect of the CD22 antibody-drug conjugate having the formula Ab-(L-D)p, D is selected from the group consisting of auristatin, dolostantin, DM1, DM3, DM4, MMAE and MMAF.

In one embodiment, said CD22 antibody-drug conjugate is anti-CD22-MC-vc-PAB-MMAE. In a specific embodiment, the anti-CD22 antibody in said conjugate is 10F4.v3.

In one aspect, the afucosylated anti-CD20 antibody binds CD20 with an KD of 10⁻⁸ M to 10⁻¹³ M.

One aspect of the invention is a composition comprising an afucosylated anti-CD20 antibody with an amount of fucose of 60% or less of the total amount of oligosaccharides (sugars) at Asn297, (in one aspect an afucosylated humanized B-Ly1 antibody), and a CD22 antibody-drug conjugate. In one embodiment of the composition according to the invention, said anti-CD20 antibody is obinutuzumab. In one aspect of the composition according to the invention, said CD22 antibody in the CD22 antibody-drug conjugate comprises (a) an HVR-L1 comprising an amino acid sequence selected from SEQ ID NOs:9, 10, 19-23, 32 and 33, and (b) at least one, two, three, four, or five HVRs selected from:
(1) an HVR-H1 comprising the amino acid sequence of SEQ ID NO:2;
(2) an HVR-H2 comprising the amino acid sequence of SEQ ID NO:4;
(3) an HVR-H3 comprising the amino acid sequence of SEQ ID NO:6;
(4) an HVR-L2 comprising the amino acid sequence of SEQ ID NO:12; and
(5) an HVR-L3 comprising an amino acid sequence of SEQ ID NO:14.

In one aspect of the composition according to the invention, said CD22 antibody binds to the same epitope as an antibody selected from ATCC PTA-7621 (10F4.4.1).

In one embodiment of the composition according to the invention, one or more additional other cytotoxic, chemotherapeutic or anti-cancer agents, or compounds or ionizing radiation that enhance the effects of such agents are administered.

In one aspect of the composition according to the invention, the CD22 antibody-drug conjugate is having the formula Ab-(L-D)p, wherein
(a) Ab is the CD22 antibody as defined herein;
(b) L is a linker;
(c) D is a drug moiety.

In one aspect of the composition according to the invention, the CD22 antibody-drug conjugate is having the formula Ab-(L-D)p, wherein L is selected from 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio) pentanoate (SPP), N-succinimidyl 4-(N-maleimidomethyl) cyclohexane-1 carboxylate (SMCC), and N-Succinimidyl (4-iodo-acetyl) aminobenzoate (SIAB).

In one aspect of the composition according to the invention, the CD22 antibody-drug conjugate is having the formula Ab-(L-D)p, wherein D is selected from the group consisting of auristatin, dolostantin, DM1, DM3, DM4, MMAE and MMAF.

In one embodiment of the composition according to the invention, the CD22 antibody-drug conjugate is anti-CD22-MC-vc-PAB-MMAE for the treatment of cancer. In a specific embodiment, the anti-CD22 antibody in said conjugate is 10F4.v3. An afucosylated anti-CD20 antibody with an amount of fucose of 60% or less of the total amount of oligosaccharides (sugars) at Asn297, for the treatment of cancer in combination with a CD22 antibody-drug conjugate.

One aspect of the disclosure is a method of treatment of patient suffering from cancer by administering an afucosylated anti-CD20 antibody with an amount of fucose of 60% or less of the total amount of oligosaccharides (sugars) at Asn297, in combination with a CD22 antibody-drug conjugate, to a patient in the need of such treatment.

In one embodiment according to the disclosure, the method is characterized in that said cancer is a CD20 expressing cancer.

In one embodiment according to the disclosure, the method is characterized in that said CD20 expressing cancer is a lymphoma or lymphocytic leukemia.

In one aspect according to the disclosure, the method is characterized in that said anti-CD20 antibody is a humanized B-Ly1 antibody.

In one embodiment according to the disclosure, the method is characterized in that said anti-CD20 antibody is obinutuzumab.

In one embodiment according to the disclosure, the method is characterized in that one or more additional other cytotoxic, chemotherapeutic or anti-cancer agents, or compounds or ionizing radiation that enhance the effects of such agents are administered.

In one aspect according to the disclosure, the method is characterized in that said CD22 antibody in the CD22 antibody-drug conjugate comprises (a) an HVR-L1 comprising an amino acid sequence selected from SEQ ID NOs:9, 10, 19-23, 32 and 33, and (b) at least one, two, three, four, or five HVRs selected from:
(1) an HVR-H1 comprising the amino acid sequence of SEQ ID NO:2;
(2) an HVR-H2 comprising the amino acid sequence of SEQ ID NO:4;
(3) an HVR-H3 comprising the amino acid sequence of SEQ ID NO:6;
(4) an HVR-L2 comprising the amino acid sequence of SEQ ID NO:12; and
(5) an HVR-L3 comprising an amino acid sequence of SEQ ID NO:14.

In one aspect according to the disclosure, the method is characterized in that said CD22 antibody binds to the same epitope as an antibody selected from ATCC PTA-7621 (10F4.4.1).

In one aspect of the method according to the disclosure, the CD22 antibody-drug conjugate is having the formula Ab-(L-D)p, wherein
(a) Ab is the CD22 antibody as disclosed herein;
(b) L is a linker;
(c) D is a drug moiety.

In one aspect of the method according to the disclosure, the CD22 antibody-drug conjugate is having the formula Ab-(L-D)p, wherein L is selected from 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio) pentanoate (SPP), N-succinimidyl 4-(N-maleimidomethyl) cyclohexane-1 carboxylate (SMCC), and N-Succinimidyl (4-iodo-acetyl) aminobenzoate (SIAB).

In one aspect of the method according to the disclosure, the CD22 antibody-drug conjugate is having the formula Ab-(L-D)p, wherein D is selected from the group consisting of auristatin, dolostantin, DM1, DM3, DM4, MMAE and MMAF.

In one embodiment of the method according to the disclosure, the CD22 antibody-drug conjugate is anti-CD22-MC-vc-PAB-MMAE.

In one embodiment of the method according to the disclosure, the anti-CD22 antibody in said CD22 antibody-drug conjugate is 10F4.v3.

One aspect according to the invention is the use of an afucosylated anti-CD20 antibody with an amount of fucose of 60% or less of the total amount of oligosaccharides (sugars) at Asn297, for the manufacture of a medicament for the treatment of cancer in combination with a CD22 antibody-drug conjugate.

One embodiment of the use according to the invention is characterized in that said cancer is a CD20 expressing cancer.

One embodiment of the use according to the invention is characterized in that said CD20 expressing cancer is a lymphoma or lymphocytic leukemia.

One aspect of the use according to the invention is characterized in that said anti-CD20 antibody is a humanized B-Ly1 antibody.

One embodiment of the use according to the invention is characterized in that said anti-CD20 antibody is obinutuzumab.

One embodiment of the use according to the invention is characterized in that one or more additional other cytotoxic, chemotherapeutic or anti-cancer agents, or compounds or ionizing radiation that enhance the effects of such agents are administered.

One aspect of the use according to the invention is characterized in that said CD22 antibody in the CD22 antibody-drug conjugate comprises (a) an HVR-L1 comprising an amino acid sequence selected from SEQ ID NOs:9, 10, 19-23, 32 and 33, and (b) at least one, two, three, four, or five HVRs selected from:
(1) an HVR-H1 comprising the amino acid sequence of SEQ ID NO:2;
(2) an HVR-H2 comprising the amino acid sequence of SEQ ID NO:4;
(3) an HVR-H3 comprising the amino acid sequence of SEQ ID NO:6;
(4) an HVR-L2 comprising the amino acid sequence of SEQ ID NO:12; and
(5) an HVR-L3 comprising an amino acid sequence of SEQ ID NO:14.

One aspect of the use according to the invention is characterized in that said CD22 antibody binds to the same epitope as an antibody selected from ATCC PTA-7621 (10F4.4.1).

One aspect of the use according to the invention is characterized in that the CD22 antibody-drug conjugate is having the formula Ab-(L-D)p, wherein
(a) Ab is the CD22 antibody as disclosed herein;
(b) L is a linker;
(c) D is a drug moiety.

One aspect of the use according to the invention is characterized in that the CD22 antibody-drug conjugate is having the formula Ab-(L-D)p, wherein L is selected from 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio) pentanoate (SPP), N-succinimidyl 4-(N-maleimidomethyl) cyclohexane-1 carboxylate (SMCC), and N-Succinimidyl (4-iodo-acetyl) aminobenzoate (SIAB).

One aspect of the use according to the invention is characterized in that the CD22 antibody-drug conjugate is having the formula Ab-(L-D)p, wherein D is selected from the group consisting of auristatin, dolostantin, DM1, DM3, DM4, MMAE and MMAF.

One embodiment of the use according to the invention is characterized in that the CD22 antibody-drug conjugate is anti-CD22-MC-vc-PAB-MMAE.

One embodiment of the use according to the invention is characterized in that the anti-CD22 antibody in said CD22 antibody-drug conjugate is 10F4.v3.

One embodiment of the use according to the invention is characterized in that one or more additional other cytotoxic, chemotherapeutic or anti-cancer agents, or compounds or ionizing radiation that enhance the effects of such agents are administered.

### Description of the Figures

**Figure 1****:** Effect of obinutuzumab (GA101), rituximab, anti-CD22-ADC and the combinations of CD22 with GA101 or rituximab on a WSU-DLCL2 lymphoma model in SCID FcgR3a transgenic mice
**Figure 2****:** Statistical analysis of the data in Figure 1 by Pairwise Wilcoxon and Pairwise Log-Rank test

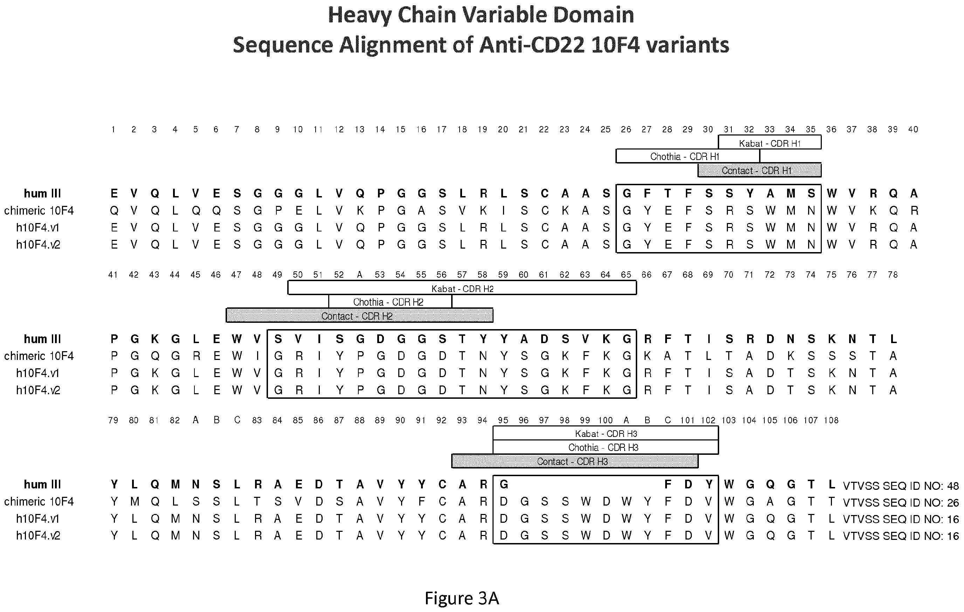

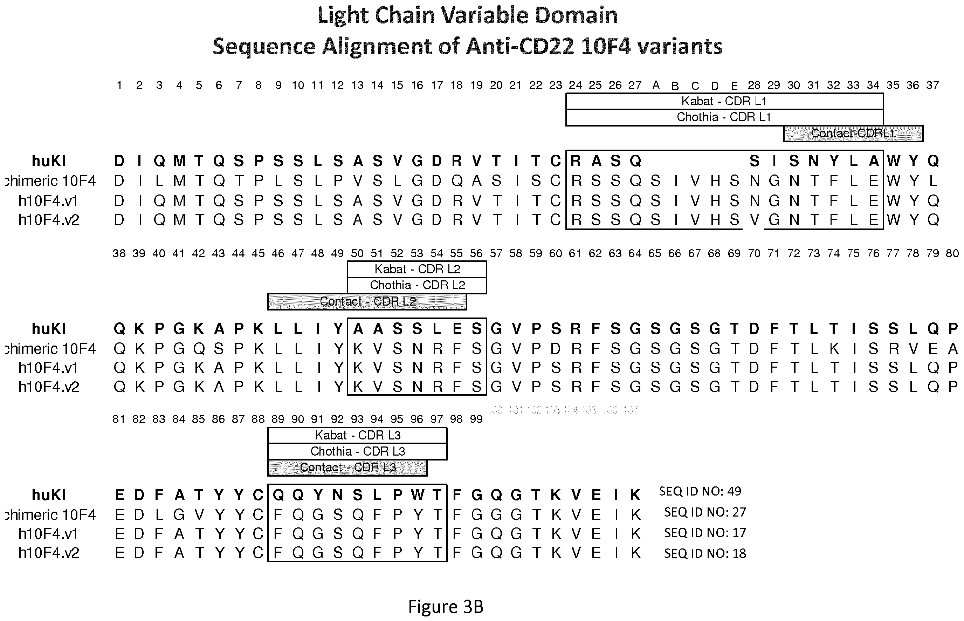

**Figure 3: Figures 3A-3B****:** **Figure 3A** depicts the amino acid sequence of the heavy chain variable region of murine 10F4 anti-CD22 antibody of the invention (m10F4) aligned with the humanized 10F4 version 1 antibody (h10F4v1) and aligned with the human subgroup III sequence. The HVRs are boxed (HVR-H1, HVR-H2, HVR-H3). The sequences bracketing the HVRs are the framework sequences (FR-H1 to FR-H4). The sequences are numbered according to Kabat numbering. The Kabat, Chothia, and contact CDRs are indicated about the boxed HVRs. **Figure 3B** depicts the amino acid sequence of the light chain variable region of murine 10F4 anti-CD22 antibody of the invention (m10F4) aligned with the humanized 10F4 version 1 antibody (h10F4v1) and aligned with the human kappa I sequence. Versions 2 and 3 of the humanized 10F4 antibody (h10F4v2 and h10F4v3) have the same amino acid sequences for the secreted mature form. The antibodies h10F4v2 and h10F4v3 differ from h10F4v1 at amino acid 28 of the HVR-L1 (N28V). The HVRs are boxed. The FR-L1, FR-L2, FR-L3, and FR-L4 sequences bracket the HVRs (HVR-L1, HVR-L2, HVR-L3). The sequences are numbered according to Kabat numbering. The Kabat, Chothia, and contact CDRs are indicated about the boxed HVRs.

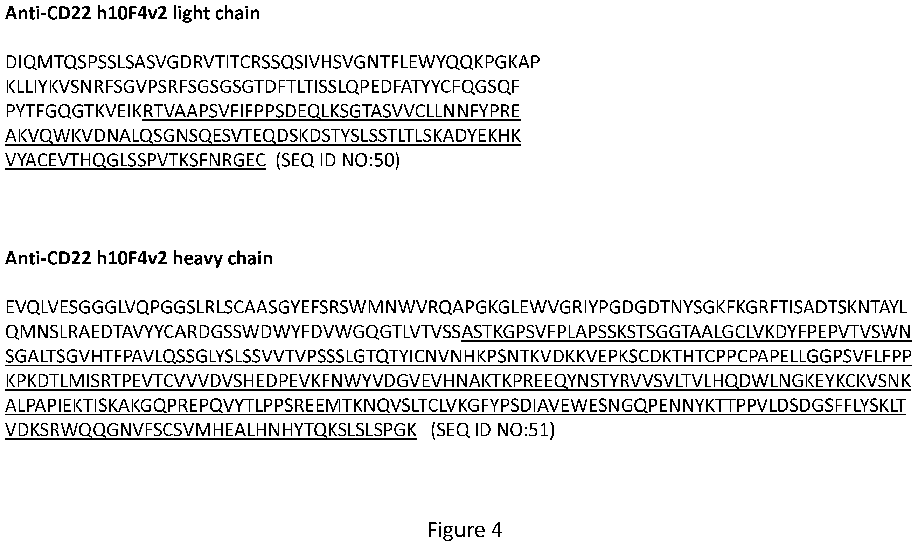

**Figure 4: Figure 4** depicts the full length amino acid sequences (variable and constant regions) of the light and heavy chains of humanized anti-CD22 antibody 10F4v2, isotype IgG1. The underlined portions depict the constant domains.

### Detailed Description of the Invention

The invention comprises an afucosylated anti-CD20 antibody of IgG1 or IgG3 isotype with an amount of fucose of 60% or less of the total amount of oligosaccharides (sugars) at Asn297, for the treatment of cancer in combination with a CD22 antibody-drug conjugate.

The invention comprises the use of an afucosylated anti-CD20 antibody of IgG1 or IgG3 isotype with an amount of fucose of 60% or less of the total amount of oligosaccharides (sugars) at Asn297, for the manufacture of a medicament for the treatment of cancer in combination with a CD22 antibody-drug conjugate.

In one aspect, the amount of fucose is between 40% and 60% of the total amount of oligosaccharides (sugars) at Asn297.

The term "antibody" encompasses the various forms of antibodies including but not being limited to whole antibodies, human antibodies, humanized antibodies and genetically engineered antibodies like monoclonal antibodies, chimeric antibodies or recombinant antibodies as well as fragments of such antibodies as long as the characteristic properties according to the invention are retained. The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. In one aspect, the human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred aspect, a murine CDR is grafted into the framework region of a human antibody to prepare the "humanized antibody." See, e.g., Riechmann, L. et al., Nature 332 (1988) 323-327; and Neuberger, M.S. et al., Nature 314 (1985) 268-270. Particularly preferred CDRs correspond to those representing sequences recognizing the antigens noted above for chimeric and bifunctional antibodies.

The term "human antibody", as used herein, is intended to include antibodies having variable and constant regions derived from human germline immunoglobulin sequences. Human antibodies are well-known in the state of the art (van Dijk, M.A., and van de Winkel, J.G., Curr. Opin. in Chem. Biol. 5 (2001) 368-374). Based on such technology, human antibodies against a great variety of targets can be produced. Examples of human antibodies are for example described in Kellermann, S.A., et al., Curr Opin Biotechnol. 13 (2002) 593-597.

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form. The recombinant human antibodies according to the invention have been subjected to in vivo somatic hypermutation. Thus, the amino acid sequences of the VH and VL regions of the recombinant antibodies are sequences that, while derived from and related to human germline VH and VL sequences, may not naturally exist within the human antibody germline repertoire in vivo.

As used herein, the term "binding" or "specifically binding" refers to the binding of the antibody to an epitope of the tumor antigen in an in vitro assay, preferably in an plasmon resonance assay (BIAcore, GE-Healthcare Uppsala, Sweden) with purified wild-type antigen. The affinity of the binding is defined by the terms ka (rate constant for the association of the antibody from the antibody/antigen complex), k_{D} (dissociation constant), and K_{D} (k_{D}/ka). Binding or specifically binding means a binding affinity (K_{D}) of 10⁻⁸ M or less, preferably 10⁻⁸ M to 10⁻¹³ M (in one aspect 10⁻⁹ M to 10⁻¹³ M). Thus, an afucosylated antibody according to the invention is specifically binding to the tumor antigen with a binding affinity (K_{D}) of 10⁻⁸ mol/l or less, preferably 10⁻⁸ M to 10⁻¹³ M (in one aspect 10⁻⁹ M to 10⁻¹³ M).

The term "nucleic acid molecule", as used herein, is intended to include DNA molecules and RNA molecules. A nucleic acid molecule may be single-stranded or double-stranded, but preferably is double-stranded DNA.

The "constant domains" are not involved directly in binding the antibody to an antigen but are involved in the effector functions (ADCC, complement binding, and CDC).

The "variable region" (variable region of a light chain (VL), variable region of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a b-sheet conformation and the CDRs may form loops connecting the b-sheet structure. The CDRs in each chain are held in their three-dimensional structure by the framework regions and form together with the CDRs from the other chain the antigen binding site.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat, et al., Sequences of Proteins of Immunological Interest, 5th ed., Public Health Service, National Institutes of Health, Bethesda, MD (1991), and/or those residues from a "hypervariable loop".

The term "afucosylated antibody" refers to an antibody of IgG1 or IgG3 isotype (preferably of IgG1 isotype) with an altered pattern of glycosylation in the Fc region at Asn297 having a reduced level of fucose residues. Glycosylation of human IgG1 or IgG3 occurs at Asn297 as core fucosylated bianntennary complex oligosaccharide glycosylation terminated with up to 2 Gal residues. These structures are designated as G0, G1 (α1,6 or α1,3) or G2 glycan residues, depending from the amount of terminal Gal residues (Raju, T.S., BioProcess Int. 1 (2003) 44-53). CHO type glycosylation of antibody Fc parts is e.g. described by Routier, F.H., Glycoconjugate J. 14 (1997) 201-207. Antibodies which are recombinantly expressed in non glycomodified CHO host cells usually are fucosylated at Asn297 in an amount of at least 85%. It should be understood that the term an afucosylated antibody as used herein includes an antibody having no fucose in its glycosylation pattern. It is commonly known that typical glycosylated residue position in an antibody is the asparagine at position 297 according to the EU numbering system ("Asn297").

The "EU numbering system" or "EU index" is generally used when referring to a residue in an immunoglobulin heavy chain constant region (e.g., the EU index reported in Kabat et al., Sequences of Proteins of Immunological Interest, 5th ed., Public Health Service, National Institutes of Health, Bethesda, MD (1991)).

Thus an afucosylated antibody according to the invention means an antibody of IgG1 or IgG3 isotype (preferably of IgG1 isotype) wherein the amount of fucose is 60% or less of the total amount of oligosaccharides (sugars) at Asn297 (which means that at least 40% or more of the oligosaccharides of the Fc region at Asn297 are afucosylated). In one aspect the amount of fucose is between 40% and 60% of the oligosaccharides of the Fc region at Asn297. In another aspect the amount of fucose is 50% or less, and in still another aspect the amount of fucose is 30% or less of the oligosaccharides of the Fc region at Asn297. According to the invention "amount of fucose" means the amount of said oligosaccharide (fucose) within the oligosaccharide (sugar) chain at Asn297, related to the sum of all oligosaccharides (sugars) attached to Asn 297 (e. g. complex, hybrid and high mannose structures) measured by MALDI-TOF mass spectrometry and calculated as average value (for a detailed procedure to determine the amount of fucose, see e.g. WO 2008/077546). Furthermore in one aspect, the oligosaccharides of the Fc region are bisected. The afucosylated antibody according to the invention can be expressed in a glycomodified host cell engineered to express at least one nucleic acid encoding a polypeptide having GnTIII activity in an amount sufficient to partially fucosylate the oligosaccharides in the Fc region. In one aspect, the polypeptide having GnTIII activity is a fusion polypeptide. Alternatively α1,6-fucosyltransferase activity of the host cell can be decreased or eliminated according to US 6,946,292 to generate glycomodified host cells. The amount of antibody fucosylation can be predetermined e.g. either by fermentation conditions (e.g. fermentation time) or by combination of at least two antibodies with different fucosylation amount. Such afucosylated antibodies and respective glycoengineering methods are described in WO 2005/044859, WO 2004/065540, WO 2007/031875, Umana, P., et al., Nature Biotechnol. 17 (1999) 176-180, WO 99/154342, WO 2005/018572, WO 2006/116260, WO 2006/114700, WO 2005/011735, WO 2005/027966, WO 97/028267, US 2006/0134709, US 2005/0054048, US 2005/0152894, WO 2003/035835, WO 2000/061739. These glycoengineered antibodies have an increased ADCC. Other glycoengineering methods yielding afucosylated antibodies according to the invention are described e.g. in Niwa, R.. et al., J. Immunol. Methods 306 (2005) 151-160; Shinkawa, T., et al., J. Biol. Chem, 278 (2003) 3466-3473; WO 03/055993 or US 2005/0249722.

Thus one aspect of the invention is an afucosylated anti-CD20 antibody of IgG1 or IgG3 isotype (preferably of IgG1 isotype) specifically binding to CD20 with an amount of fucose of 60% or less of the total amount of oligosaccharides (sugars) at Asn297, for the treatment of cancer in combination with a CD22 antibody-drug conjugate. In another aspect of the invention is the use of an afucosylated anti-CD20 antibody of IgG1 or IgG3 isotype (preferably of IgG1 isotype) specifically binding to CD20 with an amount of fucose of 60% or less of the total amount of oligosaccharides (sugars) at Asn297, for the manufacture of a medicament for the treatment of cancer in combination with a CD22 antibody-drug conjugate. In one aspect the amount of fucose is between 60% and 20% of the total amount of oligosaccharides (sugars) at Asn297. In one aspect the amount of fucose is between 60% and 40% of the total amount of oligosaccharides (sugars) at Asn297. In one aspect the amount of fucose is between 0% of the total amount of oligosaccharides (sugars) at Asn297.

CD20 ( also known as B-lymphocyte antigen CD20, B-lymphocyte surface antigen B1, Leu-16, Bp35, BM5, and LF5; the sequence is characterized by the SwissProt database entry P11836) is is a hydrophobic transmembrane protein with a molecular weight of approximately 35 kD located on pre-B and mature B lymphocytes (Valentine, M.A. et al., J. Biol. Chem. 264 (1989) 11282-11287; Tedder, T.F., et al., Proc. Natl. Acad. Sci. U.S.A. 85 (1988) 208-212; Stamenkovic, I., et al., J. Exp. Med. 167 (1988) 1975-1980; Einfeld, D.A., et al., EMBO J. 7 (1988) 711-717; Tedder, T.F., et al., J. Immunol. 142 (1989) 2560-2568). The corresponding human gene is Membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 11q12, among a cluster of family members. Alternative splicing of this gene results in two transcript variants which encode the same protein.

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms: Cell death/apoptosis induction, ADCC and CDC.

Synonyms of CD20, as recognized in the art, include B-lymphocyte antigen CD20, B-lymphocyte surface antigen B1, Leu-16, Bp35, BM5, and LF5.

The term "anti-CD20 antibody" according to the invention is an antibody that binds specifically to CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al., Blood 103 (2004) 2738-2743; and Cragg, M.S., et al., Blood 101 (2003) 1045-1052, see Table 1.

**Table 1: Properties of type I and type II anti-CD20 antibodies**

| **type I anti-CD20 antibodies** | **type II anti-CD20 antibodies** |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

Examples of type II anti-CD20 antibodies include e.g. humanized B-Ly1 antibody IgG1 (a chimeric humanized IgG1 antibody as disclosed in WO 2005/044859), 11B8 IgG1 (as disclosed in WO 2004/035607), and AT80 IgG1. Typically type II anti-CD20 antibodies of the IgG1 isotype show characteristic CDC properties. Type II anti-CD20 antibodies have a decreased CDC (if IgG1 isotype) compared to type I antibodies of the IgG1 isotype.

Examples of type I anti-CD20 antibodies include e.g. rituximab, HI47 IgG3 (ECACC, hybridoma), 2C6 IgG1 (as disclosed in WO 2005/103081), 2F2 IgG1 (as disclosed and WO 2004/035607 and WO 2005/103081) and 2H7 IgG1 (as disclosed in WO 2004/056312).

The afucosylated anti-CD20 antibodies according to the invention is in one aspect a type II anti-CD20 antibody, in another aspect an afucosylated humanized B-Ly1 antibody.

The afucosylated anti-CD20 antibodies according to the invention have an increased antibody dependent cellular cytotoxicity (ADCC) unlike anti-CD20 antibodies having no reduced fucose.

By "afucosylated anti-CD20 antibody with increased antibody dependent cellular cytotoxicity (ADCC)" is meant an afucosylated anti-CD20 antibody, as that term is defined herein, having increased ADCC as determined by any suitable method known to those of ordinary skill in the art. One accepted in vitro ADCC assay is as follows:
1) the assay uses target cells that are known to express the target antigen recognized by the antigen-binding region of the antibody;
2) the assay uses human peripheral blood mononuclear cells (PBMCs), isolated from blood of a randomly chosen healthy donor, as effector cells;
3) the assay is carried out according to following protocol:
   i) the PBMCs are isolated using standard density centrifugation procedures and are suspended at 5 x 10⁶ cells/ml in RPMI cell culture medium;
   ii) the target cells are grown by standard tissue culture methods, harvested from the exponential growth phase with a viability higher than 90%, washed in RPMI cell culture medium, labeled with 100 micro-Curies of ⁵¹Cr, washed twice with cell culture medium, and resuspended in cell culture medium at a density of 10⁵ cells/ml;
   iii) 100 microliters of the final target cell suspension above are transferred to each well of a 96-well microtiter plate;
   iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;
   v) for the maximum release (MR) controls, 3 additional wells in the plate containing the labeled target cells, receive 50 microliters of a 2% (VN) aqueous solution of non-ionic detergent (Nonidet, Sigma, St. Louis), instead of the antibody solution (point iv above);
   vi) for the spontaneous release (SR) controls, 3 additional wells in the plate containing the labeled target cells, receive 50 microliters of RPMI cell culture medium instead of the antibody solution (point iv above);
   vii) the 96-well microtiter plate is then centrifuged at 50 x g for 1 minute and incubated for 1 hour at 4°C;
   viii) 50 microliters of the PBMC suspension (point i above) are added to each well to yield an effector:target cell ratio of 25: 1 and the plates are placed in an incubator under 5% CO2 atmosphere at 37 C for 4 hours;
   ix) the cell-free supernatant from each well is harvested and the experimentally released radioactivity (ER) is quantified using a gamma counter;
   x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point V above), and SR is the average radioactivity quantified (see point ix above) for the SR controls (see point vi above);
4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by the same type of host cells, using the same standard production, purification, formulation and storage methods, which are known to those skilled in the art, but that has not been produced by host cells engineered to overexpress GnTIII.

Said "increased ADCC" can be obtained by glycoengineering of said antibodies, that means enhance said natural, cell-mediated effector functions of monoclonal antibodies by engineering their oligosaccharide component as described in Umana, P., et al., Nature Biotechnol. 17 (1999) 176-180 and US 6,602,684.

The term "complement-dependent cytotoxicity (CDC)" refers to lysis of human tumor target cells by the antibody according to the invention in the presence of complement. CDC is measured preferably by the treatment of a preparation of CD20 expressing cells with an anti-CD20 antibody according to the invention in the presence of complement. CDC is found if the antibody induces at a concentration of 100 nM the lysis (cell death) of 20% or more of the tumor cells after 4 hours. The assay is performed preferably with ⁵¹Cr or Eu labeled tumor cells and measurement of released ⁵¹Cr or Eu. Controls include the incubation of the tumor target cells with complement but without the antibody.

The "rituximab" antibody (reference antibody; example of a type I anti-CD20 antibody) is a genetically engineered chimeric human gamma 1 murine constant domain containing monoclonal antibody directed against the human CD20 antigen. This chimeric antibody contains human gamma 1 constant domains and is identified by the name "C2B8" in US 5,736,137 (Anderson et. al.) issued on April 17, 1998, assigned to IDEC Pharmaceuticals Corporation. Rituximab is approved for the treatment of patients with relapsed or refracting low-grade or follicular, CD20 positive, B cell non-Hodgkin's lymphoma. In vitro mechanism of action studies have shown that rituximab exhibits human complement--dependent cytotoxicity (CDC) (Reff, M.E., et. al., Blood 83 (1994) 435-445). Additionally, it exhibits significant activity in assays that measure antibody-dependent cellular cytotoxicity (ADCC). Rituximab is not afucosylated.

**Table 2**

| Antibody | Amount of fucose |
|---|---|
| Rituximab (non-afucosylated) | >85 % |
| Wild type afucosylated glyco-engineered humanized B-Ly1 (B-HH6-B-KV1) (non-afucosylated) | >85 % |
| afucosylated glyco-engineered humanized B-Ly1 (B-HH6-B-KV1 GE) | 45-50 % |

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO: 28; variable region of the murine light chain (VL): SEQ ID NO: 29 (see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

In one aspect, the "humanized B-Ly1 antibody" has variable region of the heavy chain (VH) selected from group of SEQ ID NO: 30 to SEQ ID NO: 46 (B-HH2 to B-HH9 and B-HL8 to B-HL17 of WO 2005/044859 and WO 2007/031875). In one specific aspect, such variable domain is selected from the group consisting of SEQ ID NOs: 30, 31, 34, 36, 38, 40 and 42 (B-HH2, BHH-3, B-HH6, B-HH8, B-HL8, B-HL11 and B-HL13 of WO 2005/044859 and WO 2007/031875). In one specific aspect, the "humanized B-Ly1 antibody" has variable region of the light chain (VL) of SEQ ID NO: 47 (B-KV1 of WO 2005/044859 and WO 2007/031875). In one specific aspect, the "humanized B-Ly1 antibody" has a variable region of the heavy chain (VH) of SEQ ID NO: 34 (B-HH6 of WO 2005/044859 and WO 2007/031875) and a variable region of the light chain (VL) of SEQ ID NO: 47 (B-KV1 of WO 2005/044859 and WO 2007/031875). Furthermore in one aspect, the humanized B-Ly1 antibody is an IgG1 antibody. According to the invention such afocusylated humanized B-Ly1 antibodies are glycoengineered (GE) in the Fc region according to the procedures described in WO 2005/044859, WO 2004/065540, WO 2007/031875, Umana, P. et al., Nature Biotechnol. 17 (1999) 176-180 and WO 99/154342. In one aspect, the afucosylated glyco-engineered humanized B-Ly1 is B-HH6-B-KV1 GE. In one embodiment, the anti-CD20 antibody is obinutuzumab (recommended INN, WHO Drug Information, Vol. 26, No. 4, 2012, p. 453). As used herein, obinutuzumab is synonymous for GA101. This replaces all previous versions (e.g. Vol. 25, No. 1, 2011, p.75-76), and is formerly known as afutuzumab (recommended INN, WHO Drug Information, Vol. 23, No. 2, 2009, p. 176;Vol. 22, No. 2, 2008, p. 124).

Such glycoengineered humanized B-Ly1 antibodies have an altered pattern of glycosylation in the Fc region, preferably having a reduced level of fucose residues. In one aspect, the amount of fucose is 60% or less of the total amount of oligosaccharides at Asn297 (in one aspect the amount of fucose is between 40% and 60%, in another aspect the amount of fucose is 50% or less, and in still another aspect the amount of fucose is 30% or less). In another aspect, the oligosaccharides of the Fc region are preferably bisected. These glycoengineered humanized B-Ly1 antibodies have an increased ADCC.

"CD22" as used herein is a 135-kDa B-cell-restricted sialoglycoprotein expressed on the B-cell surface only at the mature stages of differentiation (Dorken, B. et al., J. Immunol. 136:4470-4479 (1986)). The predominant form of CD22 in humans is CD22beta which contains seven immunoglobulin superfamily domains in the extracellular domain) (Wilson, G.L. et al., J. Exp. Med. 173:137-146 (1991)). A variant form, CD22 alpha, lacks immunoglobulin superfamily domains 3 and 4 (Stamenkovic, I. and Seed, B., Nature 345:74-77 (1990)). Ligand-binding to human CD22 has been shown to be associated with immunoglobulin superfamily domains 1 and 2 (also referred to as epitopes 1 and 2) (Engel, P. et al., J. Exp. Med. 181:1581-1586, 1995).

The term "anti-CD22 antibody" refers to an antibody which inhibits CD22.

In one aspect, an antibody that binds to CD22 in said antibody-drug conjugate according to the invention is provided, wherein the antibody comprises at least one, two, three, four, five, or six HVRs selected from:
(1) an HVR-H1 comprising the amino acid sequence of SEQ ID NO:2;
(2) an HVR-H2 comprising the amino acid sequence of SEQ ID NO:4;
(3) an HVR-H3 comprising the amino acid sequence of SEQ ID NO:6;
(4) an HVR-L1 comprising the amino acid sequence of SEQ ID NO:10;
(5) an HVR-L2 comprising the amino acid sequence of SEQ ID NO:12; and
(6) an HVR-L3 comprising the amino acid sequence of SEQ ID NO:14.

In another aspect, an antibody that binds to CD22 in said antibody-drug conjugate according to the invention comprises (a) an HVR-L1 comprising the amino acid sequence of SEQ ID NO:10, and (b) at least one, two, three, four or five HVRs selected from:
(1) an HVR-H1 comprising the amino acid sequence of SEQ ID NO:2;
(2) an HVR-H2 comprising the amino acid sequence of SEQ ID NO:4;
(3) an HVR-H3 comprising the amino acid sequence of SEQ ID NO:6;
(4) an HVR-L2 comprising the amino acid sequence of SEQ ID NO:12; and
(6) an HVR-L3 comprising the amino acid sequence of SEQ ID NO:14.

In another aspect, an antibody that binds to CD22 in said antibody-drug conjugate according to the invention comprises (a) an HVR-L1 comprising the amino acid sequence of SEQ ID NO:9, and (b) at least one, two, three, four or five HVRs selected from:
(1) an HVR-H1 comprising the amino acid sequence of SEQ ID NO:2;
(2) an HVR-H2 comprising the amino acid sequence of SEQ ID NO:4;
(3) an HVR-H3 comprising the amino acid sequence of SEQ ID NO:6;
(4) an HVR-L2 comprising the amino acid sequence of SEQ ID NO:12; and
(6) an HVR-L3 comprising the amino acid sequence of SEQ ID NO:14.

In another aspect, an antibody that binds to CD22 in said antibody-drug conjugate according to the invention comprises (a) an HVR-H3 comprising the amino acid sequence of SEQ ID NO:6, and (b) at least one, two, three, four, or five HVRs selected from:
(1) an HVR-H1 comprising the amino acid sequence of SEQ ID NO:2;
(2) an HVR-H2 comprising the amino acid sequence of SEQ ID NO:4;
(3) an HVR-L1 comprising the amino acid sequence of SEQ ID NO:9;
(4) an HVR-L2 comprising the amino acid sequence of SEQ ID NO:12; and
(5) an HVR-L3 comprising the amino acid sequence of SEQ ID NO:14.

In another aspect, an antibody that binds to CD22 in said antibody-drug conjugate according to the invention comprises (a) an HVR-H3 comprising the amino acid sequence of SEQ ID NO:6, and (b) at least one, two, three, four, or five HVRs selected from:
(1) an HVR-H1 comprising the amino acid sequence of SEQ ID NO:2;
(2) an HVR-H2 comprising the amino acid sequence of SEQ ID NO:4;
(3) an HVR-L1 comprising the amino acid sequence of SEQ ID NO:10;
(4) an HVR-L2 comprising the amino acid sequence of SEQ ID NO:12; and
(5) an HVR-L3 comprising the amino acid sequence of SEQ ID NO:14.

In one aspect, the antibody comprises an HVR-L1 comprising the amino acid sequence of SEQ ID NO:10. In one aspect, the antibody further comprises an HVR-H1 comprising the amino acid sequence of SEQ ID NO:2 and an HVR-H2 comprising the amino acid sequence of SEQ ID NO:4. In one aspect, the antibody further comprises an HVR-L2 comprising the amino acid sequence of SEQ NO:12 and an HVR-L3 comprising the amino acid sequence of SEQ ID NO:14.

In certain aspects, any of the above antibodies further comprises at least one framework selected from a VH subgroup III consensus framework and a VL subgroup I consensus framework.

In one aspect, an antibody that binds to CD22 in said antibody-drug conjugate according to the invention is provided, wherein the antibody comprises a heavy chain variable domain having at least 90%, at least 91%, at least 92%, at least 93%, at least 94%, at least 95%, at least 96%, at least 97%, at least 98%, or at least 99% sequence identity to an amino acid sequence of SEQ ID NO:16. In one aspect, the antibody comprises a heavy chain variable domain of SEQ ID NO:16.

In one aspect, the antibody further comprises a light chain variable domain having at least 90%, at least 91%, at least 92%, at least 93%, at least 94%, at least 95%, at least 96%, at least 97%, at least 98%, or at least 99% sequence identity to an amino acid sequence of SEQ ID NO:17. In one aspect, the antibody comprises a light chain variable domain of SEQ ID NO:17.

In one aspect, the antibody further comprises a light chain variable domain having at least 90%, at least 91%, at least 92%, at least 93%, at least 94%, at least 95%, at least 96%, at least 97%, at least 98%, or at least 99% sequence identity to an amino acid sequence of SEQ ID NO:18. In one aspect, the antibody comprises a light chain variable domain of SEQ ID NO:18.

In one aspect, the antibody comprises a heavy chain variable domain having at least 90%, at least 91%, at least 92%, at least 93%, at least 94%, at least 95%, at least 96%, at least 97%, at least 98%, at least 99%, or 100% sequence identity to an amino acid sequence of SEQ ID NO:16 and a light chain variable domain having at least 90%, at least 91%, at least 92%, at least 93%, at least 94%, at least 95%, at least 96%, at least 97%, at least 98%, at least 99%, or 100% sequence identity to an amino acid sequence of SEQ ID NO:17. In one aspect, the antibody comprises a heavy chain variable domain having at least 90%, at least 91%, at least 92%, at least 93%, at least 94%, at least 95%, at least 96%, at least 97%, at least 98%, at least 99%, or 100% sequence identity to an amino acid sequence of SEQ ID NO:16 and a light chain variable domain having at least 90%, at least 91%, at least 92%, at least 93%, at least 94%, at least 95%, at least 96%, at least 97%, at least 98%, at least 99%, or 100% sequence identity to an amino acid sequence of SEQ ID NO:18. In one aspect, the heavy chain variable domain comprises the amino acid sequence of SEQ ID NO:16, and the light chain variable domain comprises the amino acid sequence of SEQ ID NO:17. In one aspect, the heavy chain variable domain comprises the amino acid sequence of SEQ ID NO:16, and the light chain variable domain comprises the amino acid sequence of SEQ ID NO:18.

In one aspect, the invention provides an anti-CD22 antibody comprising (a) one, two, or three VH HVRs selected from those shown in Figure 3A and/or (b) one, two, or three VL HVRs selected from those shown in Figure 3B. In one aspect, the invention provides an anti-CD22 antibody comprising a heavy chain variable domain selected from those shown in Figure 3A and a light chain variable domain selected from those shown in Figure 3B.

In certain aspects, a polynucleotide encoding any of the above antibodies is provided. In one aspect, a vector comprising the polynucleotide is provided. In one aspect, a host cell comprising the vector is provided. In one aspect, the host cell is eukaryotic. In one aspect, the host cell is a Chinese hamster ovary (CHO) cell. In one aspect, a method of making an anti-CD22 antibody is provided, wherein the method comprises culturing the host cell under conditions suitable for expression of the polynucleotide encoding the antibody, and isolating the antibody.

In one aspect, an antibody that binds to CD22 expressed on the surface of a cell in said antibody-drug conjugate according to the invention is provided. In one aspect, the antibody binds to an epitope within a region of human or mouse CD22 comprising domain 1 or domain 2 or domains 1 and 2. In one aspect, the cell is mammalian cell. In one aspect, the cell is a human cell. In one aspect, the cell is a cancer cell. In one aspect the cell is a B cell. In one embodiment the cancer cell is a B cell.

In certain aspects, any of the above antibodies is a monoclonal antibody. In one aspect, the antibody is an antibody fragment selected from a Fab, Fab'-SH, Fv, scFv, or (Fab')2 fragment. In one aspect, the antibody is humanized. In one aspect, the antibody is human.

In one aspect, the antibodies of the invention include cysteine engineered antibodies where one or more amino acids of a parent antibody are replaced with a free cysteine amino acid as disclosed in WO2006/034488. Any form of anti-CD22 antibody may be so engineered, i.e. mutated. For example, a parent Fab antibody fragment may be engineered to form a cysteine engineered Fab, referred to herein as "ThioFab." Similarly, a parent monoclonal antibody may be engineered to form a "ThioMab." It should be noted that a single site mutation yields a single engineered cysteine residue in a ThioFab, while a single site mutation yields two engineered cysteine residues in a ThioMab, due to the dimeric nature of the IgG antibody. The cysteine engineered anti-CD22 antibodies of the invention include monoclonal antibodies, humanized or chimeric monoclonal antibodies, and antigen-binding fragments of antibodies, fusion polypeptides and analogs that preferentially bind cell-associated CD22 polypeptides. A cysteine engineered antibody may alternatively comprise an antibody comprising a cysteine at a position disclosed herein in the antibody or Fab, resulting from the sequence design and/or selection of the antibody, without necessarily altering a parent antibody, such as by phage display antibody design and selection or through de novo design of light chain and/or heavy chain framework sequences and constant regions. A cysteine engineered antibody comprises one or more free cysteine amino acids having a thiol reactivity value in the ranges of 0.6 to 1.0; 0.7 to 1.0 or 0.8 to 1.0. A free cysteine amino acid is a cysteine residue which has been engineered into the parent antibody and is not part of a disulfide bridge. Cysteine engineered antibodies are useful for attachment of cytotoxic and/or imaging compounds at the site of the engineered cysteine through, for example, a maleimide or haloacetyl. The nucleophilic reactivity of the thiol functionality of a Cys residue to a maleimide group is about 1000 times higher compared to any other amino acid functionality in a protein, such as amino group of lysine residues or the N-terminal amino group. Thiol specific functionality in iodoacetyl and maleimide reagents may react with amine groups, but higher pH (>9.0) and longer reaction times are required (Garman, 1997, Non-Radioactive Labelling: A Practical Approach, Academic Press, London).

In an aspect, a cysteine engineered anti-CD22 antibody in said antibody-drug conjugate according to the invention comprises an engineered cysteine at any one of the following positions, where the position is number according to Kabat et al. in the light chain (see Kabat et al (1991) Sequences of Proteins of Immunological Interest, 5th Ed. Public Health Service, National Institutes of Health, Bethesda, MD) and according to EU numbering in the heavy chain (including the Fc region) (see Kabat et al. (1991), supra), wherein the light chain constant region begins at position 108 (Kabat numbering) and the heavy chain constant region begins at position 118 (EU numbering). The position may also be referred to by its position in sequential numbering of the amino acids of the full length light chain or heavy chain. According to one aspect of the invention, an anti-CD22 antibody in said antibody-drug conjugate according to the invention comprises an engineered cysteine at LC-V205C (Kabat number: Val 205; sequential number 210 engineered to be Cys at that position). According to one aspect, an anti-CD22 antibody comprises an engineered cysteine at HC-A118C (EU number: Ala 118; sequential number 121 engineered to be Cys at that position). According to one aspect, an anti-CD22 antibody comprises an engineered cysteine at Fc-S400C (EU number: Ser 400; sequential number 403 engineered to be Cys at that position). In other aspects, the engineered cysteine of the heavy chain (including the Fc region) is at any one of the following positions (according to EU numbering): 41, 88, 116, 118, 120, 171, 282, 375, or 400. Thus, changes in the amino acid at these positions for a parent anti-CD22 antibody of the invention are: A41C, A88C, S116C, A118C, T120C, A171C, V282C, S375C, or S400C. In other aspects, the engineered cysteine of the light chain is at any one of the following positions (according to Kabat numbering): 15, 43, 110, 144, 168, 205. Thus, changes in the amino acid at these positions for a parent anti-CD22 antibody of the invention are: V15C, A43C, V110C, A144C, S168C, or V205C.

A cysteine engineered anti-CD22 antibody in said antibody-drug conjugate according to the invention comprises one or more free cysteine amino acids wherein the cysteine engineered anti-CD22 antibody binds to a CD22 polypeptide and is prepared by a process comprising replacing one or more amino acid residues of a parent anti-CD22 antibody by cysteine wherein the parent antibody comprises at least one HVR sequence selected from:
(a) an HVR-L1 sequence RSSQSIVHSNGNTFLE (SEQ ID NO:9) or sequence RSSQSIVHSVGNTFLE (SEQ ID NO:10) (Figure 3B);
(b) an HVR-L2 sequence KVSNRFS SEQ ID NO:12 (Figure 3B);
(c) an HVR-L3 sequence FQGSQFPYT (SEQ ID NO:14) (Figure 3B);
(d) an HVR-H1 sequence GYEFSRSWMN (SEQ ID NO:2) (Figure 3A);
(e) an HVR-H2 sequence GRIYPGDGDTNYSGKFKG (SEQ ID NO:4 (Figure 3A); and
(f) an HVR-H3 sequence DGSSWDWYFDV (SEQ ID NO:6) (Figure 3A).

In a certain aspect, the invention concerns a cysteine engineered anti-CD22 antibody in said antibody-drug conjugate according to the invention, comprising an amino acid sequence having at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, 99% or 100% amino acid sequence identity, to a cysteine engineered antibody having a full-length amino acid sequence as disclosed herein, or a cysteine engineered antibody amino acid sequence lacking the signal peptide as disclosed herein.

In a yet further aspect, the invention concerns an isolated cysteine engineered anti-CD22 antibody in said antibody-drug conjugate according to the invention comprising an amino acid sequence that is encoded by a nucleotide sequence that hybridizes to the complement of a DNA molecule encoding (a) a cysteine engineered antibody having a full-length amino acid sequence as disclosed herein, (b) a cysteine engineered antibody amino acid sequence lacking the signal peptide as disclosed herein, (c) an extracellular domain of a transmembrane cysteine engineered antibody protein, with or without the signal peptide, as disclosed herein, (d) an amino acid sequence encoded by any of the nucleic acid sequences disclosed herein or (e) any other specifically defined fragment of a full-length cysteine engineered antibody amino acid sequence as disclosed herein.

In a specific aspect, the invention provides an isolated cysteine engineered anti-CD22 antibody in said antibody-drug conjugate according to the invention without the N-terminal signal sequence and/or without the initiating methionine and is encoded by a nucleotide sequence that encodes such an amino acid sequence as described in. Processes for producing the same are also herein described, wherein those processes comprise culturing a host cell comprising a vector which comprises the appropriate encoding nucleic acid molecule under conditions suitable for expression of the cysteine engineered antibody and recovering the cysteine engineered antibody from the cell culture.

Another aspect of the invention provides an isolated cysteine engineered anti-CD22 antibody in said antibody-drug conjugate according to the invention which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the cysteine engineered antibody and recovering the cysteine engineered antibody from the cell culture.

In other aspects, the invention provides isolated anti-CD22 chimeric cysteine engineered antibodies in said antibody-drug conjugate according to the invention comprising any of the herein described cysteine engineered antibody fused to a heterologous (non-CD22) polypeptide. Example of such chimeric molecules comprise any of the herein described cysteine engineered antibodies fused to a heterologous polypeptide such as, for example, an epitope tag sequence or a Fc region of an immunoglobulin.

The cysteine engineered anti-CD22 antibody in said antibody-drug conjugate according to the invention may be a monoclonal antibody, antibody fragment, chimeric antibody, humanized antibody, single-chain antibody or antibody that competitively inhibits the binding of an anti-CD22 polypeptide antibody to its respective antigenic epitope. Antibodies of the present invention may optionally be conjugated to a growth inhibitory agent or cytotoxic agent such as a toxin, including, for example, an auristatin, an antibiotic, a radioactive isotope, a nucleolytic enzyme, or the like. The antibodies of the present invention may optionally be produced in CHO cells or bacterial cells and preferably inhibit the growth or proliferation of or induce the death of a cell to which they bind. For diagnostic purposes, the antibodies of the present invention may be detectably labeled, attached to a solid support, or the like.

In other aspects of the present invention, the invention provides vectors comprising DNA encoding any of the herein described anti-CD22 antibodies in said antibody-drug conjugate according to the invention and anti-CD22 cysteine engineered antibodies in said antibody-drug conjugate according to the invention. Host cells comprising any such vector are also provided. By way of example, the host cells may be CHO cells, E. coli cells, or yeast cells. A process for producing any of the herein described polypeptides is further provided and comprises culturing host cells under conditions suitable for expression of the desired polypeptide and recovering the desired polypeptide from the cell culture.

Cysteine engineered antibodies may be useful in the treatment of cancer and include antibodies specific for cell surface and transmembrane receptors, and tumor-associated antigens (TAA). Such antibodies may be used as naked antibodies (unconjugated to a drug or label moiety) or as antibody-drug conjugates (ADC). Cysteine engineered antibodies of the invention may be site-specifically and efficiently coupled with a thiol-reactive reagent. The thiol-reactive reagent may be a multifunctional linker reagent, a capture label reagent, a fluorophore reagent, or a drug-linker intermediate. The cysteine engineered antibody may be labeled with a detectable label, immobilized on a solid phase support and/or conjugated with a drug moiety. Thiol reactivity may be generalized to any antibody where substitution of amino acids with reactive cysteine amino acids may be made within the ranges in the light chain selected from amino acid ranges: L-10 to L-20; L-38 to L-48; L-105 to L-115; L-139 to L-149; L-163 to L-173; and within the ranges in the heavy chain selected from amino acid ranges: H-35 to H-45; H-83 to H-93; H-114 to H-127; and H-170 to H-184, and in the Fc region within the ranges selected from H-268 to H-291; H-319 to H-344; H-370 to H-380; and H-395 to H-405, where the numbering of amino acid positions begins at position 1 of the Kabat numbering system (Kabat et al. (1991) Sequences of Proteins of Immunological Interest, 5th Ed. Public Health Service, National Institutes of Health, Bethesda, MD) and continues sequentially thereafter as disclosed in WO2006034488. Thiol reactivity may also be generalized to certain domains of an antibody, such as the light chain constant domain (CL) and heavy chain constant domains, CH1, CH2 and CH3. Cysteine replacements resulting in thiol reactivity values of 0.6 and higher may be made in the heavy chain constant domains α, δ, ε, γ, and µ of intact antibodies: IgA, IgD, IgE, IgG, and IgM, respectively, including the IgG subclasses: IgG1, IgG2, IgG3, IgG4, IgA, and IgA2. Such antibodies and their uses are disclosed in WO2006/034488.

Cysteine engineered antibodies of the invention preferably retain the antigen binding capability of their wild type, parent antibody counterparts. Thus, cysteine engineered antibodies are capable of binding, preferably specifically, to antigens. Such antigens include, for example, tumor-associated antigens (TAA), cell surface receptor proteins and other cell surface molecules, transmembrane proteins, signaling proteins, cell survival regulatory factors, cell proliferation regulatory factors, molecules associated with (for e.g., known or suspected to contribute functionally to) tissue development or differentiation, lymphokines, cytokines, molecules involved in cell cycle regulation, molecules involved in vasculogenesis and molecules associated with (for e.g., known or suspected to contribute functionally to) angiogenesis. The tumor-associated antigen may be a cluster differentiation factor (i.e., a CD protein, including but not limited to CD22). Cysteine engineered anti-CD22 antibodies of the invention retain the antigen binding apability of their parent anti-CD22 antibody. Thus, cysteine engineered anti-CD22 antibodies of the invention are capable of binding, preferably specifically, to CD22 antigens including human anti-CD22 isoforms beta and/or alpha, including when such antigens are expressed on the surface of cells, including, without limitation, B cells.

A detailed description of exemplary anti-CD22 antibodies as part of the antibody-drug conjugate in the inventive combination with an anti-CD20 antibody as defined herein is as follows:

### Specific aspects of anti-CD22 antibodies

In one aspect, the invention provides a combination of an anti-CD20 antibody as defined herein with an antibody in said antibody-drug conjugate according to the invention comprising at least one, two, three, four, five, or six HVRs selected from (a) an HVR-H1 comprising the amino acid sequence of SEQ ID NO:2; (b) an HVR-H2 comprising the amino acid sequence of SEQ ID NO:4; (c) an HVR-H3 comprising an amino acid sequence selected from SEQ ID NO:6; (d) an HVR-L1 comprising the amino acid sequence of any one of SEQ ID NO:9, 10, 19, 20, 21, 22, 23; (e) an HVR-L2 comprising the amino acid sequence of SEQ ID NO:12; and (f) an HVR-L3 comprising an amino acid sequence selected from SEQ ID NO:14.

In one aspect, the invention provides a combination of an anti-CD20 antibody as defined herein with an anti-CD22 antibody in said antibody-drug conjugate according to the invention comprising at least one, at least two, or all three VH HVR sequences selected from (a) an HVR-H1 comprising the amino acid sequence of SEQ ID NO:2; (b) an HVR-H2 comprising the amino acid sequence of SEQ ID NO:4; (c) an HVR-H3 comprising an amino acid sequence selected from SEQ ID NO:6. In one aspect, the invention provides an anti-CD22 antibody in said antibody-drug conjugate according to the invention comprising an HVR-H1 comprising the amino acid sequence of SEQ ID NO:2. In one aspect, the invention provides an anti-CD22 antibody comprising an HVR-H2 comprising the amino acid sequence of SEQ ID NO:4. In one aspect, the invention provides an anti-CD22 antibody comprising an HVR-H3 comprising an amino acid sequence selected from SEQ ID NO:6.

In one aspect, the invention provides a combination of an anti-CD20 antibody as defined herein with an anti-CD22 antibody in said antibody-drug conjugate according to the invention comprising an HVR-H3 comprising an amino acid sequence selected from SEQ ID NO:6 and an HVR-H1 comprising an amino acid sequence selected from SEQ ID NO:2.

In one aspect, the invention provides a combination of an anti-CD20 antibody as defined herein with an anti-CD22 antibody in said antibody-drug conjugate according to the invention comprising an HVR-H3 comprising an amino acid sequence selected from SEQ ID NO:6 and an HVR-H2 comprising an amino acid sequence selected from SEQ ID NO:4.

In one aspect, the invention provides a combination of an anti-CD20 antibody as defined herein with an anti-CD22 antibody comprising an HVR-H1 comprising the amino acid sequence of SEQ ID NO:2 and an HVR-H2 comprising the amino acid sequence of SEQ ID NO:4.

In one aspect, the invention provides a combination of an anti-CD20 antibody as defined herein with an anti-CD22 antibody in said antibody-drug conjugate according to the invention comprising an HVR-H1 comprising the amino acid sequence of SEQ ID NO:2; an HVR-H2 comprising the amino acid sequence of SEQ ID NO:4; and an HVR-H3 comprising the amino acid sequence of SEQ ID NO:6.

In one aspect, the invention provides a combination of an anti-CD20 antibody as defined herein with an anti-CD22 antibody in said antibody-drug conjugate according to the invention comprising at least one, at least two, or all three VL HVR sequences selected from (a) an HVR-L1 comprising the amino acid sequence of SEQ ID NO:9 or SEQ ID NO:10; (b) an HVR-L2 comprising the amino acid sequence of SEQ ID NO:12; and (c) an HVR-L3 comprising an amino acid sequence selected from SEQ ID NO:14. In one aspect, the invention provides a combination of an anti-CD20 antibody as defined herein with an anti-CD22 antibody comprising an HVR-L1 comprising an amino acid sequence selected from SEQ ID NO:9. In one aspect, the invention provides a combination of an anti-CD20 antibody as defined herein with an anti-CD22 antibody comprising an HVR-L1 comprising an amino acid sequence selected from SEQ ID NO:10. In one aspect, the invention provides a combination of an anti-CD20 antibody as defined herein with an anti-CD22 antibody in said antibody-drug conjugate according to the invention comprising an HVR-L1 comprising an amino acid sequence selected from SEQ ID NO:19-23. In one aspect, the HVR-L1 comprises the amino acid sequence of SEQ ID NO:9 wherein N28 is replaced by V (an N28V amino acid change, which generates SEQ ID NO:10). In one aspect, the HVR-L1 comprises the amino acid sequence of SEQ ID NO:9 wherein N28 is replaced by A (an N28A amino acid change, which generates SEQ ID NO:19). In one aspect, the HVR-L1 comprises the amino acid sequence of SEQ ID NO:9 wherein N28 is replaced by Q (an N28Q amino acid change, which generates SEQ ID NO:20). In one aspect, the HVR-L1 comprises the amino acid sequence of SEQ ID NO:9 wherein N28 is replaced by S (an N28S amino acid change, which generates SEQ ID NO:21). In one aspect, the HVR-L1 comprises the amino acid sequence of SEQ ID NO:9 wherein N28 is replaced by D (an N28D amino acid change, which generates SEQ ID NO:22). In one aspect, the HVR-L1 comprises the amino acid sequence of SEQ ID NO:9 wherein N28 is replaced by I (an N28I amino acid change, which generates SEQ ID NO:23). In one aspect, the invention provides a combination of an anti-CD20 antibody as defined herein with an anti-CD22 antibody in said antibody-drug conjugate according to the invention comprising an HVR-L1 comprising the amino acid sequence of any one of SEQ ID NO:9, 10, 19, 20, 21, 22, 23. In one aspect, the HVR-L1 is any one of SEQ ID NO:9, 10, 19, 20, 21, 22, or 23 and the amino acid at position N30 (asparagine at position 30) is replaced by A (an N30A amino acid change). In one aspect, the HVR-L1 is any one of SEQ ID NO:9, 10, 19, 20, 21, 22, or 23 and the amino acid at position N30 (asparagine at position 30) is replaced by Q (an N30Q amino acid change).

In one aspect, the invention provides a combination of an anti-CD20 antibody as defined herein with an anti-CD22 antibody in said antibody-drug conjugate according to the invention comprising (a) an HVR-H3 comprising an amino acid sequence of SEQ ID NO:6 and (b) an HVR-L3 comprising an amino acid sequence of SEQ ID NO:14. In some aspects, the CD22 antibody in said antibody-drug conjugate according to the invention further comprises (a) an HVR-H1 comprising SEQ ID NO:2 and an HVR-H2 comprising SEQ ID NO:4.

In one aspect, the invention provides a combination of an anti-CD20 antibody as defined herein with an anti-CD22 antibody in said antibody-drug conjugate according to the invention comprising (a) an HVR-H3 comprising an amino acid sequence of SEQ ID NO:6 and (b) an HVR-L2 comprising an amino acid sequence of SEQ ID NO:12. In some aspects, the CD22 antibody in said antibody-drug conjugate according to the invention further comprises (a) an HVR-H1 comprising SEQ ID NO:2 and an HVR-H2 comprising SEQ ID NO:4.

In one aspect, the invention provides a combination of an anti-CD20 antibody as defined herein with an anti-CD22 antibody in said antibody-drug conjugate according to the invention comprising (a) an HVR-H3 comprising an amino acid sequence of SEQ ID NO:6 and (b) an HVR-L1 comprising an amino acid sequence selected from SEQ ID NO:9, 10, 19, 20, 21, 22, and 23. In some aspects, the CD22 antibody further comprises (a) an HVR-H1 comprising SEQ ID NO:2 and an HVR-H2 comprising SEQ ID NO:4. In some aspects, the amino acid sequence of SEQ ID NO:9, 10, 19, 20, 21, 22, or 23 comprises an N30A or N30Q amino acid change. In some aspects, the CD22 antibody in said antibody-drug conjugate according to the invention further comprises HVR-L2 comprising the amino acid sequence of SEQ ID NO:12. In some aspects, the CD22 antibody in said antibody-drug conjugate according to the invention further comprises HVR-L3 comprising the amino acid sequence of SEQ ID NO:14.

In one aspect, the invention provides a combination of an anti-CD20 antibody as defined herein with an anti-CD22 antibody in said antibody-drug conjugate according to the invention comprising (a) an HVR-H1 comprising the amino acid sequence of SEQ ID NO:2; (b) an HVR-H2 comprising the amino acid sequence of SEQ ID NO:4; (c) an HVR-H3 comprising the amino acid sequence of SEQ ID NO:6; (d) an HVR-L1 comprising the amino acid sequence selected from SEQ ID NO:9, 10, 19, 20, 21, 22, 23; (e) an HVR-L2 comprising the amino acid sequence of SEQ ID NO:12; and an HVR-L3 comprising the amino acid sequence of SEQ ID NO:14. In some aspects, the invention further provides that the amino acid sequence SEQ ID NO:9, 10, 19, 20, 21, 22, or 23 selected as HVR-L1 is modified by an N30A or an N30Q amino acid change.

In one aspect, the invention provides a combination of an anti-CD20 antibody as defined herein with an anti-CD22 antibody in said antibody-drug conjugate according to the invention comprising a heavy chain variable domain comprising SEQ ID NO:16 (see Figure 3A, h10F4v1). In one aspect, the invention provides an anti-CD22 antibody in said antibody-drug conjugate according to the invention comprising a light chain variable domain comprising SEQ ID NO:17 (see Figure 3B, h10F4v1). In one aspect, the invention provides an anti-CD22 antibody in said antibody-drug conjugate according to the invention comprising a light chain variable domain comprising SEQ ID NO:18 (see Figure 3B, h10F4v3).

In one aspect, the invention provides a combination of an anti-CD20 antibody as defined herein with an anti-CD22 antibody in said antibody-drug conjugate according to the invention comprising a heavy chain comprising SEQ ID NO:26 (see Figure 3A, m10F4). In one aspect, the invention provides an anti-CD22 antibody in said antibody-drug conjugate according to the invention comprising a light chain comprising SEQ ID NO:27 (see Figure 3B, m10F4).

In one aspect, the invention provides a combination of an anti-CD20 antibody as defined herein with an anti-CD22 antibody in said antibody-drug conjugate according to the invention comprising 1, 2, 3, 4, 5, or 6 of the HVR sequences of the antibody 10F4.4.1 produced by the hybridoma deposited with the ATCC and having accession number PTA-7621.

In one aspect, the invention provides a combination of an anti-CD20 antibody as defined herein with an anti-CD22 antibody in said antibody-drug conjugate according to the invention comprising 1, 2, 3, 4, 5, or 6 of the HVR sequences of the antibody 5E8.1.8 produced by the hybridoma deposited with the ATCC and having accession number PTA-7620.

An anti-CD22 antibody in said antibody-drug conjugate according to the invention for combination with an anti-CD20 antibody as defined herein may comprise any suitable framework variable domain sequence, provided that the antibody retains the ability to bind CD22. For example, in some aspects, anti-CD22 antibodies in said antibody-drug conjugate according to the invention comprise a human subgroup III heavy chain framework consensus sequence. In one aspect of these antibodies, the heavy chain framework consensus sequence comprises substitution(s) at position 71, 73 and/or 78. In one aspect of these antibodies, position 71 is A, position 73 is T, and/or position 78 is A. In one aspect, these antibodies comprise a heavy chain variable domain framework sequence of huMAb4D5-8, e.g., SEQ ID NO:1, 3, 5, 7 (FR-H1, FR-H2, FR-H3, FR-H4, respectively). huMAb4D5-8 is commercially known as HERCEPTIN® anti-HER2 antibody, Genentech, Inc., South San Francisco, CA, USA; also referred to in U.S. Pat. Nos. 6,407,213 & 5,821,337, and Lee et al., J. Mol. Biol. (2004), 340(5): 1073-93. In one such aspect, these antibodies further comprise a human κI light chain framework consensus sequence. In one such aspect, these antibodies comprise a light chain variable domain framework sequence of huMAb4D5-8, e.g. SEQ ID NO:8, 1, 13, 15 (FR-L1, FR-L2, FR-L3, FR-L4, respectively).

In one aspect, an anti-CD22 antibody in said antibody-drug conjugate according to the invention for combination with an anti-CD20 antibody as defined herein comprises a heavy chain variable domain comprising a framework sequence and hypervariable regions, wherein the framework sequence comprises the FR-H1-FR-H4 sequences SEQ ID NO:1, 3, 5, and 7, respectively; the HVR H1 comprises the amino acid sequence of SEQ ID NO:2; the HVR-H2 comprises the amino acid sequence of SEQ ID NO:4; and the HVR-H3 comprises an amino acid sequence selected from SEQ ID NO:6. In one aspect, an anti-CD22 antibody comprises a light chain variable domain comprising a framework sequence and hypervariable regions, wherein the framework sequence comprises the FR-L1-FR-L4 sequences of SEQ ID NO:8, 11, 13, and 15, respectively; the HVR-L1 comprises the amino acid sequence selected from SEQ ID NO:9, 10, 19, 20, 21, 22, and 23, wherein any one of SEQ ID NOS:9-10 or 19-23 may comprise a N30A or N30Q amino acid change; the HVR-L2 comprises the amino acid sequence of SEQ ID NO:12; and the HVR-L3 comprises an amino acid sequence selected from SEQ ID NO:14. In one aspect of these antibodies, the heavy chain variable domain comprises SEQ ID NO:16 and the light chain variable domain comprises SEQ ID NO:17 or 18.

In some aspects, the invention provides a combination of an anti-CD20 antibody as defined herein with an anti-CD22 antibody in said antibody-drug conjugate according to the invention comprising a heavy chain variable domain comprising an amino acid sequence having at least 90%, 91%, 92%, 93%, 94%, 95%, 96%, 97%, 98%, or 99% sequence identity to an amino acid sequence SEQ ID NO:16. In some aspects, an amino acid sequence having at least 90%, 91%, 92%, 93%, 94%, 95%, 96%, 97%, 98%, or 99% sequence identity contains substitutions, insertions, or deletions relative to the reference sequence, but an antibody comprising that amino acid sequence retains the ability to bind to CD22. In some aspects, a total of 1 to 10 amino acids have been substituted, inserted, or deleted in a sequence SEQ ID NO:16. In some aspects, the substitutions, insertions, or deletions occur in regions outside the HVRs (i.e., in the FRs). In some aspects, an anti-CD22 antibody in said antibody-drug conjugate according to the invention for combination with an anti-CD20 antibody as defined herein comprises a heavy chain variable domain comprising an amino acid sequence selected from SEQ ID NO:16.

In some embodiments, the invention provides a combination of an anti-CD20 antibody as defined herein with an anti-CD22 antibody in said antibody-drug conjugate according to the invention comprising a heavy chain variable domain as depicted in below.
1 Glu Val Gln Leu Val Glu Ser Gly Gly Gly Leu Val Gln Pro Gly Gly Ser Leu Arg Leu Ser Cys Ala Ala Ser Gly Tyr Glu Phe Ser Arg Ser Trp Met Asn Trp Val Arg Gln Ala Pro Gly Lys Gly Leu Glu Trp Val Gly Arg Ile Tyr Pro Gly Asp Gly Asp Thr Asn Tyr Ser Gly Lys Phe Lys Gly Arg Phe Thr Ile Ser Ala Asp Thr Ser Lys Asn Thr Ala Tyr Leu Gln Met Asn Ser Leu Arg Ala Glu Asp Thr Ala Val Tyr Tyr Cys Ala Arg Asp Gly Ser Ser Trp Asp Trp Tyr Phe Asp Val Trp Gly Gln Gly Thr Leu Val Thr Val Ser Ser 113 (SEQ ID NO:16) (HVR residues are underlined).

In some aspects, the heavy chain HVR and FR sequences comprise the following:
HVR-H1 (Gly Tyr Glu Phe Ser Arg Ser Trp Met Asn, SEQ ID NO:2)
HVR-H3 (Asp Gly Ser Ser Trp Asp Trp Tyr Phe Asp Val, SEQ ID NO:6)
FR-H2 (Trp Val Arg Gln Ala Pro Gly Lys Gly Leu Glu Trp Val, SEQ ID NO:3)
FR-H4 (Trp Gly Gln Gly Thr Leu Val Thr Val Ser Ser, SEQ ID NO:7)

In some embodiments, the invention provides a combination of an anti-CD20 antibody as defined herein with an anti-CD22 antibody in said antibody-drug conjugate according to the invention comprising a light chain variable domain as depicted in below.
1 Asp lie Gln Met Thr Gln Ser Pro Ser Ser Leu Ser Ala Ser Val Gly Asp Arg Val Thr Ile Thr Cys Arg Ser Ser Gln Ser Ile Val His Ser **Asn** Gly Asn Thr Phe Leu Glu Trp Tyr Gln Gln Lys Pro Gly Lys Ala Pro Lys Leu Leu Ile Tyr Lys Val Ser Asn Arg Phe Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Phe Gln Gly Ser Gln Phe Pro Tyr Thr Phe Gly Gln Gly Thr Lys Val Glu Ile Lys 108 (SEQ ID NO:17) (HVR residues are underlined and position N28 is in bold type) 1 Asp Ile Gln Met Thr Gln Ser Pro Ser Ser Leu Ser Ala Ser Val Gly Asp Arg Val Thr Ile Thr Cys Arg Ser Ser Gln Ser Ile Val His Ser Val Gly Asn Thr Phe Leu Glu Trp Tyr Gln Gln Lys Pro Gly Lys Ala Pro Lys Leu Leu Ile Tyr Lys Val Ser Asn Arg Phe Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Phe Gln Gly Ser Gln Phe Pro Tyr Thr Phe Gly Gln Gly Thr Lys Val Glu Ile Lys 108 (SEQ ID NO:18) (HVR residues are underlined and N28V is in bold type).

In some aspects, the light chain HVR sequences comprise the following: HVR-L2 (Lys Val Ser Asn Arg Phe Ser, SEQ ID NO:12)
HVR-L3 (Phe Gln Gly Ser Gln Phe Pro Tyr Thr, SEQ ID NO:14).

In some aspects, the light chain FR sequences comprise the following: FR-L2 (Trp Tyr Gln Gln Lys Pro Gly Lys Ala Pro Lys Leu Leu Ile Tyr, SEQ ID NO:11); FR-L4 (Phe Gly Gln Gly Thr Lys Val Glu Ile Lys , SEQ ID NO:15).

In one aspect, the invention provides a combination of an anti-CD20 antibody as defined herein with an anti-CD22 antibody in said antibody-drug conjugate according to the invention comprising a light chain variable domain comprising an amino acid sequence having at least 90%, 91%, 92%, 93%, 94%, 95%, 96%, 97%, 98%, or 99% sequence identity to an amino acid sequence selected from SEQ ID NO:17 or 18. In some aspects, an amino acid sequence having at least 90%, 91%, 92%, 93%, 94%, 95%, 96%, 97%, 98%, or 99% sequence identity contains substitutions, additions, or deletions relative to the reference sequence, but an antibody comprising that amino acid sequence retains the ability to bind to CD22. In some aspects, a total of 1 to 10 amino acids have been substituted, inserted, or deleted in a sequence selected from SEQ ID NO:17 or 18. In some aspects, the substitutions, insertions, or deletions occur in regions outside the HVRs (i.e., in the FRs). In some aspects, an anti-CD22 antibody in said antibody-drug conjugate according to the invention for combination with an anti-CD20 antibody as defined herein comprises a light chain variable domain comprising an amino acid sequence selected from SEQ ID NO:17 or 18.

In one aspect, the invention provides a combination of an anti-CD20 antibody as defined herein with an anti-CD22 antibody in said antibody-drug conjugate according to the invention comprising (a) a heavy chain variable domain comprising an amino acid sequence having at least 90%, 91%, 92%, 93%, 94%, 95%, 96%, 97%, 98%, or 99% sequence identity to an amino acid sequence selected from SEQ ID NO:16; and (b) a light chain variable domain comprising an amino acid sequence having at least 90%, 91%, 92%, 93%, 94%, 95%, 96%, 97%, 98%, or 99% sequence identity to an amino acid sequence selected from SEQ ID NO:17 or 18. In some aspects, an amino acid sequence having at least 90%, 91%, 92%, 93%, 94%, 95%, 96%, 97%, 98%, or 99% sequence identity contains substitutions, additions, or deletions relative to the reference sequence, but an antibody comprising that amino acid sequence retains the ability to bind to CD22. In some aspects, a total of 1 to 10 amino acids have been substituted, inserted, or deleted in the reference sequence. In some aspects, the substitutions, insertions, or deletions occur in regions outside the HVRs (i.e., in the FRs). In some aspects, an anti-CD22 antibody in said antibody-drug conjugate according to the invention for combination with an anti-CD20 antibody as defined herein comprises a heavy chain variable domain comprising an amino acid sequence of SEQ ID NO: 16 and a light chain variable domain comprising an amino acid sequence selected from SEQ ID NO:18.

In one aspect, the anti-CD22 antibody in said antibody-drug conjugate according to the invention comprises 1, 2, 3, 4, 5, or 6 of the hypervariable regions of the 5E8.1.8 antibody produced by the hybridoma deposited with the ATCC and having accession no. PTA-7620.

### Antibody Fragments

The present invention encompasses antibody fragments. Antibody fragments may be generated by traditional means, such as enzymatic digestion, or by recombinant techniques. In certain circumstances there are advantages of using antibody fragments, rather than whole antibodies. The smaller size of the fragments allows for rapid clearance, and may lead to improved access to solid tumors. For a review of certain antibody fragments, see Hudson et al. (2003) Nat. Med. 9:129-134.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from E. coli, thus allowing the facile production of large amounts of these fragments. Antibody fragments can be isolated from the antibody phage libraries discussed above. Alternatively, Fab'-SH fragments can be directly recovered from E. coli and chemically coupled to form F(ab')₂ fragments (Carter et al., Bio/Technology 10:163-167 (1992)). According to another approach, F(ab')₂ fragments can be isolated directly from recombinant host cell culture. Fab and F(ab')₂ fragment with increased in vivo half-life comprising salvage receptor binding epitope residues are described in U.S. Pat. No. 5,869,046. Other techniques for the production of antibody fragments will be apparent to the skilled practitioner. In certain aspects, an antibody is a single chain Fv fragment (scFv). See WO 93/16185; U.S. Pat. Nos. 5,571,894; and 5,587,458. Fv and scFv are the only species with intact combining sites that are devoid of constant regions; thus, they may be suitable for reduced nonspecific binding during in vivo use. scFv fusion proteins may be constructed to yield fusion of an effector protein at either the amino or the carboxy terminus of an scFv. See Antibody Engineering, ed. Borrebaeck, supra. The antibody fragment may also be a "linear antibody", e.g., as described in U.S. Pat. No. 5,641,870, for example. Such linear antibodies may be monospecific or bispecific.

### Humanized Antibodies

The invention encompasses humanized antibodies. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies can be important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody (Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies (Carter et al. (1992) Proc. Natl. Acad. Sci. USA, 89:4285; Presta et al. (1993) J. Immunol., 151:2623.

It is further generally desirable that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

### Human Antibodies

Human anti-CD22 antibodies of the invention can be constructed by combining Fv clone variable domain sequence(s) selected from human-derived phage display libraries with known human constant domain sequences(s) as described above. Alternatively, human monoclonal anti-CD22 antibodies of the invention can be made by the hybridoma method. Human myeloma and mouse-human heteromyeloma cell lines for the production of human monoclonal antibodies have been described, for example, by Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (e.g. mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci USA, 90: 2551 (1993); Jakobovits et al., Nature, 362: 255 (1993); Bruggermann et al., Year in Immunol., 7: 33 (1993).

Gene shuffling can also be used to derive human antibodies from non-human, e.g. rodent, antibodies, where the human antibody has similar affinities and specificities to the starting non-human antibody. According to this method, which is also called "epitope imprinting", either the heavy or light chain variable region of a non-human antibody fragment obtained by phage display techniques as described herein is replaced with a repertoire of human V domain genes, creating a population of non-human chain/human chain scFv or Fab chimeras. Selection with antigen results in isolation of a non-human chain/human chain chimeric scFv or Fab wherein the human chain restores the antigen binding site destroyed upon removal of the corresponding non-human chain in the primary phage display clone, i.e. the epitope governs (imprints) the choice of the human chain partner. When the process is repeated in order to replace the remaining non-human chain, a human antibody is obtained (see PCT WO 93/06213 published April 1, 1993). Unlike traditional humanization of non-human antibodies by CDR grafting, this technique provides completely human antibodies, which have no FR or CDR residues of non-human origin.

### Bispecific Antibodies

Bispecific antibodies are monoclonal antibodies that have binding specificities for at least two different antigens. In certain aspects, bispecific antibodies are human or humanized antibodies. In certain aspects, one of the binding specificities is for CD22 and the other is for any other antigen. In certain aspects, bispecific antibodies may bind to two different epitopes of CD22. Bispecific antibodies may also be used to localize cytotoxic agents to cells which express CD22. These antibodies possess a CD22-binding arm and an arm which binds a cytotoxic agent, such as, e.g., saporin, anti-interferon-a, vinca alkaloid, ricin A chain, methotrexate or radioactive isotope hapten. Bispecific antibodies can be prepared as full length antibodies or antibody fragments (e.g. F(ab')₂ bispecific antibodies).

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

According to a different approach, antibody variable domains with the desired binding specificities (antibody-antigen combining sites) are fused to immunoglobulin constant domain sequences. The fusion, for example, is with an immunoglobulin heavy chain constant domain, comprising at least part of the hinge, CH2, and CH3 regions. In certain aspects, the first heavy-chain constant region (CH1), containing the site necessary for light chain binding, is present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in aspects when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one aspect of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach, the interface between a pair of antibody molecules can be engineered to maximize the percentage of heterodimers which are recovered from recombinant cell culture. The interface comprises at least a part of the C_{H}3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/00373, and EP 03089). Heteroconjugate antibodies may be made using any convenient cross-linking method. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from E. coli, which can be chemically coupled to form bispecific antibodies. Shalaby et al., J. Exp. Med., 175: 217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the HER2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. In certain aspects, the dimerization domain comprises (or consists of) an Fc region or a hinge region. In this scenario, the antibody will comprise an Fc region and three or more antigen binding sites amino-terminal to the Fc region. In certain aspects, a multivalent antibody comprises (or consists of) three to about eight antigen binding sites. In one such aspect, a multivalent antibody comprises (or consists of) four antigen binding sites. The multivalent antibody comprises at least one polypeptide chain (for example, two polypeptide chains), wherein the polypeptide chain(s) comprise two or more variable domains. For instance, the polypeptide chain(s) may comprise VD1-(X1)n -VD2-(X2)n -Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein may further comprise at least two (for example, four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### Single-Domain Antibodies

In some aspects, an antibody of the invention is a single-domain antibody. A single-domain antibody is a single polypeptide chain comprising all or a portion of the heavy chain variable domain or all or a portion of the light chain variable domain of an antibody. In certain aspects, a single-domain antibody is a human single-domain antibody (Domantis, Inc., Waltham, MA; see, e.g., U.S. Patent No. 6,248,516 B1). In one aspect, a single-domain antibody consists of all or a portion of the heavy chain variable domain of an antibody.

### Antibody Variants

In some aspects, amino acid sequence modification(s) of the antibodies described herein are contemplated. For example, it may be desirable to improve the binding affinity and/or other biological properties of the antibody. Amino acid sequence variants of the antibody may be prepared by introducing appropriate changes into the nucleotide sequence encoding the antibody, or by peptide synthesis. Such modifications include, for example, deletions from, and/or insertions into and/or substitutions of, residues within the amino acid sequences of the antibody. Any combination of deletion, insertion, and substitution can be made to arrive at the final construct, provided that the final construct possesses the desired characteristics. The amino acid alterations may be introduced in the subject antibody amino acid sequence at the time that sequence is made.

A useful method for identification of certain residues or regions of the antibody that are preferred locations for mutagenesis is called "alanine scanning mutagenesis" as described by Cunningham and Wells (1989) Science, 244:1081-1085. Here, a residue or group of target residues are identified (e.g., charged residues such as arg, asp, his, lys, and glu) and replaced by a neutral or negatively charged amino acid (e.g., alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed immunoglobulins are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

In certain aspects, an antibody of the invention is altered to increase or decrease the extent to which the antibody is glycosylated. Glycosylation of polypeptides is typically either N-linked or O-linked. N-linked refers to the attachment of a carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition or deletion of glycosylation sites to the antibody is conveniently accomplished by altering the amino acid sequence such that one or more of the above-described tripeptide sequences (for N-linked glycosylation sites) is created or removed. The alteration may also be made by the addition, deletion, or substitution of one or more serine or threonine residues to the sequence of the original antibody (for O-linked glycosylation sites).

Where the antibody comprises an Fc region, the carbohydrate attached thereto may be altered. For example, antibodies with a mature carbohydrate structure that lacks fucose attached to an Fc region of the antibody are described in US Pat Appl No US 2003/0157108 (Presta, L.). See also US 2004/0093621 (Kyowa Hakko Kogyo Co., Ltd). Antibodies with a bisecting N-acetylglucosamine (GlcNAc) in the carbohydrate attached to an Fc region of the antibody are referenced in WO 2003/011878, Jean-Mairet et al. and US Patent No. 6,602,684, Umana et al. Antibodies with at least one galactose residue in the oligosaccharide attached to an Fc region of the antibody are reported in WO 1997/30087, Patel et al. See, also, WO 1998/58964 (Raju, S.) and WO 1999/22764 (Raju, S.) concerning antibodies with altered carbohydrate attached to the Fc region thereof. See also US 2005/0123546 (Umana et al.) on antigen-binding molecules with modified glycosylation.

In certain aspects, a glycosylation variant comprises an Fc region, wherein a carbohydrate structure attached to the Fc region lacks fucose. Such variants have improved ADCC function. Optionally, the Fc region further comprises one or more amino acid substitutions therein which further improve ADCC, for example, substitutions at positions 298, 333, and/or 334 of the Fc region (Eu numbering of residues). Examples of publications related to "defucosylated" or "fucose-deficient" antibodies include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; Okazaki et al. J. Mol. Biol. 336:1239-1249 (2004); Yamane-Ohnuki et al. Biotech. Bioeng. 87: 614 (2004). Examples of cell lines producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, FUT8, knockout CHO cells (Yamane-Ohnuki et al. Biotech. Bioeng. 87: 614 (2004)).

In one aspect, the antibody is altered to improve its serum half-life. To increase the serum half life of the antibody, one may incorporate a salvage receptor binding epitope into the antibody (especially an antibody fragment) as described in US 5739277, for example. As used herein, the term "salvage receptor binding epitope" refers to an epitope of the Fc region of an IgG molecule (e.g., IgG1, IgG2, IgG3, or IgG4) that is responsible for increasing the in vivo serum half-life of the IgG molecule (US 2003/0190311, US6821505; US 6165745; US 5624821; US 5648260; US 6165745;US 5834 597).

Another type of variant is an amino acid substitution variant. These variants have at least one amino acid residue in the antibody molecule replaced by a different residue. Sites of interest for substitutional mutagenesis include the hypervariable regions, but FR alterations are also contemplated. Conservative substitutions are shown in Table 1 under the heading of "preferred substitutions." If such substitutions result in a desirable change in biological activity, then more substantial changes, denominated "exemplary substitutions" in Table 1, or as further described below in reference to amino acid classes, may be introduced and the products screened.

**TABLE 2**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)):
(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class. Such substituted residues also may be introduced into the conservative substitution sites or, into the remaining (non-conserved) sites.

One type of substitutional variant involves substituting one or more hypervariable region residues of a parent antibody (e.g. a humanized or human antibody). Generally, the resulting variant(s) selected for further development will have modified (e.g., improved) biological properties relative to the parent antibody from which they are generated. A convenient way for generating such substitutional variants involves affinity maturation using phage display. Briefly, several hypervariable region sites (e.g. 6-7 sites) are mutated to generate all possible amino acid substitutions at each site. The antibodies thus generated are displayed from filamentous phage particles as fusions to at least part of a phage coat protein (e.g., the gene III product of M13) packaged within each particle. The phage-displayed variants are then screened for their biological activity (e.g. binding affinity). In order to identify candidate hypervariable region sites for modification, scanning mutagenesis (e.g., alanine scanning) can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to techniques known in the art, including those elaborated herein. Once such variants are generated, the panel of variants is subjected to screening using techniques known in the art, including those described herein, and antibodies with superior properties in one or more relevant assays may be selected for further development.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to introduce one or more amino acid modifications in an Fc region of antibodies of the invention, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions including that of a hinge cysteine.

In accordance with this description and the teachings of the art, it is contemplated that in some aspects, an antibody of the invention may comprise one or more alterations as compared to the wild type counterpart antibody, e.g. in the Fc region. These antibodies would nonetheless retain substantially the same characteristics required for therapeutic utility as compared to their wild type counterpart. For example, it is thought that certain alterations can be made in the Fc region that would result in altered (i.e., either improved or diminished) C1q binding and/or Complement Dependent Cytotoxicity (CDC), e.g., as described in WO99/51642. See also Duncan & Winter Nature 322:738-40 (1988); U.S. Patent No. 5,648,260; U.S. Patent No. 5,624,821; and WO94/29351 concerning other examples of Fc region variants. WO00/42072 (Presta) and WO 2004/056312 (Lowman) describe antibody variants with improved or diminished binding to FcRs. See, also, Shields et al. J. Biol. Chem. 9(2): 6591-6604 (2001). Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). These antibodies comprise an Fc region with one or more substitutions therein which improve binding of the Fc region to FcRn. Polypeptide variants with altered Fc region amino acid sequences and increased or decreased C1q binding capability are described in US patent No. 6,194,551B1, WO99/51642. See, also, Idusogie et al. J. Immunol. 164: 4178-4184 (2000).

In one aspect, the invention provides antibodies comprising modifications in the interface of Fc polypeptides comprising the Fc region, wherein the modifications facilitate and/or promote heterodimerization. These modifications comprise introduction of a protuberance into a first Fc polypeptide and a cavity into a second Fc polypeptide, wherein the protuberance is positionable in the cavity so as to promote complexing of the first and second Fc polypeptides. Methods of generating antibodies with these modifications are known in the art, e.g., as described in U.S. Pat. No. 5,731,168.

### Antibody Derivatives

The antibodies of the present invention can be further modified to contain additional nonproteinaceous moieties that are known in the art and readily available. Preferably, the moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another aspect, conjugates of an antibody and nonproteinaceous moiety that may be selectively heated by exposure to radiation are provided. In one aspect, the nonproteinaceous moiety is a carbon nanotube (Kam et al., Proc. Natl. Acad. Sci. 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

The term "CD22 antibody-drug conjugate" according to the invention refers to agents having the formula Ab-(L-D)p, wherein
(a) Ab is the CD22 antibody as disclosed herein;
(b) L is a linker;
(c) D is a drug moiety.

In one embodiment of the method according to the invention, the CD22 antibody-drug conjugate is anti-CD22-MC-vc-PAB-MMAE.

### Antibody-Drug Conjugates

In another aspect, the invention provides immunoconjugates, or antibody-drug conjugates (ADC), comprising an antibody conjugated to a cytotoxic agent such as a chemotherapeutic agent, a drug, a growth inhibitory agent, a toxin (e.g., an enzymatically active toxin of bacterial, fungal, plant, or animal origin, or fragments thereof), or a radioactive isotope (i.e., a radioconjugate). In another aspect, the invention further provides methods of using the immunoconjugates. In one aspect, an immunoconjugate comprises any of the above anti-CD22 antibodies covalently attached to a cytotoxic agent or a detectable agent for combination with an anti-CD20 antibody as defined herein.

The use of antibody-drug conjugates for the local delivery of cytotoxic or cytostatic agents, i.e. drugs to kill or inhibit tumor cells in the treatment of cancer (Syrigos and Epenetos (1999) Anticancer Research 19:605-614; Niculescu-Duvaz and Springer (1997) Adv. Drg Del. Rev. 26:151-172; U.S. patent 4,975,278) allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al. (ed.s), pp. 475-506). Maximal efficacy with minimal toxicity is sought thereby. Both polyclonal antibodies and monoclonal antibodies have been reported as useful in these strategies (Rowland et al., (1986) Cancer Immunol. Immunother., 21:183-87). Drugs used in these methods include daunomycin, doxorubicin, methotrexate, and vindesine (Rowland et al., (1986) supra). Toxins used in antibody-toxin conjugates include bacterial toxins such as diphtheria toxin, plant toxins such as ricin, small molecule toxins such as geldanamycin (Mandler et al (2000) Jour. of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may affect their cytotoxic and cytostatic effects by mechanisms including tubulin binding, DNA binding, or topoisomerase inhibition. Some cytotoxic drugs tend to be inactive or less active when conjugated to large antibodies or protein receptor ligands.

ZEVALIN® (ibritumomab tiuxetan, Biogen/Idec) is an antibody-radioisotope conjugate composed of a murine IgG1 kappa monoclonal antibody directed against the CD20 antigen found on the surface of normal and malignant B lymphocytes and ¹¹¹In or ⁹⁰Y radioisotope bound by a thiourea linker-chelator (Wiseman et al (2000) Eur. Jour. Nucl. Med. 27(7):766-77; Wiseman et al (2002) Blood 99(12):4336-42; Witzig et al (2002) J. Clin. Oncol. 20(10):2453-63; Witzig et al (2002) J. Clin. Oncol. 20(15):3262-69). Although ZEVALIN has activity against B-cell non-Hodgkin's Lymphoma (NHL), administration results in severe and prolonged cytopenias in most patients. MYLOTARG™ (gemtuzumab ozogamicin, Wyeth Pharmaceuticals), an antibody drug conjugate composed of a hu CD33 antibody linked to calicheamicin, was approved in 2000 for the treatment of acute myeloid leukemia by injection (Drugs of the Future (2000) 25(7):686; US Patent Nos. 4970198; 5079233; 5585089; 5606040; 5693762; 5739116; 5767285; 5773001). Cantuzumab mertansine (Immunogen, Inc.), an antibody drug conjugate composed of the huC242 antibody linked via the disulfide linker SPP to the maytansinoid drug moiety, DM1, is advancing into Phase II trials for the treatment of cancers that express CanAg, such as colon, pancreatic, gastric, and others. MLN-2704 (Millennium Pharm., BZL Biologics, Immunogen Inc.), an antibody drug conjugate composed of the anti-prostate specific membrane antigen (PSMA) monoclonal antibody linked to the maytansinoid drug moiety, DM1, is under development for the potential treatment of prostate tumors. The auristatin peptides, auristatin E (AE) and monomethylauristatin (MMAE), synthetic analogs of dolastatin, were conjugated to chimeric monoclonal antibodies cBR96 (specific to Lewis Y on carcinomas) and cAC10 (specific to CD30 on hematological malignancies) (Doronina et al (2003) Nature Biotechnology 21(7):778-784) and are under therapeutic development.

Chemotherapeutic agents useful in the generation of immunoconjugates are described herein. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. See, e.g., WO 93/21232 published October 28, 1993. A variety of radionuclides are available for the production of radioconjugated antibodies. Examples include ²¹²Bi, ¹³¹I, ¹³¹In, ⁹⁰Y, and ¹⁸⁶Re. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al (1987) Science, 238:1098. Carbon-14-labeled 1-isothiocyanatobenzyl-3-methyldiethylene triaminepentaacetic acid (MX-DTPA) is an exemplary chelating agent for conjugation of radionucleotide to the antibody (WO94/11026).

Conjugates of an antibody and one or more small molecule toxins, such as a calicheamicin, maytansinoids, dolastatins, auristatins, a trichothecene, and CC1065, and the derivatives of these toxins that have toxin activity, are also contemplated herein.

### Maytansine and maytansinoids

In some aspects, the immunoconjugate comprises an antibody (full length or fragments) of the invention conjugated to one or more maytansinoid molecules.

Maytansinoids are mitototic inhibitors which act by inhibiting tubulin polymerization. Maytansine was first isolated from the east African shrub Maytenus serrata (U.S. Patent No. 3896111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

Maytansinoid drug moieties are attractive drug moieties in antibody drug conjugates because they are: (i) relatively accessible to prepare by fermentation or chemical modification, derivatization of fermentation products, (ii) amenable to derivatization with functional groups suitable for conjugation through the non-disulfide linkers to antibodies, (iii) stable in plasma, and (iv) effective against a variety of tumor cell lines.

Maytansine compounds suitable for use as maytansinoid drug moieties are well known in the art, and can be isolated from natural sources according to known methods, produced using genetic engineering techniques (see Yu et al (2002) PNAS 99:7968-7973), or maytansinol and maytansinol analogues prepared synthetically according to known methods.

Exemplary maytansinoid drug moieties include those having a modified aromatic ring, such as: C-19-dechloro (US 4256746) (prepared by lithium aluminum hydride reduction of ansamytocin P2); C-20-hydroxy (or C-20-demethyl) +/-C-19-dechloro (US Pat. Nos. 4361650 and 4307016) (prepared by demethylation using Streptomyces or Actinomyces or dechlorination using LAH); and C-20-demethoxy, C-20-acyloxy (-OCOR), +/-dechloro (U.S. Pat. No. 4,294,757) (prepared by acylation using acyl chlorides), and those having modifications at other positions

Exemplary maytansinoid drug moieties also include those having modifications such as: C-9-SH (US 4424219) (prepared by the reaction of maytansinol with H₂S or P₂S₅); C-14-alkoxymethyl(demethoxy/CH₂ OR)(US 4331598); C-14-hydroxymethyl or acyloxymethyl (CH₂OH or CH₂OAc) (US 4450254) (prepared from Nocardia); C-15-hydroxy/acyloxy (US 4364866) (prepared by the conversion of maytansinol by Streptomyces); C-15-methoxy (US Pat. Nos. 4313946 and 4315929) (isolated from Trewia nudlflora); C-18-N-demethyl (US Pat. Nos. 4362663 and 4322348) (prepared by the demethylation of maytansinol by Streptomyces); and 4,5-deoxy (US 4371533) (prepared by the titanium trichloride/LAH reduction of maytansinol).

Exemplary aspects of maytansinoid drug moieities include: DM1; DM3; and DM4, having the structures: 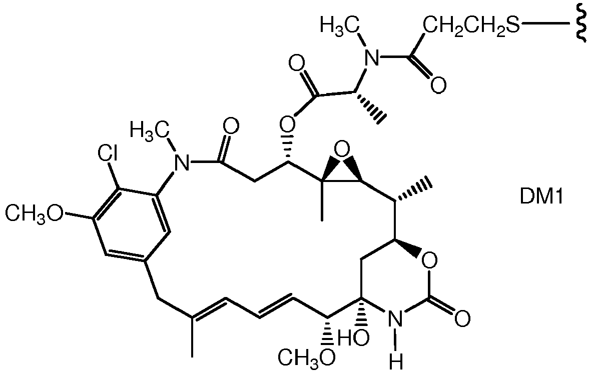 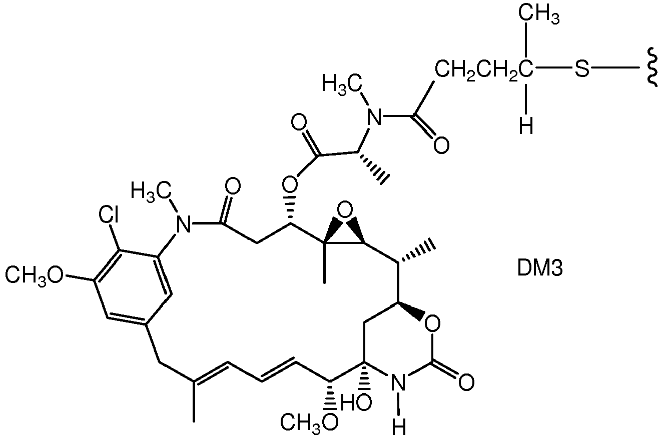 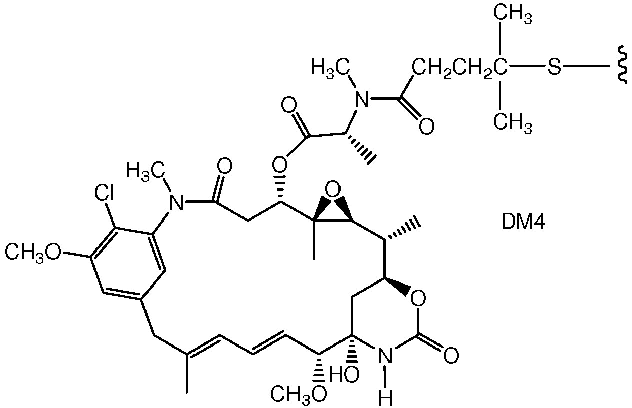 wherein the wavy line indicates the covalent attachment of the sulfur atom of the drug to a linker (L) of an antibody drug conjugate. HERCEPTIN® (trastuzumab, anti-HER2 antibody) linked by SMCC to DM1 has been reported (WO 2005/037992). An antibody drug conjugate of the present invention may be prepared according to the procedures disclosed therein.

Other exemplary maytansinoid antibody drug conjugates have the following structures and abbreviations, (wherein Ab is antibody and p is 1 to about 8):

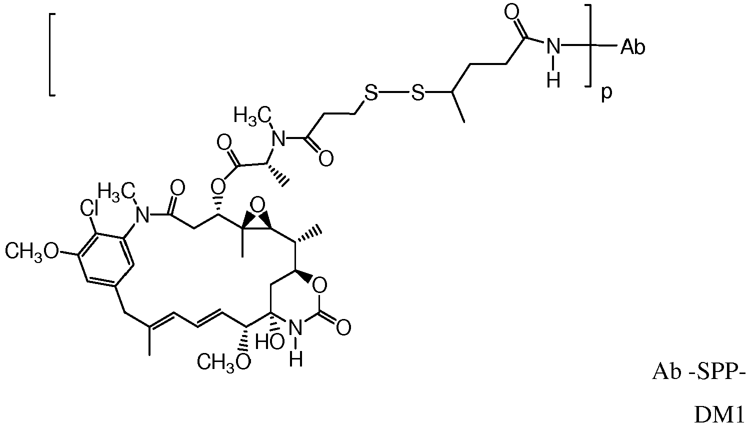

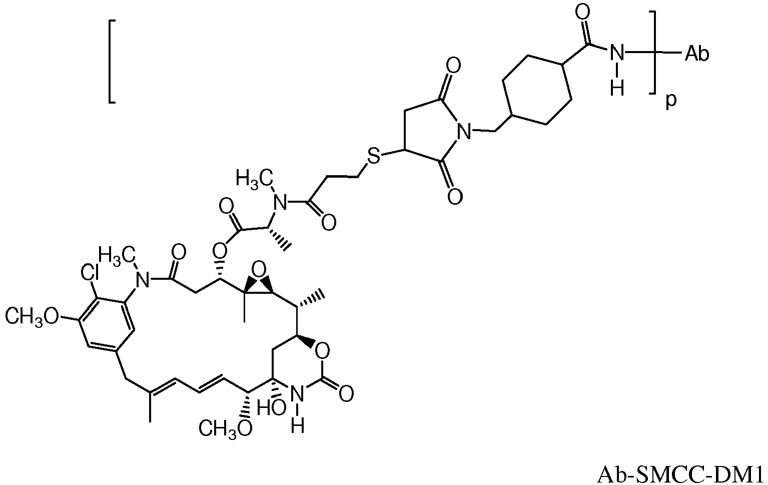

Exemplary antibody-drug conjugates where DM1 is linked through a BMPEO linker to a thiol group of the antibody have the structure and abbreviation: 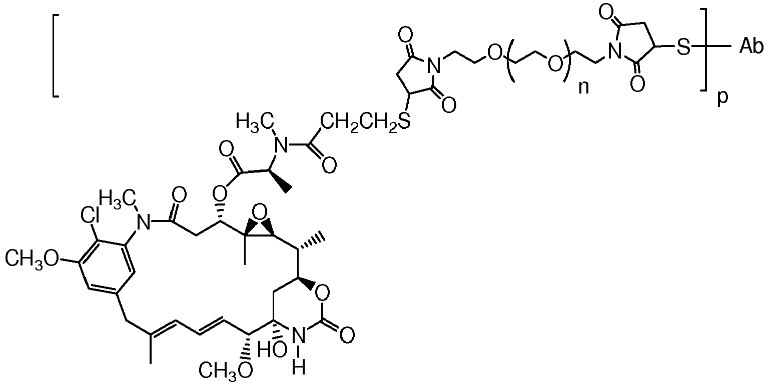 where Ab is antibody; n is 0, 1, or 2; and p is 1, 2, 3, or 4.

Immunoconjugates containing maytansinoids, methods of making same, and their therapeutic use are disclosed, for example, in U.S. Patent Nos. 5,208,020; 5,416,064; 6,441163 and European Patent EP 0 425 235 B1, Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an in vivo tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/neu oncogene. The cytotoxicity of the TA.1-maytansonoid conjugate was tested in vitro on the human breast cancer cell line SK-BR-3, which expresses 3 x 10⁵ HER-2 surface antigens per cell. The drug conjugate achieved a degree of cytotoxicity similar to the free maytansinoid drug, which could be increased by increasing the number of maytansinoid molecules per antibody molecule. The A7-maytansinoid conjugate showed low systemic cytotoxicity in mice.

Anti-CD22 antibody-maytansinoid conjugates for combination with an anti-CD20 antibody as defined herein are prepared by chemically linking an antibody to a maytansinoid molecule without significantly diminishing the biological activity of either the antibody or the maytansinoid molecule. See, e.g., U.S. Patent No. 5,208,020. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent Nos. 5208020, 6441163, or EP Patent 0 425 235 B1, Chari et al., Cancer Research 52:127-131 (1992), and US 2005/0169933 A1. Antibody-maytansinoid conjugates comprising the linker component SMCC may be prepared as disclosed in U.S. Patent Application Publication No. 2005/0276812, filed 31 May 2005, "Antibody Drug Conjugates and Methods". The linking groups include disulfide groups, thioether groups, acid labile groups, photolabile groups, peptidase labile groups, or esterase labile groups, as disclosed in the above-identified patents. Additional linking groups are described and exemplified herein.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 (1978)) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hydroxymethyl, the C-15 position modified with a hydroxyl group, and the C-20 position having a hydroxyl group. In a preferred aspect, the linkage is formed at the C-3 position of maytansinol or a maytansinol analogue.

In one aspect, any of the antibodies of the invention (full length or fragment) is conjugated to one or more maytansinoid molecules. In one aspect of the immunoconjugate, the cytotoxic agent D, is a maytansinoid DM1. In one aspect of the immunoconjugate, the linker is SMCC. In one aspect of the antibody-linker drug conjugate for combination of an anti-CD20 antibody as defined herein, the antibody-linker-drug conjugate is an anti-CD22 antibody as disclosed herein to which is covalently DM1 cytotoxic agent via the SMCC linker.

### Auristatins and dolostatins

In some aspects, the immunoconjugate comprises an antibody of the invention conjugated to dolastatins or dolostatin peptidic analogs and derivatives, the auristatins (US Patent Nos. 5635483; 5780588). Dolastatins and auristatins have been shown to interfere with microtubule dynamics, GTP hydrolysis, and nuclear and cellular division (Woyke et al (2001) Antimicrob. Agents and Chemother. 45(12):3580-3584) and have anticancer (US 5663149) and antifungal activity (Pettit et al (1998) Antimicrob. Agents Chemother. 42:2961-2965). The dolastatin or auristatin drug moiety may be attached to the antibody through the N (amino) terminus or the C (carboxyl) terminus of the peptidic drug moiety (WO 02/088172).

Exemplary auristatin aspects include the N-terminus linked monomethylauristatin drug moieties DE and DF, disclosed in "Senter et al, Proceedings of the American Association for Cancer Research, Volume 45, Abstract Number 623, presented March 28, 2004.

An exemplary auristatin embodiment is MMAE (wherein the wavy line indicates the covalent attachment to a linker (L) of an antibody drug conjugate).

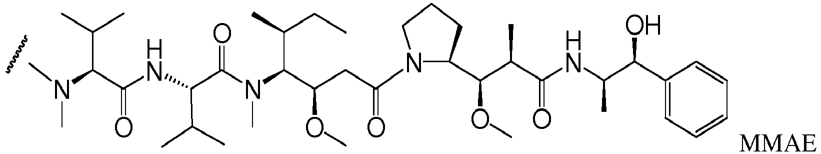

Another exemplary auristatin aspect is MMAF, wherein the wavy line indicates the covalent attachment to a linker (L) of an antibody drug conjugate (US 2005/0238649):

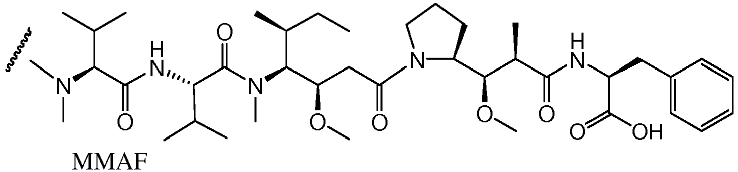

Additional exemplary embodiments comprising MMAE or MMAF and various linker components (described further herein) have the following structures and abbreviations (wherein Ab means antibody and p is 1 to about 8):

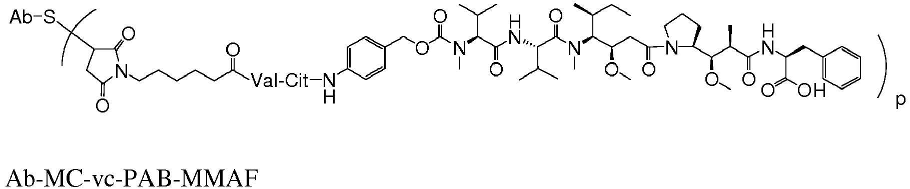

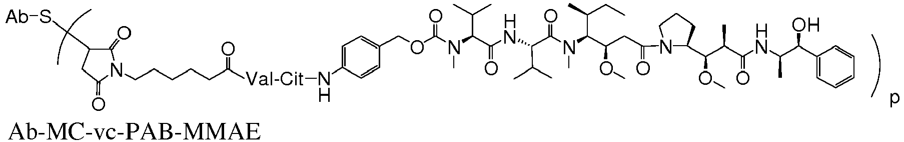

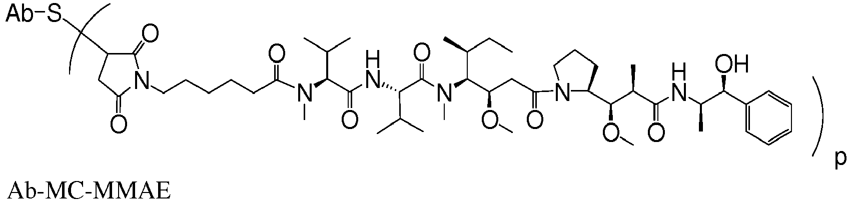

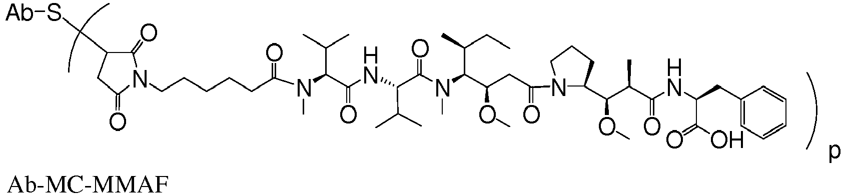

Typically, peptide-based drug moieties can be prepared by forming a peptide bond between two or more amino acids and/or peptide fragments. Such peptide bonds can be prepared, for example, according to the liquid phase synthesis method (see E. Schroder and K. Lübke, "The Peptides", volume 1, pp 76-136, 1965, Academic Press) that is well known in the field of peptide chemistry. The auristatin/dolastatin drug moieties may be prepared according to the methods of: US 5635483; US 5780588; Pettit et al (1989) J. Am. Chem. Soc. 111:5463-5465; Pettit et al (1998) Anti-Cancer Drug Design 13:243-277; Pettit, G.R., et al. Synthesis, 1996, 719-725; Pettit et al (1996) J. Chem. Soc. Perkin Trans. 1 5:859-863; and Doronina (2003) Nat Biotechnol 21(7):778-784.

### Calicheamicin

In other aspects, the immunoconjugate comprises an antibody of the invention conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, γ₁^{I}, α₂^{I}, α₃^{I}, N-acetyl-γ₁^{I}, PSAG and θ^{I}₁ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

### Other cytotoxic agents

Other antitumor agents that can be conjugated to the antibodies of the invention include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated antibodies. Examples include At²¹¹, I¹³¹, I¹²⁵ , Y⁹⁰, Re¹⁸⁶, Re¹⁸⁸, Sm¹⁵³, Bi²¹², P³², Pb²¹² and radioactive isotopes of Lu. When the conjugate is used for detection, it may comprise a radioactive atom for scintigraphic studies, for example tc^{99m} or I¹²³, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as tc^{99m} or I¹²³, .Re¹⁸⁶, Re¹⁸⁸ and In¹¹¹ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

The compounds of the invention expressly contemplate, but are not limited to, ADC prepared with cross-linker reagents: BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A). See pages 467-498, 2003-2004 Applications Handbook and Catalog.

### Preparation of antibody drug conjugates:

In the antibody drug conjugates (ADC) of the invention, an antibody (Ab) is conjugated to one or more drug moieties (D), e.g. about 1 to about 20 drug moieties per antibody, through a linker (L). The ADC of Formula I may be prepared by several routes, employing organic chemistry reactions, conditions, and reagents known to those skilled in the art, including: (1) reaction of a nucleophilic group of an antibody with a bivalent linker reagent, to form Ab-L, via a covalent bond, followed by reaction with a drug moiety D; and (2) reaction of a nucleophilic group of a drug moiety with a bivalent linker reagent, to form D-L, via a covalent bond, followed by reaction with the nucleophilic group of an antibody. Additional methods for preparing ADC are described herein.

Ab-(L-D)ₚ Formula I

The linker may be composed of one or more linker components. Exemplary linker components include 6-maleimidocaproyl ("MC"), maleimidopropanoyl ("MP"), valine-citrulline ("val-cit"), alanine-phenylalanine ("ala-phe"), p-aminobenzyloxycarbonyl ("PAB"), N-Succinimidyl 4-(2-pyridylthio) pentanoate ("SPP"), N-Succinimidyl 4-(N-maleimidomethyl) cyclohexane-1 carboxylate ("SMCC'), and N-Succinimidyl (4-iodo-acetyl) aminobenzoate ("SIAB"). Additional linker components are known in the art and some are described herein.

In some aspects, the linker may comprise amino acid residues. Exemplary amino acid linker components include a dipeptide, a tripeptide, a tetrapeptide or a pentapeptide. Exemplary dipeptides include: valine-citrulline (vc or val-cit), alanine-phenylalanine (af or ala-phe). Exemplary tripeptides include: glycine-valine-citrulline (gly-val-cit) and glycine-glycine-glycine (gly-gly-gly). Amino acid residues which comprise an amino acid linker component include those occurring naturally, as well as minor amino acids and non-naturally occurring amino acid analogs, such as citrulline. Amino acid linker components can be designed and optimized in their selectivity for enzymatic cleavage by a particular enzymes, for example, a tumor-associated protease, cathepsin B, C and D, or a plasmin protease.

Exemplary linker component structures are shown below (wherein the wavy line indicates sites of covalent attachment to other components of the ADC):

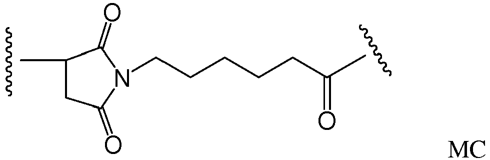

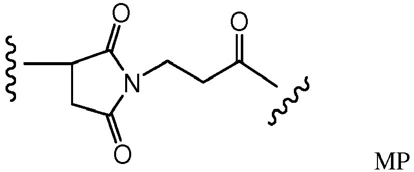

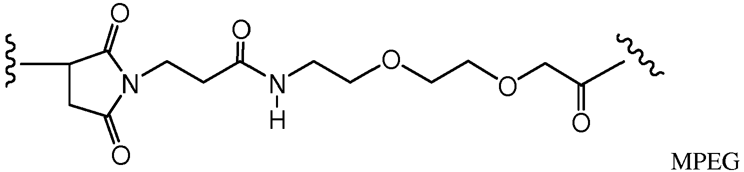

Additional exemplary linker components and abbreviations include (wherein the antibody (Ab) and linker are depicted, and p is 1 to about 8):

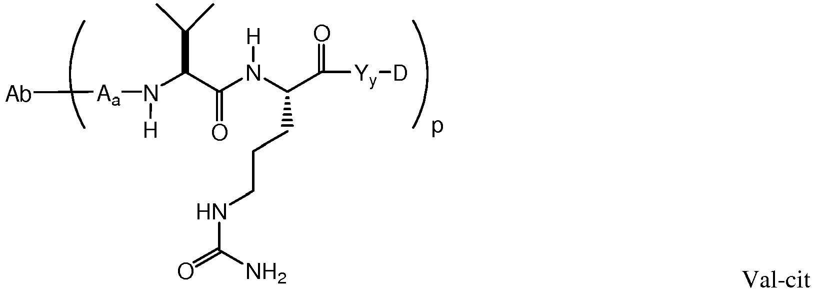

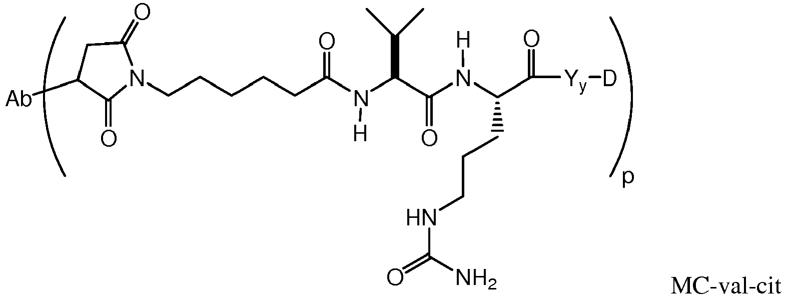

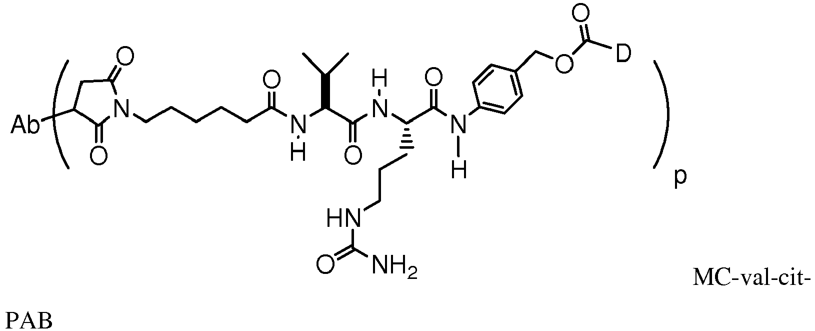

Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by introducing one, two, three, four, or more cysteine residues (e.g., preparing mutant antibodies comprising one or more non-native cysteine amino acid residues).

Antibody drug conjugates of the invention for combination with an anti-CD20 antibody as defined herein, may also be produced by modification of the antibody to introduce electrophilic moieties, which can react with nucleophilic subsituents on the linker reagent or drug. The sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In one aspect, reaction of the carbohydrate portion of a glycosylated antibody with either glactose oxidase or sodium meta-periodate may yield carbonyl (aldehyde and ketone) groups in the protein that can react with appropriate groups on the drug (Hermanson, Bioconjugate Techniques). In another aspect, proteins containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such aldehyde can be reacted with a drug moiety or linker nucleophile.

Likewise, nucleophilic groups on a drug moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

In yet another aspect, the antibody has one or more lysine residues that can be chemically modified to introduce one or more sulfhydryl groups. The antibody unit bonds to the Linker unit via the sulfhydryl group's sulfur atom. The reagents that can be used to modify lysines include, but are not limited to, N-succinimidyl S-acetylthioacetate (SATA) and 2-Iminothiolane hydrochloride (Traut's Reagent).

In another embodiment, the antibody can have one or more carbohydrate groups that can be chemically modified to have one or more sulfhydryl groups. The antibody unit bonds to the Linker Unit, such as the Stretcher Unit, via the sulfhydryl group's sulfur atom, as disclosed herein.

In yet another aspect, the antibody can have one or more carbohydrate groups that can be oxidized to provide an aldehyde (-CHO) group (see, for e.g., Laguzza, et al., J. Med. Chem. 1989, 32(3), 548-55). The corresponding aldehyde can form a bond with a Reactive Site on a Stretcher. Reactive sites on a Stretcher that can react with a carbonyl group on an antibody include, but are not limited to, hydrazine and hydroxylamine. Other protocols for the modification of proteins for the attachment or association of Drug Units are described in Coligan et al., Current Protocols in Protein Science, vol. 2, John Wiley & Sons (2002).

Methods for the conjugation of linker-drug moieties to cell-targeted proteins such as antibodies, immunoglobulins or fragments thereof are found, for example, in US5,208,020; US6,441,163; WO2005037992; WO2005081711; and WO2006/034488.

Alternatively, a fusion protein comprising the antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

In yet another aspect, the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

In one aspect of the immunoconjugate, the cytotoxic agent, D, is an auristatin of formula D_{E} or D_{F} 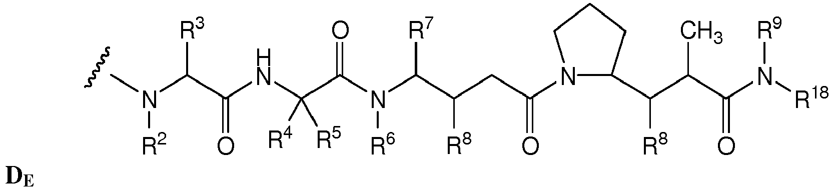 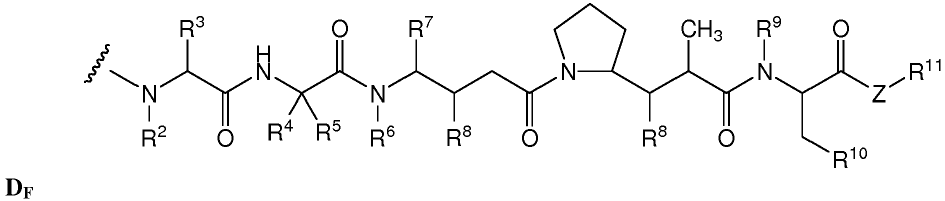 and wherein R² and R⁶ are each methyl, R³ and R⁴ are each isopropyl, R⁷ is sec-butyl, each R⁸ is independently selected from CH₃, O-CH₃, OH, and H; R⁹ is H; R¹⁰ is aryl; Z is -O- or -NH-; R¹¹ is H, C₁-C₈ alkyl, or -(CH₂)₂-O-(CH₂)₂-O-(CH₂)₂-O-CH₃; and R¹⁸ is -C(R⁸)₂-C(R⁸)₂-aryl; and
(d) p ranges from about 1 to 8.

The following aspects are further provided for any of the above immunoconjugates. In one aspect, an immunoconjugate has in vitro or in vivo cell killing activity. In one aspect, the linker is attached to the antibody through a thiol group on the antibody. In one aspect, the linker is cleavable by a protease. In one embodiment, the linker comprises a val-cit dipeptide. In one aspect, the linker comprises a p-aminobenzyl unit. In one aspect, the p-aminobenzyl unit is disposed between the drug and a protease cleavage site in the linker. In one embodiment, the p-aminobenzyl unit is p-aminobenzyloxycarbonyl (PAB). In one embodiment, the linker comprises 6-maleimidocaproyl. In one embodiment, the 6-maleimidocaproyl is disposed between the antibody and a protease cleavage site in the linker. The above embodiments may occur singly or in any combination with one another.

In one embodiment, the drug is selected from MMAE and MMAF. In one embodiment, the immunoconjugate has the formula 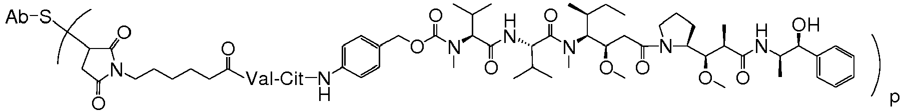 wherein Ab is any of the above anti-CD22 antibodies, S is a sulfur atom, and p ranges from 2 to 5. In one embodiment, the immunoconjugate has the formula 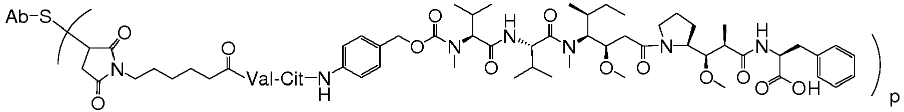 wherein Ab is any of the above anti-CD22 antibodies, S is a sulfur atom, and p ranges from about 1 to about 6, from about 2 to about 5, from about 2 to about 6, from about 2 to about 4, from about 2 to about 3, from about 3 to about 4, from about 3 to about 5, from about 3 to about 6, or from about 4 to about 6.

### In Vitro Activity Assay for IC50 determination of a CD22 antibody-drug conjugate according to the invention

"IC50" refers to the concentration of a particular compound required to inhibit 50% of a specific measured activity. IC50 of the agents that inhibit the CD22 interaction can be measured, inter alia, as is described subsequently.

The term "cytotoxic activity" refers to a cell-killing, cytostatic or growth inhibitory effect of an antibody-drug conjugate or an intracellular metabolite of an antibody-drug conjugate. Cytotoxic activity may be expressed as the IC₅₀ value, which is the concentration (molar or mass) per unit volume at which half the cells survive.

### Surface expression of human CD22 on multiple lymphoma cell lines

Nineteen lymphoma cell lines expressing varying amounts of CD22 on their surface were cultured and harvested in log phase growth. Cells were resuspended in FACS wash buffer (PBS; 0.5% bovine serum albumin; 0.1% sodium azide) containing 100 µg/ml each normal mouse IgG and normal human IgG and maintained on ice. Approximately 1 x 10^6 cells/100 µl were stained with anti-huCD22 APC (mIgG1, clone RFB4, Southern Biotech #9361-11) or murine IgG1 APC isotype (BD Pharmingen #555751) for 30 minutes on ice. Dead cells were stained with 7-AAD (BD Pharmingen #559925). Data were acquired on a BD FacsCalibur™ flow cytometer and analyzed with FlowJo™ software. The IC50 determination for hu10F4v3-SMCC-DM1 or each free drug (DM1, MMAF, or MMAE) were determined by culturing lymphoma cells as above, harvesting the cultured cells in log phase and seeding 5,000 cells in 90 µl culture medium per well in 96 well plate. ADC and free drug were diluted serially within the detection range (starting at 300 µg/ml for ADC, or 90 nM for free drug and diluting to essentially zero assay target). Aliquots of 10 µl diluted ADC or free drug were added to replicate wells containing cells and incubated for 3 days at 37 °C. To each well, 100 µl CellTiter Glo™ was added and incubated for 30 min. Chemiluminescence was detected and data were analyzed using Prism™ software.

The oligosaccharide component can significantly affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. Some generalizations between oligosaccharide structure and glycoprotein function can be made. For example, certain oligosaccharide structures mediate rapid clearance of the glycoprotein from the bloodstream through interactions with specific carbohydrate binding proteins, while others can be bound by antibodies and trigger undesired immune reactions (Jenkins, N., et al., Nature Biotechnol. 14 (1996) 975-981).

Mammalian cells are the excellent hosts for production of therapeutic glycoproteins, due to their capability to glycosylate proteins in the most compatible form for human application (Cumming, D.A., et al., Glycobiology 1 (1991) 115-130; Jenkins, N., et al., Nature Biotechnol. 14 (1996) 975-981). Bacteria very rarely glycosylate proteins, and like other types of common hosts, such as yeasts, filamentous fungi, insect and plant cells, yield glycosylation patterns associated with rapid clearance from the blood stream, undesirable immune interactions, and in some specific cases, reduced biological activity. Among mammalian cells, Chinese hamster ovary (CHO) cells have been most commonly used during the last two decades. In addition to giving suitable glycosylation patterns, these cells allow consistent generation of genetically stable, highly productive clonal cell lines. They can be cultured to high densities in simple bioreactors using serum free media, and permit the development of safe and reproducible bioprocesses. Other commonly used animal cells include baby hamster kidney (BHK) cells, NSO- and SP2/0-mouse myeloma cells. More recently, production from transgenic animals has also been tested (Jenkins, N., et al., Nature Biotechnol. 14 (1996) 975-981).

All antibodies contain carbohydrate structures at conserved positions in the heavy chain constant regions, with each isotype possessing a distinct array of N-linked carbohydrate structures, which variably affect protein assembly, secretion or functional activity (Wright, A., and Morrison, S.L., Trends Biotech. 15 (1997) 26-32). The structure of the attached N-linked carbohydrate varies considerably, depending on the degree of processing, and can include high-mannose, multiply-branched as well as biantennary complex oligosaccharides (Wright, A., and Morrison, S.L., Trends Biotech. 15 (1997) 26-32). Typically, there is heterogeneous processing of the core oligosaccharide structures attached at a particular glycosylation site such that even monoclonal antibodies exist as multiple glycoforms. Likewise, it has been shown that major differences in antibody glycosylation occur between cell lines, and even minor differences are seen for a given cell line grown under different culture conditions (Lifely, M.R., et al., Glycobiology 5 (1995) 813-822).

One way to obtain large increases in potency, while maintaining a simple production process and potentially avoiding significant, undesirable side effects, is to enhance the natural, cell-mediated effector functions of monoclonal antibodies by engineering their oligosaccharide component as described in Umana, P. et al., Nature Biotechnol. 17 (1999) 176-180 and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A. and Morrison, S.L., Trends Biotechnol. 15 (1997) 26-32).

It was previously shown that overexpression in Chinese hamster ovary (CHO) cells of β(1,4)-N-acetylglucosaminyltransferase I11 ("GnTII17y), a glycosyltransferase catalyzing the formation of bisected oligosaccharides, significantly increases the in vitro ADCC activity of an antineuroblastoma chimeric monoclonal antibody (chCE7) produced by the engineered CHO cells (see Umana, P. et al., Nature Biotechnol. 17 (1999) 176-180; and WO 99/154342). The antibody chCE7 belongs to a large class of unconjugated monoclonal antibodies which have high tumor affinity and specificity, but have too little potency to be clinically useful when produced in standard industrial cell lines lacking the GnTIII enzyme (Umana, P., et al., Nature Biotechnol. 17 (1999) 176-180). That study was the first to show that large increases of ADCC activity could be obtained by engineering the antibody producing cells to express GnTIII, which also led to an increase in the proportion of constant region (Fc)-associated, bisected oligosaccharides, including bisected, non-fucosylated oligosaccharides, above the levels found in naturally-occurring antibodies.

The term "cancer" as used herein includes lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, including refractory versions of any of the above cancers, or a combination of one or more of the above cancers. In one embodiment, the term cancer refers to a CD20 expressing cancer.

The term "expression of the CD20" antigen is intended to indicate an significant level of expression of the CD20 antigen in a cell, preferably on the cell surface of a T- or B- cell, more preferably a B-cell, from a tumor or cancer, respectively, preferably a non-solid tumor. Patients having a "CD20 expressing cancer" can be determined by standard assays known in the art. For example CD20 antigen expression can be measured using immunohistochemical (IHC) detection, FACS or via PCR-based detection of the corresponding mRNA.

The term "CD20 expressing cancer" as used herein refers to all cancers in which the cancer cells show an expression of the CD20 antigen. Preferably CD20 expressing cancer as used herein refers to lymphomas (preferably B-Cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma) c) marginal zone lymphomas (including extranodal marginal zone B cell lymphoma (Mucosa-associated lymphatic tissue lymphomas, MALT), nodal marginal zone B cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma) f) hairy cell leukemia, g) lymphocytic lymphoma, waldenstrom's macroglobulinemia, h) acute lymphocytic leukemia (ALL), chronic lymphocytic leukemia (CLL)/ small lymphocytic lymphoma (SLL), B-cell prolymphocytic leukemia, i) plasma cell neoplasms, plasma cell myeloma, multiple myeloma, plasmacytoma j) Hodgkin's disease.

In one embodiment, the CD20 expressing cancer is a B-Cell Non-Hodgkin's lymphomas (NHL). In another embodiment, the CD20 expressing cancer is a Mantle cell lymphoma (MCL), acute lymphocytic leukemia (ALL), chronic lymphocytic leukemia (CLL), B-cell diffuse large cell lymphoma (DLCL), Burkitt's lymphoma, hairy cell leukemia, follicular lymphoma, multiple myeloma, marginal zone lymphoma, post transplant lymphoproliferative disorder (PTLD), HIV associated lymphoma, waldenstrom's macroglobulinemia, or primary CNS lymphoma.

The term "a method of treating" or the respective formulation as a use claim, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "co-administration" or "co-administering" refer to the administration of said afucosylated anti-CD20, and said CD22 antibody-drug conjugate as two separate formulations (or as one single formulation). The co-administration can be simultaneous or sequential in either order, wherein preferably there is a time period while both (or all) active agents simultaneously exert their biological activities. Said anti-CD20 afucosylated antibody and said CD22 antibody-drug conjugate are co-administered either simultaneously or sequentially (e.g. intravenous (i.v.) through a continuous infusion (one for the anti-CD20 antibody and eventually one for said CD22 antibody-drug conjugate; or e.g. the anti-CD20 antibody is administered intravenous (i.v.) through a continuous infusion and said CD22 antibody-drug conjugate is administered orally). When both therapeutic agents are co-administered sequentially the dose is administered either on the same day in two separate administrations, or one of the agents is administered on day 1 and the second is co-administered on day 2 to day 7, preferably on day 2 to 4. Thus in one embodiment the term "sequentially" means within 7 days after the dose of the first component (anti-CD20 antibody or CD22 antibody-drug conjugate), preferably within 4 days after the dose of the first component; and the term "simultaneously" means at the same time. The terms "co-administration" with respect to the maintenance doses of said afucosylated anti-CD20 antibody and said CD22 antibody-drug conjugate mean that the maintenance doses can be either co-administered simultaneously, if the treatment cycle is appropriate for both drugs, e.g. every week. Or CD22 antibody-drug conjugate is e.g. administered e.g. every first to third day and said afucosylated antibody is administered every week. Or the maintenance doses are co-administered sequentially, either within one or within several days.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The amount of co-administration of said anti-CD20 afucosylated antibody and said CD22 antibody-drug conjugate and the timing of co-administration will depend on the type (species, gender, age, weight, etc.) and condition of the patient being treated and the severity of the disease or condition being treated. Said afucosylated anti-CD20 antibody and said CD22 antibody-drug conjugate are suitably co-administered to the patient at one time or over a series of treatments e.g. on the same day or on the day after.

If the administration is intravenous the initial infusion time for said afucosylated anti-CD20 antibody or said CD22 antibody-drug conjugate may be longer than subsequent infusion times, for instance approximately 90 minutes for the initial infusion, and approximately 30 minutes for subsequent infusions (if the initial infusion is well tolerated).

Depending on the type and severity of the disease, about 0.1 mg /kg to 50 mg/kg (e.g. 0.1-20 mg/kg) of said afucosylated anti-CD20 antibody; and 1 µg /kg to 50 mg/kg (e.g. 0.1-20 mg/kg) of said CD22 antibody-drug conjugate is an initial candidate dosage for co-administration of both drugs to the patient. In one aspect the preferred dosage of said afucosylated anti-CD20 antibody (preferably the afocusylated humanized B-Ly1 antibody) will be in the range from about 0.05mg/kg to about 30mg/kg. Thus, one or more doses of about 0.5mg/kg, 2.0mg/kg, 4.0mg/kg, 10mg/kg or 30mg/kg (or any combination thereof) may be co-administered to the patient. In one aspect the preferred dosage of said CD22 antibody-drug conjugate will be in the range from about 0.05mg/kg to about 30mg/kg. Thus, one or more doses of about 0.5mg/kg, 2.0mg/kg, 4.0mg/kg, 10mg/kg or 30mg/kg (or any combination thereof) may be co-administered to the patient.

For treating these cancers, in one aspect, said CD22 antibody-drug conjugate are administered via intravenous infusion, as mentioned above. The dosage administered via infusion is in the range of about 1 µg/m² to about 10,000 µg/m² per dose, generally one dose per week for a total of one, two, three or four doses. Alternatively, the dosage range is of about 1 µg/m² to about 1000 µg/m², about 1 µg/m² to about 800 µg/m², about 1 µg/m² to about 600 µg/m², about 1 µg/m² to about 400 µg/m², about 10 µg/m² to about 500 µg/m², about 10 µg/m² to about 300 µg/m², about 10 µg/m² to about 200 µg/m², and about 1 µg/m² to about 200 µg/m². The dose may be administered once per day, once per week, multiple times per week, but less than once per day, multiple times per month but less than once per day, multiple times per month but less than once per week, once per month or intermittently to relieve or alleviate symptoms of the disease. Administration may continue at any of the disclosed intervals until remission of the tumor or symptoms of the lymphoma, leukemia being treated. Administration may continue after remission or relief of symptoms is achieved where such remission or relief is prolonged by such continued administration.

Depending on the on the type (species, gender, age, weight, etc.) and condition of the patient and on the type of afucosylated anti-CD20 antibody , the dosage and the administration schedule of said afucosylated anti-CD20 antibody can differ from said CD22 antibody-drug conjugate. E.g. the said afucosylated anti-CD20 antibody may be administered e.g. every one to three weeks and said CD22 antibody-drug conjugate may be administered daily or every 2 to 10 days. An initial higher loading dose, followed by one or more lower doses may also be administered.

In one aspect, the preferred dosage of said afucosylated anti-CD20 antibody (preferably the afocusylated humanized B-Ly1 antibody) in the combination with the said CD22 antibody-drug conjugate according to the invention will be 800 to 1600 mg (in on aspect 800 to 1200 mg) on day 1, 8, 15 of a 3- to 6-weeks-dosage-cycle and then in a dosage of 400 to 1200 (in one aspect 800 to 1200 mg on day 1 of up to nine 3- to 4-weeks-dosage-cycles. Most preferably, the dose is a flat dose 1000 mg in a three-weeks-dosage schedule, with the possibility of an adddtional cycle of a flat dose of 1000 mg in the second week.

In yet another aspect, the dose of the said CD22 antibody-drug conjugate in the combination with the afucosylated anti-CD20 antibody according to the invention is about 1.5mg/kg to about 3 mg/kg in a three-weeks-dosage schedule, preferably about 1.7 mg/kg to about 2.5 mg/kg, most preferably about 1.8 mg/kg or about 2.4 mg/kg. Said most preferred dosages are currently tested in clinical trials for CD22 antibody-drug conjugate monotherapy.

In yet another aspect, the dose of the afucosylated anti-CD20 antibody in the combination with the said CD22 antibody-drug conjugate according to the invention is a flat dose of about 1000 mg on day 1 (cycle 1 day 1 (C1D1)), another flat dose of about 1000 mg day 8 (C1D8) and another flat dose of about 1000 mg day 15 (C1D15) followed by six more a flat doses of about 1000 mg of said afucosylated anti-CD20 antibody (Cycle 2) every three weeks: day 22 (C2D1), day 43 (C2D2), day 64 (C2D3), day 85 (C2D4), day 106 (C2D5), and day 127 (C2D6). In said aspect, the dose of the CD22 antibody-drug conjugate in the combination with the afucosylated anti-CD20 antibody according to the invention is about 2.4 mg/kg every three weeks or alternatively 1.8 mg/kg every three weeks. In said aspect, the dosing of the said CD22 antibody-drug conjugate in the combination with the afucosylated anti-CD20 antibody according to the invention is day 1 (C1D1), day 22 (C2D1), day 43 (C2D2), day 64 (C2D3), day 85 (C2D4), day 106 (C2D5) and day 127 (C2D6).

Preferably, in said dosage regimens as described above, the afucosylated anti-CD20 antibody is obinutuzumab or GA101. Also preferably, in said dosage regimens as described above, said said CD22 antibody-drug conjugate is anti-CD22-MC-vc-PAB-MMAE.

The invention also provides a method of alleviating an autoimmune disease, comprising administering to a patient suffering from the autoimmune disease, a therapeutically effective amount of said afucosylated anti-CD20 antibody as disclosed herein and a humanized 10F4 antibody-drug conjugate of any one of the preceding aspects. In preferred aspects the antibody is administered intravenously or subcutaneously. The antibody-drug conjugate is administered intravenously at a dosage in the range of about 1 µg/m² to about 100 mg/m² per dose and in a specific aspect, the dosage is 1 µg/m² to about 500 µg/m². The dose may be administered once per day, once per week, multiple times per week, but less than once per day, multiple times per month but less than once per day, multiple times per month but less than once per week, once per month or intermittently to relieve or alleviate symptoms of the disease. Administration may continue at any of the disclosed intervals until relief from or alleviation of symptoms of the autoimmune disease being treated. Administration may continue after relief from or alleviation of symptoms is achieved where such alleviation or relief is prolong by such continued administration.

The invention also provides a method of treating a B cell disorder comprising administering to a patient suffering from a B cell disorder, such as a B cell proliferative disorder (including without limitation lymphoma and leukemia) or an autoimmune disease, a therapeutically effective amount of said afucosylated anti-CD20 antibody as disclosed herein and a humanized 10F4 antibody of any one of the preceding aspects, which antibody is not conjugated to a cytotoxic molecule or a detectable molecule. The antibody will typically be administered in a dosage range of about 1 µg/m² to about 1000 mg/m².

The recommended dose may vary whether there is a further co-administration of chemotherapeutic agent and based on the type of chemotherapeutic agent

In an embodiment, the medicament is useful for preventing or reducing metastasis or further dissemination in such a patient suffering from cancer, preferably CD20 expressing cancer. The medicament is useful for increasing the duration of survival of such a patient, increasing the progression free survival of such a patient, increasing the duration of response, resulting in a statistically significant and clinically meaningful improvement of the treated patient as measured by the duration of survival, progression free survival, response rate or duration of response. In a preferred embodiment, the medicament is useful for increasing the response rate in a group of patients.

In the context of this invention, additional other cytotoxic, chemotherapeutic or anti-cancer agents, or compounds that enhance the effects of such agents (e.g. cytokines) may be used in the afucosylated anti-CD20 antibody and said CD22 antibody-drug conjugate combination treatment of cancer. Such molecules are suitably present in combination in amounts that are effective for the purpose intended. In one embodiment, the said afucosylated anti-CD20 antibody and said CD22 antibody-drug conjugate combination treatment is used without such additional cytotoxic, chemotherapeutic or anti-cancer agents, or compounds that enhance the effects of such agents.

Such agents include, for example: alkylating agents or agents with an alkylating action, such as cyclophosphamide (CTX; e.g. cytoxan®), chlorambucil (CHL; e.g. leukeran®), cisplatin (CisP; e.g. platinol®) busulfan (e.g. myleran®), melphalan, carmustine (BCNU), streptozotocin, triethylenemelamine (TEM), mitomycin C, and the like; anti-metabolites, such as methotrexate (MTX), etoposide (VP16; e.g. vepesid®), 6-mercaptopurine (6MP), 6-thiocguanine (6TG), cytarabine (Ara-C), 5-fluorouracil (5-FU), capecitabine (e.g. Xeloda®), dacarbazine (DTIC), and the like; antibiotics, such as actinomycin D, doxorubicin (DXR; e.g. adriamycin®), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol®) and paclitaxel derivatives, the cytostatic agents, glucocorticoids such as dexamethasone (DEX; e.g. decadron®) and corticosteroids such as prednisone, nucleoside enzyme inhibitors such as hydroxyurea, amino acid depleting enzymes such as asparaginase, leucovorin and other folic acid derivatives, and similar, diverse antitumor agents. The following agents may also be used as additional agents: arnifostine (e.g. ethyol®), dactinomycin, mechlorethamine (nitrogen mustard), streptozocin, cyclophosphamide, lomustine (CCNU), doxorubicin lipo (e.g. doxil®), gemcitabine (e.g. gemzar®), daunorubicin lipo (e.g. daunoxome®), procarbazine, mitomycin, docetaxel (e.g. taxotere®), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon beta, interferon alpha, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, chlorambucil. In one embodiment, the afucosylated anti-CD20 antibody and said CD22 antibody-drug conjugate combination treatment is used without such additional agents.

The use of the cytotoxic and anticancer agents described above as well as anti-proliferative target-specific anticancer drugs like protein kinase inhibitors in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the cytotoxic agents may vary depending upon the patient's cultured cell response determined by using histoculture methods. Generally, the dosage will be reduced compared to the amount used in the absence of additional other agents.

Typical dosages of an effective cytotoxic agent can be in the ranges recommended by the manufacturer, and where indicated by in vitro responses or responses in animal models, can be reduced by up to about one order of magnitude concentration or amount. Thus, the actual dosage will depend upon the judgment of the physician, the condition of the patient, and the effectiveness of the therapeutic method based on the in vitro responsiveness of the primary cultured malignant cells or histocultured tissue sample, or the responses observed in the appropriate animal models.

In the context of this invention, an effective amount of ionizing radiation may be carried out and/or a radiopharmaceutical may be used in addition to the afucosylated anti-CD20 antibody and said CD22 antibody-drug conjugate combination treatment of CD20 expressing cancer. The source of radiation can be either external or internal to the patient being treated. When the source is external to the patient, the therapy is known as external beam radiation therapy (EBRT). When the source of radiation is internal to the patient, the treatment is called brachytherapy (BT). Radioactive atoms for use in the context of this invention can be selected from the group including, but not limited to, radium, cesium-137, iridium-192, americium-241, gold-198, cobalt-57, copper-67, technetium-99, iodine-123, iodine-131, and indium-111. Is also possible to label the antibody with such radioactive isotopes. In one embodiment, the afucosylated anti-CD20 antibody and said CD22 antibody-drug conjugate combination treatment is used without such ionizing radiation.

Radiation therapy is a standard treatment for controlling unresectable or inoperable tumors and/or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment of this invention there is synergy when tumors in human patients are treated with the combination treatment of the invention and radiation. In other words, the inhibition of tumor growth by means of the agents comprising the combination of the invention is enhanced when combined with radiation, optionally with additional chemotherapeutic or anticancer agents. Parameters of adjuvant radiation therapies are, for example, contained in WO 99/60023.

The afucosylated anti-CD20 antibodies and/or the anti-CD22 antibody-drug conjugate according to the invention are administered to a patient according to known methods, by intravenous administration as a bolus or by continuous infusion over a period of time, by intramuscular, intraperitoneal, intracerobrospinal, subcutaneous, intra-articular, intrasynovial, or intrathecal routes. In one embodiment, the administration of the antibody is intravenous or subcutaneous.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

### Pharmaceutical Compositions:

Pharmaceutical compositions can be obtained by processing the anti-CD20 antibody and/or the CD22 antibody-drug conjugate according to this invention with pharmaceutically acceptable, inorganic or organic carriers. Lactose, corn starch or derivatives thereof, talc, stearic acids or it's salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

In one aspect of the invention the composition comprises both said afucosylated anti-CD20 antibody with an amount of fucose is 60% or less (preferably said afucosylated humanized B-Ly1 antibody) and said CD22 antibody-drug conjugate for use in the treatment of cancer, in particular of CD20 expressing cancer (preferably a lymphoma or lymphocytic leukemiae.g., a B-Cell Non-Hodgkin's lymphoma (NHL).

Said pharmaceutical composition may further comprise one or more pharmaceutically acceptable carriers.

The present invention further provides a pharmaceutical composition, e.g. for use in cancer, comprising (i) an effective first amount of an afucosylated anti-CD20 antibody with an amount of fucose is 60% or less (preferably an afucosylated humanized B-Ly1 antibody), and (ii) an effective second amount of a CD22 antibody-drug conjugate. Such composition optionally comprises pharmaceutically acceptable carriers and / or excipients.

Pharmaceutical compositions of the afucosylated anti-CD20 antibody alone used in accordance with the present invention are prepared for storage by mixing an antibody having the desired degree of purity with optional pharmaceutically acceptable carriers, excipients or stabilizers (Remington's Pharmaceutical Sciences 16th edition, Osol, A. (ed.) (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

Pharmaceutical compositions of antibody CD22 antibody-drug conjugates can be similar to those describe above for the afucosylated anti-CD20 antibody.

Pharmaceutical compositions of small molecule CD22 antibody-drug conjugate include those suitable for oral, nasal, topical (including buccal and sublingual), rectal, vaginal and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of a formula I compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent. Methods of preparing these compositions include the step of bringing into association a CD22 antibody-drug conjugate with the carrier and, optionally, one or more accessory ingredients. In general, the pharmaceutical compositions of the CD22 antibody-drug conjugate are prepared by uniformly and intimately bringing into association a CD22 antibody-drug conjugate with liquid carriers, or finely divided solid carriers, or both, and then, if necessary, shaping the product, compositions suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In one further embodiment of the invention, the afucosylated anti-CD20 antibody and the CD22 antibody-drug conjugate are formulated into two separate pharmaceutical compositions.

The active ingredients may also be entrapped in microcapsules prepared, for example, by coacervation techniques or by interracial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethylmethacrylate), or poly(vinylalcohol)), polylactides (US 3,773,919), copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

One embodiment is a composition comprising a humanized B-Ly1 antibody which is afucosylated with an amount of fucose of 60% or less of the total amount of oligosaccharides (sugars) at Asn297, and CD22 antibody-drug conjugate as disclosed herein, for the treatment of cancer.

The present invention further provides a method for the treatment of cancer, comprising administering to a patient in need of such treatment (i) an effective first amount of an afucosylated anti-CD20 antibody with an amount of fucose is 60% or less, (preferably an afucosylated humanized B-Ly1 antibody); and (ii) an effective second amount of a CD22 antibody-drug conjugate.

In one aspect, the amount of fucose of is between 40% and 60%.

Preferably said cancer is a CD20 expressing cancer.

Preferably said CD20 expressing cancer is a lymphoma or lymphocytic leukemia.

Preferably said afucosylated anti-CD20 antibody is a type II anti-CD20 antibody.

Preferably said antibody is a humanized B-Ly1 antibody. Preferably, said humanized B-Ly1 antibody is obinutuzumab.

Preferably said CD22 antibody-drug conjugate is anti-CD22-MC-vc-PAB-MMAE.

Preferably said afucosylated anti-CD20 antibody is a humanized B-Ly1 antibody and said CD22 antibody-drug conjugate is anti-CD22-MC-vc-PAB-MMAE and said cancer is a CD20 expressing cancer, preferably a lymphoma or lymphocytic leukemia.

As used herein, the term "patient" preferably refers to a human in need of treatment with an afucosylated anti-CD20 antibody (e.g. a patient suffering from CD20 expressing cancer) for any purpose, and more preferably a human in need of such a treatment to treat cancer, or a precancerous condition or lesion. However, the term "patient" can also refer to non-human animals, preferably mammals such as dogs, cats, horses, cows, pigs, sheep and non-human primates, among others.

The invention further comprises an afucosylated anti-CD20 antibody with an amount of fucose is 60% or less, and a CD22 antibody-drug conjugate for use in the treatment of cancer.

Preferably said afucosylated anti-CD20 antibody is a humanized B-Ly1 antibody.

Preferably said CD22 antibody-drug conjugate is in one embodiment of the method according to the invention, the CD22 antibody-drug conjugate is anti-CD22-MC-vc-PAB-MMAE.

Preferably said afucosylated anti-CD20 antibody is a humanized B-Ly1 antibody and said CD22 antibody-drug conjugate is anti-CD22-MC-vc-PAB-MMAE.

Preferably, said cancer is a CD20 expressing cancer, preferably a lymphoma or lymphocytic leukemia.

The following examples and figures are provided to aid the understanding of the present invention, the true scope of which is set forth in the appended claims.

### Experimental Procedures

Primary aim of the study was to investigate the effect of GA101 (obinutuzumab), as defined herein) in combination with an CD22 antibody drug conjugate (CD22-ADC) in the disseminated WSU-DLCL2 DLBCL xenograft model in human CD16 transgenic SCID mice as compared to single agent therapy with GA101, single agent therapy with rituximab and the combination of rituximab with CD22-ADC. The study design is depicted in table 3.

**Table 3: Study design**

| Group | Number of animals | Compound | Dose (µg) | Route of administration | No of treatments |
|---|---|---|---|---|---|
| 1 | 10 | vehicle | - | i.v., once/week | 3 |
| 2 | 10 | GA101 in 20 mM Histidine, 140 mM NaCl, pH 6.0, 65% afucosylation | 600 µg (30 mg/kg) | i.v., once/week | 3 |
| 3 | 10 | Rituximab in 25 mM NaCitrate, 154 mM NaCl, 0.07 w/v % Tween80, pH 6.5 ± 0.3, 8% afucosylation | 600 µg (30 mg/kg) | i.v., once/week | 3 |
| 4 | 10 | CD22-ADC in 20mM Histidine Acetate, 240mM Sucrose, 0.02% PS 20, pH 5.5 | 80 µg (4 mg/kg) | i.v., once | 1 |
| 5 | | GA101 | 600 µg | i.v., once/week | 3 |
| | 10 | CD22-ADC | 80 ug | i.v., once | 1 |
| 6 | | Rituximab | 600 µg | i.v., once/week | 3 |
| | 10 | CD22-ADC | 80 ug | i.v., once | 1 |

### Cell culture and cell application

WSU-DLCL human diffuse large B cell lymphoma cells were originally obtained from Wayne-State University and after expansion deposited in the Roche Glycart internal cell bank. The tumor cell line was routinely cultured in DMEM containing 10 % FCS (Gibco) at 37 °C in a water-saturated atmosphere at 5 % CO2. Passage 15 was used for transplantation in human CD16 transgenic Scid mice. At a viability of 96% 5x106 cells were injected i.v. per animal into the tail vein in 200µl of Aim V cell culture medium (GIBCO). Expression of CD20 and CD22 was confirmed on WSU-DLCL2 lymphoma cells by FACS. For this purpose 0.2 Mio cells were stained in triplicates with anti-human CD20 PE (BD Bioscience #555623), anti-human CD22-PE (BD Bioscience #337899) or the isotype controls mouse IgG1 (BD Bioscience #555749) or mouse IgG2b (BD Bioscience #555743). Mean fluorescence was measured using the plate protocol in the FACS CantoII (Software FACS Diva).

### Animals

60 SCID FcgR3 transgenic female mice (SCID CD16 tg mice), age 7-8 weeks at start of experiment (purchased from Charles River), were maintained under specific-pathogen-free condition with daily cycles of 12 h light /12 h darkness according to committed guidelines (GV-Solas; Felasa; TierschG). Experimental study protocol was reviewed and approved by local veterinary office, license no. P2008016. After arrival animals were maintained for one week to get accustomed to new environment and for observation. Continuous health monitoring was carried out on regular basis.

### Treatment

Animals were randomized for CD16 Expression on murine NK cells with an average of 70%. Treatment started 12 days after cell transplantation. Therapeutic antibodies GA101 and rituximab and the corresponding vehicle were given i.v. on study day 12, 19 and 26 at the dose of 30 mg/kg as single agent. The CD22-ADC was given once on study day 12 at the dose of 4mg/kg. The antibody dilutions were prepared freshly from stock before use. The study was terminated on day 273.

### Monitoring, termination criteria and autopsy

Animals were controlled daily for clinical symptoms and detection of adverse effects. Termination criteria for animals were visible sickness: scruffy fur, arched back, heavy breathing, impaired locomotion, HLP (hind leg paralysis). Mice were sacrificed according to the termination criteria.

### Statistics

Survival data were statistically analyzed by pairwise Wilcoxon and pairwise log-rank test.

### Results

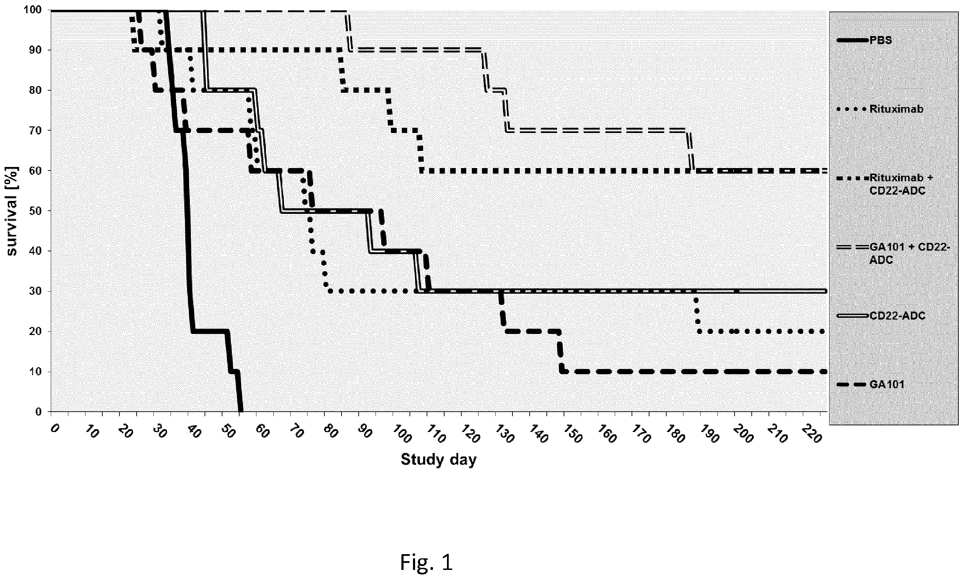

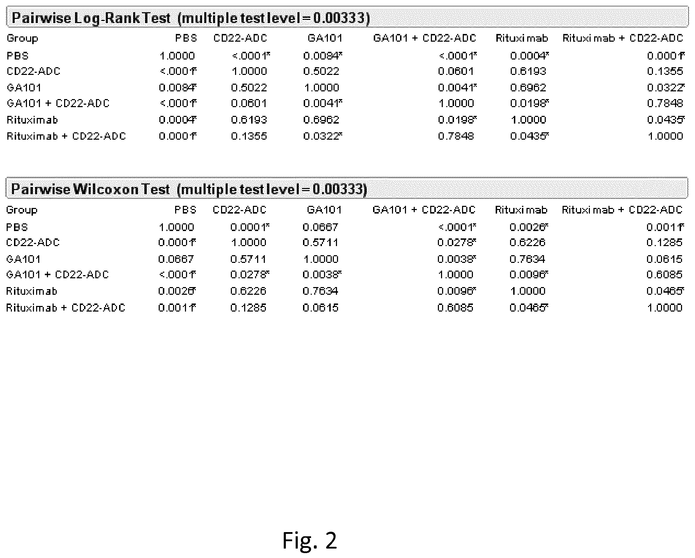

The diffuse large B-cell lymphoma cell line WSU-DLCL2 was intravenously inoculated (5x106 cells) into the tail vein of the human CD16 transgenic Scid mice. Mice were randomized for equal CD16 expression of NK cells and treatment started at day 12 after cell transplantation. The CD22-antibody drug conjugate was given once at day 12 at a dose of 4 mg/kg whereas GA101, rituximab and the corresponding vehicle were given i.v. on study days 12, 19 and 26 at a dose of 30 mg/kg. Animals in the control group received PBS. All animals were controlled daily for clinical symptoms and detection of adverse effects and sacrificed according to the set termination criteria. Study termination was on study day 273. Survival data were represented with the survival curve (Figure 1) and were statistically analyzed by Pairwise Wilcoxon and Pairwise Log-Rank test (Figure 2). Values marked with * in Figure 2 indicate a significant difference. Median and overall survival values for the different treatment groups are given in table 4 and table 5. For the combination of GA101 + CD22-ADC and rituximab + CD22-ADC the median survival was not reached. All animals surviving until day 273 when the experiment was finished appeared tumor free during biopsy.

**Table 4: Median survival**

| Group | PBS | Rituximab | Rituximab + CD22-ADC | GA101 + CD22-ADC | CD22-ADC | GA101 |
|---|---|---|---|---|---|---|
| Median Survival [days] | 40 | 75 | - | - | 80 | 86.5 |

**Table 5: Overall Survival at end of experiment (day 273):**

| Group | PBS | Rituximab | Rituximab + CD22-ADC | GA101 + CD22-ADC | CD22-ADC | GA101 |
|---|---|---|---|---|---|---|
| Overall Survival | 0/10 | 2/10 | 6/10 | 6/10 | 3/10 | 1/10 |

All groups are significantly different from the vehicle group except of GA101 in the Pairwise Wilcoxon test although GA101 and rituximab show no significant difference. Both combinations, GA101 plus CD22-ADC as well as rituximab plus CD22-ADC, show significantly increased survival compared to the respective monotherapies in the Wilcoxon test. In the log rank test the combinations show no significant difference to the monotherapy of anti-CD22-ADC, however, for both combinations the median survival of all groups was not reached and could not be analyzed because in the combinations, GA101 plus CD22-ADC and rituximab plus CD22-ADC, 6 out of 10 animals were tumor free when experiment was finished. In contrast, in the groups treated with GA101, rituximab and anti-CD22-ADC as single agent only 1, 2 and 3 animals respectively were tumor free at the end of the study. Thus, the combination of GA101 and CD22-ADC and the combination of rituximab and CD22-ADC exhibited increased efficacy compared to the respective single agent therapies in terms of overall survival.

## Claims

1. Obinutuzumab, for use in the treatment of cancer in combination with the CD22 antibody-drug conjugate anti-CD22-MC-vc-PAB-MMAE, wherein the anti-CD22 antibody in said CD22 antibody-drug conjugatecomprises the light chain variable domain SEQ ID NO: 50 and the heavy chain variable domain SEQ ID NO: 51.

2. Obinutuzumab, for use in the treatment of cancer in combination with the CD22 antibody-drug conjugate anti-CD22-MC-vc-PAB-MMAE according to claim 1, **characterized in that** said cancer is a CD20 expressing cancer.

3. Obinutuzumab, for use in the treatment of cancer in combination with the CD22 antibody-drug conjugate anti-CD22-MC-vc-PAB-MMAE according to any one of claims 1 to 2, **characterized in that** said CD20 expressing cancer is a lymphoma or lymphocytic leukemia.

4. Obinutuzumab, for use in the treatment of cancer in combination with the CD22 antibody-drug conjugate anti-CD22-MC-vc-PAB-MMAE according to any one of claims 1 to 3, **characterized in that** one or more additional other cytotoxic, chemotherapeutic or anti-cancer agents, or compounds or ionizing radiation that enhance the effects of such agents are administered.

5. A composition for use in the treatment of cancer comprising obinutuzumab and the CD22 antibody-drug conjugate anti-CD22-MC-vc-PAB-MMAE, wherein the anti-CD22 antibody in said CD22 antibody-drug conjugate comprises the light chain variable domain SEQ ID NO: 50 and the heavy chain variable domain SEQ ID NO: 51.

6. The composition for use in the treatment of cancer according to claim 5, **characterized in that** one or more additional other cytotoxic, chemotherapeutic or anti-cancer agents, or compounds or ionizing radiation that enhance the effects of such agents are administered.

## Patentansprüche

1. Obinutuzumab zur Verwendung bei der Behandlung von Krebs in Kombination mit dem CD22-Antikörper-Wirkstoff-Konjugat Anti-CD22-MC-vc-PAB-MMAE, wobei der Anti-CD22-Antikörper in dem CD22-Antikörper-Wirkstoff-Konjugat die variable Domäne der leichten Kette SEQ ID NO: 50 und die variable Domäne der schweren Kette SEQ ID NO: 51 umfasst.

2. Obinutuzumab zur Verwendung bei der Behandlung von Krebs in Kombination mit dem CD22-Antikörper-Wirkstoff-Konjugat Anti-CD22-MC-vc-PAB-MMAE nach Anspruch 1, **dadurch gekennzeichnet, dass** der Krebs ein CD20-exprimierender Krebs ist.

3. Obinutuzumab zur Verwendung bei der Behandlung von Krebs in Kombination mit dem CD22-Antikörper-Wirkstoff-Konjugat Anti-CD22-MC-vc-PAB-MMAE nach einem der Ansprüche 1 bis 2, **dadurch gekennzeichnet, dass** der CD20-exprimierende Krebs ein Lymphom oder lymphatische Leukämie ist.

4. Obinutuzumab zur Verwendung bei der Behandlung von Krebs in Kombination mit dem CD22-Antikörper-Wirkstoff-Konjugat Anti-CD22-MC-vc-PAB-MMAE nach einem der Ansprüche 1 bis 3, **dadurch gekennzeichnet, dass** ein oder mehrere zusätzliche andere zytotoxische, chemotherapeutische oder Antikrebsmittel oder Verbindungen oder ionisierende Strahlung, die die Wirkungen solcher Mittel verstärken, verabreicht werden.

5. Zusammensetzung zur Verwendung bei der Behandlung von Krebs, umfassend Obinutuzumab und das CD22-Antikörper-Wirkstoff-Konjugat Anti-CD22-MC-vc-PAB-MMAE, wobei der Anti-CD22-Antikörper in dem CD22-Antikörper-Wirkstoff-Konjugat die variable Domäne der leichten Kette SEQ ID NO: 50 und die variable Domäne der schweren Kette SEQ ID NO: 51 umfasst.

6. Zusammensetzung zur Verwendung bei der Behandlung von Krebs nach Anspruch 5, **dadurch gekennzeichnet, dass** ein oder mehrere zusätzliche andere zytotoxische, chemotherapeutische oder Antikrebsmittel oder Verbindungen oder ionisierende Strahlung, die die Wirkungen solcher Mittel verstärken, verabreicht werden.

## Revendications

1. Obinutuzumab pour une utilisation dans le traitement d'un cancer en association avec le conjugué anticorps anti-CD22-médicament anti-CD22-MC-vc-PAB-MMAE, dans lequel l'anticorps anti-CD22 dans ledit conjugué anticorps anti-CD22 médicament comprend le domaine variable de chaîne légère SEQ ID NO: 50 et le domaine variable de chaîne lourde SEQ ID NO: 51.

2. Obinutuzumab pour une utilisation dans le traitement d'un cancer en association avec le conjugué anticorps anti-CD22-médicament anti-CD22-MC-vc-PAB-MMAE selon la revendication 1, **caractérisé en ce que** ledit cancer est un cancer exprimant CD20.

3. Obinutuzumab pour une utilisation dans le traitement d'un cancer en association avec le conjugué anticorps anti-CD22-médicament anti-CD22-MC-vc-PAB-MMAE selon l'une quelconque des revendications 1 à 2, **caractérisé en ce que** ledit cancer exprimant CD20 est un lymphome ou la leucémie lymphoïde.

4. Obinutuzumab pour une utilisation dans le traitement d'un cancer en association avec le conjugué anticorps anti-CD22-médicament anti-CD22-MC-vc-PAB-MMAE selon l'une quelconque des revendications 1 à 3, **caractérisé en ce que** sont administrés un ou plusieurs autres agents cytotoxiques, chimiothérapeutiques ou anticancéreux supplémentaires, ou des composés ou des rayonnements ionisants qui améliorent les effets de ces agents.

5. Composition pour une utilisation dans le traitement d'un cancer comprenant de l'obinutuzumab et le conjugué anticorps anti-CD22-médicament anti-CD22-MC-vc-PAB-MMAE, dans laquelle l'anticorps anti-CD22 dans ledit conjugué anticorps anti-CD22-médicament comprend le domaine variable de chaîne légère SEQ ID NO: 50 et le domaine variable de chaîne lourde SEQ ID NO: 51.

6. Composition pour une utilisation dans le traitement d'un cancer selon la revendication 5, **caractérisée en ce que** sont administrés un ou plusieurs autres agents cytotoxiques, chimiothérapeutiques ou anticancéreux supplémentaires, ou des composés ou des rayonnements ionisants qui améliorent les effets de ces agents.
